# EUROPEAN PATENT APPLICATION

(11) **EP 4 768 581 A1**
(43) Date of publication of application: **01.07.2026**
(21) Application number: 24859712.2
(22) Date of filing: 26.08.2024
(51) Int. Cl.: C12N 15/11, C12N 15/09

(54) **STABLE TARGET EDITING GUIDE RNA HAVING PHOSPHATE MOIETY MODIFICATION INTRODUCED THEREINTO**

(30) Priority: 25.08.2023 JP 2023137467
(71) Applicant: Fukuoka University, Fukuoka-shi, Fukuoka 814-0180 (JP); Daiichi Sankyo Company, Limited, Tokyo 103-8426 (JP)
(72) Inventor: FUKUDA, Masatora, Fukuoka-shi, Fukuoka 814-0180 (JP); KOIZUMI, Makoto, Tokyo 103-8426 (JP); IWASHITA, Shinzo, Tokyo 103-8426 (JP); ONISHI, Yoshiyuki, Tokyo 103-8426 (JP)
(74) Representative: Vossius & Partner Patentanwälte Rechtsanwälte mbB
(86) International application number: PCT/JP2024/030306
(87) International publication number: WO 2025/047679

(57) **Abstract**

Provided is an oligonucleotide capable of inducing the editing activity of ADAR in a cell and having excellent stability. The oligonucleotide includes a first oligonucleotide and a second oligonucleotide linked to the 5' side of the first oligonucleotide, and is capable of inducing the site-specific editing in the target RNA. The first oligonucleotide is composed of a target-corresponding nucleoside residue corresponding to an adenosine residue in the target RNA, an oligonucleotide containing 3 to 6 residues, linked to the 5' side of the target-corresponding nucleoside residue, and having a base sequence complementary to the target RNA and an oligonucleotide containing 10 to 24 residues, linked to the 3' side of the target-corresponding nucleoside residue, and having a base sequence complementary to the target RNA, and at least one phosphorus-containing linking group selected from the group consisting of a phosphorus-containing linking group linking a 1st nucleoside residue and a 2nd nucleoside residue and a phosphorus-containing linking group linking a 4th nucleoside residue and a 5th nucleoside residue, counting in the 3' direction from the target-corresponding nucleoside residue, is at least one selected from the group consisting of an alkylphosphonic residue, an alkyl phosphate residue, and a substituted phosphoramide residue. At the 3' end of the second oligonucleotide, a nucleoside residue corresponding to a nucleoside residue of the target RNA is deleted, or, at the 3' end, the second oligonucleotide has a nucleoside residue that does not form a complementary pair with the nucleoside residue of the target RNA, and contains 2 to 10 residues, and nucleoside residues at least other than at the 3' end are complementary to the target RNA.

## Description

### TECHNICAL FIELD

The present invention relates to a stable target-editing guide RNA having a modified phosphate moiety introduced thereinto.

### BACKGROUND ART

The development of genome-editing technology has triggered the use of methods of controlling biological phenomena by modifying the genetic information serving as design drawings of organisms, i.e., modifying DNA information in cells. Such methods have begun to be used as disease treatment approaches in the medical and drug discovery fields. DNA is a constant and immutable molecule within a cell. Hence, the effects of DNA modification persist permanently in the target cell or organism. On the other hand, RNA is a transient genetic information molecule. Accordingly, modifying RNA information can provide a target organism with a non-permanent, transient modification effect on the genetic information.

For example, WO2016/097212 describes, as RNA modification technology, an oligonucleotide construct for site-specific editing of nucleotides in a target RNA sequence. The construct includes: a target-oriented portion containing an antisense sequence complementary to part of a target RNA; and a recruitment portion capable of binding and recruiting an RNA editing entity that exists in cells and can edit nucleotides. For example, WO2017/010556 describes a site-specific RNA mutation method that involves allowing a double-stranded specific adenosine deaminase (ADAR) to act on a complex of a target RNA and a target-editing guide RNA. Additionally, WO2022/124345 describes a stable target-editing guide RNA having a chemically modified nucleic acid introduced thereinto.

### SUMMARY OF THE INVENTION

### PROBLEMS TO BE SOLVED BY THE INVENTION

An aspect of the present invention aims to provide an oligonucleotide that can induce the editing activity of ADAR in a cell, and has excellent stability in a living organism.

### SOLUTIONS TO THE PROBLEMS

A specific means for solving the problem is as described below. The present invention encompasses the following aspects.

(1-1) An oligonucleotide or a pharmaceutically acceptable salt thereof, the oligonucleotide comprising:
a first oligonucleotide that identifies a target RNA; and
a second oligonucleotide linked to the 5' side of the first oligonucleotide,
wherein the first oligonucleotide is composed of:
   a target-corresponding nucleoside residue corresponding to an adenosine residue in the target RNA;
   an oligonucleotide containing 3 to 6 residues, linked to the 5' side of the target-corresponding nucleoside residue, and having a base sequence complementary to the target RNA; and
   an oligonucleotide containing 10 to 24 residues, linked to the 3' side of the target-corresponding nucleoside residue, and having a base sequence complementary to the target RNA,
   wherein, in the oligonucleotide linked to the 3' side of the target-corresponding nucleoside residue, at least one phosphorus-containing linking group selected from the group consisting of a phosphorus-containing linking group linking a 1st nucleoside residue and a 2nd nucleoside residue and a phosphorus-containing linking group linking a 4th nucleoside residue and a 5th nucleoside residue, counting in the 3' direction from the target-corresponding nucleoside residue, is at least one selected from the group consisting of an alkylphosphonic residue, an alkyl phosphate residue, and a substituted phosphoramide residue,
   wherein, at the 3' end of the second oligonucleotide, a nucleoside residue corresponding to a nucleoside residue of the target RNA is deleted, or, at the 3' end, the second oligonucleotide has a nucleoside residue that does not form a complementary pair with the nucleoside residue of the target RNA,
   wherein the second oligonucleotide contains 2 to 10 residues, and nucleoside residues at least other than at the 3' end of the second oligonucleotide form a double-stranded structure complementary to the target RNA,
wherein at least one of the first oligonucleotide or the second oligonucleotide comprises a phosphorothioate bond, and
wherein the oligonucleotide or a pharmaceutically acceptable salt thereof induces site-specific editing in the target RNA.

(1-2) The oligonucleotide or a pharmaceutically acceptable salt thereof according to (1-1), wherein a nucleoside residue linked to the 3' side of the target-corresponding nucleoside residue is a 2'-deoxynucleoside residue.

(1-3) The oligonucleotide or a pharmaceutically acceptable salt thereof according to (1-1) or (1-2), wherein, in the oligonucleotide linked to the 3' side of the target-corresponding nucleoside residue, a 3rd nucleoside residue counting in the 3' direction from the target-corresponding nucleoside residue is a 2'-deoxy-2'-fluoronucleoside residue.

(1-4) The oligonucleotide or a pharmaceutically acceptable salt thereof according to any one of (1-1) to (1-3), wherein the second oligonucleotide contains 4 to 8 residues.

(1-5) The oligonucleotide or a pharmaceutically acceptable salt thereof according to any one of (1-1) to (1-4), wherein, at the 3' end of the second oligonucleotide, the nucleoside residue corresponding to the nucleoside residue of the target RNA is deleted.

(1-6) The oligonucleotide or a pharmaceutically acceptable salt thereof according to any one of (1-1) to (1-5), wherein the site-specific editing is caused by an enzymatic reaction due to adenosine deaminase 1.

(1-7) The oligonucleotide or a pharmaceutically acceptable salt thereof according to any one of (1-1) to (1-6), wherein the first oligonucleotide comprises a phosphorothioate bond.

(1-8) The oligonucleotide or a pharmaceutically acceptable salt thereof according to any one of (1-1) to (1-7), wherein at least one phosphorus-containing linking group selected from the group consisting of: a phosphorus-containing linking group linking the 2nd nucleoside residue and the 3rd nucleoside residue, a phosphorus-containing linking group linking an 8th nucleoside residue and a 9th nucleoside residue, a phosphorus-containing linking group linking a 10th nucleoside residue and an 11th nucleoside residue, and a phosphorus-containing linking group linking the 11th nucleoside residue and a 12th nucleoside residue, counting in the 3' direction from the target-corresponding nucleoside residue; and a phosphorus-containing linking group linking a 6th nucleoside residue and the 5th nucleoside residue, counting in the 5' direction from the target-corresponding nucleoside residue, is a phosphoric residue.

(1-9) The oligonucleotide or a pharmaceutically acceptable salt thereof according to any one of (1-1) to (1-8), wherein, in the first oligonucleotide, the 2nd nucleoside residue and the 3rd nucleoside residue counting in the 3' direction from the target-corresponding nucleoside residue are linked by a phosphodiester bond.

(1-10) The oligonucleotide or a pharmaceutically acceptable salt thereof according to any one of (1-1) to (1-9), wherein the first oligonucleotide comprises at least one modified nucleoside residue selected from the group consisting of a 2'-O-alkylribonucleoside residue, a 2'-deoxy-2'-fluororibonucleoside residue, a bridged nucleoside residue, and a 2'-deoxyribonucleoside residue.

(1-11) The oligonucleotide or a pharmaceutically acceptable salt thereof according to any one of (1-1) to (1-10), the oligonucleotide comprising a partial structure in which the target-corresponding nucleoside residue is linked to an adjacent nucleoside residue via a phosphorothioate bond.

(1-12) The oligonucleotide or a pharmaceutically acceptable salt thereof according to any one of (1-1) to (1-11), wherein a nucleoside residue linked to the 5' side of the target-corresponding nucleoside residue is a 2'-O-alkylribonucleoside residue.

(1-13) The oligonucleotide or a pharmaceutically acceptable salt thereof according to any one of (1-1) to (1-12), wherein the second oligonucleotide comprises at least one modified nucleoside residue selected from the group consisting of a 2'-O-alkylribonucleoside residue, a 2'-deoxy-2'-fluororibonucleoside residue, and a bridged nucleoside residue.

(1-14) The oligonucleotide or a pharmaceutically acceptable salt thereof according to any one of (1-1) to (1-13), wherein the oligonucleotide linked to the 3' side of the target-corresponding nucleoside residue has a base sequence in which 2'-deoxy-2'-fluoronucleoside residues and 2'-O-alkylribonucleoside residues are alternately linked.

(1-15) The oligonucleotide or a pharmaceutically acceptable salt thereof according to any one of (1-1) to (1-14), wherein the oligonucleotide linked to the 3' side of the target-corresponding nucleoside residue has a base sequence in which bridged nucleoside residues and 2'-O-alkylribonucleoside residues are alternately linked.

(1-16) The oligonucleotide or a pharmaceutically acceptable salt thereof according to any one of (1-1) to (1-15), wherein, in the first oligonucleotide, the oligonucleotide linked to the 5' side of the target-corresponding nucleoside residue has a base sequence in which 2'-O-alkylribonucleoside residues are consecutive.

(1-17) The oligonucleotide or a pharmaceutically acceptable salt thereof according to any one of (1-1) to (1-16), wherein the second oligonucleotide has a base sequence in which 2'-O-alkylribonucleoside residues and bridged nucleoside residues are alternately linked.

(1-18) The oligonucleotide or a pharmaceutically acceptable salt thereof according to any one of (1-1) to (1-17), wherein the target-corresponding nucleoside residue is an N-alkylpyrimidine nucleoside residue or a 2'-deoxycytidine residue.

(1-19) The oligonucleotide or a pharmaceutically acceptable salt thereof according to any one of (1-1) to (1-18), wherein the second oligonucleotide comprises nucleoside residues linked by a phosphorothioate bond.

(1-20) The oligonucleotide or a pharmaceutically acceptable salt thereof according to any one of (1-1) to (1-19), wherein the first oligonucleotide contains a bridged nucleoside residue as the 10th or a subsequent nucleoside residue counting in the 3' direction from the target-corresponding nucleoside residue.

(1-21) The oligonucleotide or a pharmaceutically acceptable salt thereof according to (1-20), wherein, in the first oligonucleotide, an oligonucleotide containing the 10th nucleoside residue and subsequent nucleoside residues counting in the 3' direction from the target-corresponding nucleoside residue has a base sequence in which 2'-O-alkylribonucleoside residues and bridged nucleoside residues are alternately linked.

(1-22) The oligonucleotide or a pharmaceutically acceptable salt thereof according to any one of (1-1) to (1-21), wherein the second oligonucleotide contains a bridged nucleoside residue as the 2nd or a subsequent nucleoside residue counting in the 5' direction from a nucleoside residue at the 3' end of the second oligonucleotide.

(1-23) The oligonucleotide or a pharmaceutically acceptable salt thereof according to (1-22), wherein the second oligonucleotide comprises three or more nucleoside residues, and wherein an oligonucleotide containing the 2nd nucleoside residue and subsequent nucleoside residues counting in the 5' direction from the nucleoside residue at the 3' end of the second oligonucleotide has a base sequence in which 2'-O-alkylribonucleoside residues and bridged nucleoside residues are alternately linked.

(1-24) The oligonucleotide or a pharmaceutically acceptable salt thereof according to any one of claims 1 to 7, the oligonucleotide comprising a modified oligonucleotide having a chemical modification pattern represented by any one of the following Formulas (1) to (20).

(1) 5'- HO-MsLsMsLsMsFsXFsMsMsdCsDqMsFsMqFsMsFsMsFsMsLsMsLsM-OH -
3'

(2) 5’- HO-MsLsMsLsMsFsXFsMsMsdCsDqMsFsMqFsMsFsMsFsMsMsMsLsM-OH -
3’

(3) 5’- HO-MsLsMsMsMsFsXFsMsMsdCsDqMsFsMqFsMsFsMsFsMsLsMsLsM-OH -
3’

(4) 5'- HO-MsLsMsMsMsFsXFsMsMsdCsDqMsFsMqFsMsFsMsFsMsMsMsLsM-OH
-3'

(5) 5'- HO-LsMsMsMsMsFsXFsMsMsdCsDqMsFsMqFsMsFsMsFsMsMsMsLsM-OH

-3’

(6) 5'- HO-MsLsMsLsMsFsXFsMsMsdCsDqMpFsMqFsMsFsMsFsMsLsMsLsM-OH -
3'

(7) 5’- HO-MsLsMsLsMsFsXFpMsMsdCsDqMpFsMqFsMsFsMsFsMsLsMsLsM-OH -
3’

(8) 5’- HO-MsLsMsLsMsFsXFsMpMsdCsDqMsFsMqFsMsFsMsFsMsLsMsLsM-OH -
3’

(9) 5’- HO-MsLsMsLpMsFsXFsMsMsdCsDqMpFsMqFsMsFsMsFsMsLpMsLsM-OH -
3’

(10) 5’- HO-MsLsMsLpMsFsXFsMsMsdCsDqMpFsMqFsMpFsMsFsMsLpMsLsM-
OH -3’

(11) 5’- HO-MsLsMsLpMsFsXFsMsMsdCsDqMpFsMqFsMsFsMpFsMsLpMsLsM-OH
-3’

(12) 5'- HO-MsLsMsLpMsFsXFsMsMsdCsDqMpFsMqFsMsFsMsFsMpLpMsLsM-
OH -3'

(13) 5'- HO-MsLsMsLsMsFsXFsMsMsdCsDqMpFsMqFsMsFsMsFsMsMsMsLsM-OH
-3'

(14) 5’- HO-MsLsMsLsMsFsXFsMsMsdCsDqMpFsMqFsMpFsMsFsMsMsMsLsM-
OH -3’

(15) 5'- HO-MsLsMsLsMsFsXFsMsMsdCsDqMpFsMqFsMsFsMpFsMsMsMsLsM-
OH -3'

(16) 5'- HO-MsLsMsLsMsFsXFsMsMsdCsDqMpFsMqFsMsFsMsFsMpMsMsLsM-
OH -3'

(17) 5'- HO-MsLsMsLsMsFsXFsMsMsdCsDqMpFsMqFsMpFsMpFsMsMsMsLsM-
OH -3'

(18) 5'- HO-MsLsMsLsMsFsXFsMsMsdCsDqMpFsMqFsMpFsMsFsMpMsMsLsM-
OH -3'

(19) 5'- HO-MsLsMsLsMsFsXFsMsMsdCsDqMpFsMqFsMsFsMpFsMpMsMsLsM-
OH -3'

(20)   5'-   HO-MsLsMsLsMsFsXFsMsMsdCsDqMpFsMqFsMpFsMpFsMpMsMsLsM-

OH -3'

In the Formulas, D represents a 2'-deoxyribonucleoside residue, F represents a 2'-fluoro-2'-deoxyribonucleoside residue, L represents a bridged nucleoside residue, M represents a 2'-O-methyl- ribonucleoside residue, dC represents a target nucleoside residue, a 2'-deoxycytidine residue. X indicates that one nucleoside residue is missing from the target RNA. p represents a phosphate residue, s represents a thiophosphate residue, q represents an alkylphosphonate residue, an alkyl phosphate residue, or a substituted phosphoramide residue. HO- represents a hydroxyl group at the 5' end, and -OH represents a hydroxyl group at the 3' end.

(1-25) The oligonucleotide or a pharmaceutically acceptable salt thereof according to any one of claims 1 to 7, the oligonucleotide comprising a modified oligonucleotide having a chemical modification pattern represented by any one of the following Formulas (1a) to (20a).

(1a) 5'- HO-MsLsMsLsMsFsXFsMsMsdCsDpmMsFsMpmFsMsFsMsFsMsLsMsLsM-
OH -3'

(2a) 5'- HO-MsLsMsLsMsFsXFsMsMsdCsDpmMsFsMpmFsMsFsMsFsMsMsMsLsM-
OH -3'

(3a) 5'- HO-MsLsMsMsMsFsXFsMsMsdCsDpmMsFsMpmFsMsFsMsFsMsLsMsLsM-
OH -3'

(4a) 5’-HO-MsLsMsMsMsFsXFsMsMsdCsDpmMsFsMpmFsMsFsMsFsMsMsMsLsM-
OH -3’

(5a) 5'-HO-LsMsMsMsMsFsXFsMsMsdCsDpmMsFsMpmFsMsFsMsFsMsMsMsLsM-
OH -3'

(6a) 5'- HO-MsLsMsLsMsFsXFsMsMsdCsDrMpFsMrFsMsFsMsFsMsLsMsLsM-OH -
3'

(7a) 5'- HO-MsLsMsLsMsFsXFpMsMsdCsDrMpFsMrFsMsFsMsFsMsLsMsLsM-OH -
3'

(8a) 5'- HO-MsLsMsLsMsFsXFsMpMsdCsDrMsFsMrFsMsFsMsFsMsLsMsLsM-OH -
3'

(9a) 5'- HO-MsLsMsLpMsFsXFsMsMsdCsDrMpFsMrFsMsFsMsFsMsLpMsLsM-OH
-3'

(10a) 5'- HO-MsLsMsLpMsFsXFsMsMsdCsDrMpFsMrFsMpFsMsFsMsLpMsLsM-
OH -3'

(11a) 5’- HO-MsLsMsLpMsFsXFsMsMsdCsDrMpFsMrFsMsFsMpFsMsLpMsLsM-
OH -3’

(12a) 5'- HO-MsLsMsLpMsFsXFsMsMsdCsDrMpFsMrFsMsFsMsFsMpLpMsLsM-
OH -3'

(13a) 5'- HO-MsLsMsLsMsFsXFsMsMsdCsDrMpFsMrFsMsFsMsFsMsMsMsLsM-
OH -3'

(14a) 5'- HO-MsLsMsLsMsFsXFsMsMsdCsDrMpFsMrFsMpFsMsFsMsMsMsLsM-
OH -3'

(15a) 5'- HO-MsLsMsLsMsFsXFsMsMsdCsDrMpFsMrFsMsFsMpFsMsMsMsLsM-
OH -3'

(16a) 5'- HO-MsLsMsLsMsFsXFsMsMsdCsDrMpFsMrFsMsFsMsFsMpMsMsLsM-
OH -3'

(17a) 5'- HO-MsLsMsLsMsFsXFsMsMsdCsDrMpFsMrFsMpFsMpFsMsMsMsLsM-
OH -3'

(18a) 5’- HO-MsLsMsLsMsFsXFsMsMsdCsDrMpFsMrFsMpFsMsFsMpMsMsLsM-
OH -3’

(19a) 5’- HO-MsLsMsLsMsFsXFsMsMsdCsDrMpFsMrFsMsFsMpFsMpMsMsLsM-
OH -3’

(20a) 5'- HO-MsLsMsLsMsFsXFsMsMsdCsDrMpFsMrFsMpFsMpFsMpMsMsLsM-OH -3'

In the Formulas, D represents a 2'-deoxyribonucleoside residue, F represents a 2'-fluoro-2'-deoxyribonucleoside residue, L represents a bridged nucleoside residue, M represents a 2'-O-methyl- ribonucleoside residue, dC represents a target nucleoside residue, a 2'-deoxycytidine residue. X indicates that one nucleoside residue is missing from the target RNA. p represents a phosphate residue, s represents a thiophosphate residue, q represents an alkylphosphonate residue, an alkyl phosphate residue, or a substituted phosphoramide residue. HO- represents a hydroxyl group at the 5' end, and -OH represents a hydroxyl group at the 3' end.

(1-26) The oligonucleotide or a pharmaceutically acceptable salt thereof according to any one of (1-1) to (1-7), the oligonucleotide comprising a modified oligonucleotide having a chemical modification pattern represented by any one of the following Formulas (1) to (22).

(1) 5'- HO-MsLsMsLsMsFsXFsMsMsdCsDqMsFsMqFsMsFsMsFsMsLsMsLsM-OH -
3'

(2) 5'- HO-MsLsMsLsMsFsXFsMsMsdCsDqMsFsMqFsMsFsMsFsMsMsMsLsM-OH -
3'

(3) 5’- HO-MsLsMsMsMsFsXFsMsMsdCsDqMsFsMqFsMsFsMsFsMsLsMsLsM-OH -
3’

(4) 5'- HO-MsLsMsMsMsFsXFsMsMsdCsDqMsFsMqFsMsFsMsFsMsMsMsLsM-OH
-3'

(5) 5'- HO-LsMsMsMsMsFsXFsMsMsdCsDqMsFsMqFsMsFsMsFsMsMsMsLsM-OH
-3'

(6) 5'- HO-MsLsMsLsMsFsXFsMsMsdCsDqMpFsMqFsMsFsMsFsMsLsMsLsM-OH -
3'

(7) 5'- HO-MsLsMsLsMsFsXFpMsMsdCsDqMpFsMqFsMsFsMsFsMsLsMsLsM-OH -
3'

(8) 5’- HO-MsLsMsLsMsFsXFsMpMsdCsDqMsFsMqFsMsFsMsFsMsLsMsLsM-OH -
3’

(9) 5’- HO-MsLsMsLpMsFsXFsMsMsdCsDqMpFsMqFsMsFsMsFsMsLpMsLsM-OH -
3’

(10) 5'- HO-MsLsMsLpMsFsXFsMsMsdCsDqMpFsMqFsMpFsMsFsMsLpMsLsM-
OH -3'

(11) 5'- HO-MsLsMsLpMsFsXFsMsMsdCsDqMpFsMqFsMsFsMpFsMsLpMsLsM-OH
-3'

(12) 5'- HO-MsLsMsLpMsFsXFsMsMsdCsDqMpFsMqFsMsFsMsFsMpLpMsLsM-
OH -3'

(13) 5’- HO-MsLsMsLsMsFsXFsMsMsdCsDqMpFsMqFsMsFsMsFsMsMsMsLsM-OH
-3’

(14) 5'- HO-MsLsMsLsMsFsXFsMsMsdCsDqMpFsMqFsMpFsMsFsMsMsMsLsM-
OH -3'

(15) 5'- HO-MsLsMsLsMsFsXFsMsMsdCsDqMpFsMqFsMsFsMpFsMsMsMsLsM-
OH -3'

(16) 5'- HO-MsLsMsLsMsFsXFsMsMsdCsDqMpFsMqFsMsFsMsFsMpMsMsLsM-
OH -3'

(17) 5'- HO-MsLsMsLsMsFsXFsMsMsdCsDqMpFsMqFsMpFsMpFsMsMsMsLsM-
OH -3'

(18) 5'- HO-MsLsMsLsMsFsXFsMsMsdCsDqMpFsMqFsMpFsMsFsMpMsMsLsM-
OH -3'

(19)  5'-  HO-MsLsMsLsMsFsXFsMsMsdCsDqMpFsMqFsMsFsMpFsMpMsMsLsM-
OH -3'

(20) 5'- HO-MsLsMsLsMsFsXFsMsMsdCsDqMpFsMqFsMpFsMpFsMpMsMsLsM-
OH -3'

(21) 5'- HO-MsLsMsLsMsFsXFsMsMsdCsDqMpFsMsFsMsFsMsFsMsLsMsLsM-OH
-3'

(22) 5’- HO-MsLsMsLsMsFsXFsMsMsdCsDqMpFsMsFsMsFsMsFsMsMsMsLsM-OH
-3’

In the Formulas, D represents a 2'-deoxyribonucleoside residue, F represents a 2'-fluoro-2'-deoxyribonucleoside residue, L represents a bridged nucleoside residue, M represents a 2'-O-methyl- ribonucleoside residue, dC represents a target nucleoside residue, a 2'-deoxycytidine residue. X indicates that one nucleoside residue is missing from the target RNA. p represents a phosphate residue, s represents a thiophosphate residue, q represents an alkylphosphonate residue, an alkyl phosphate residue, or a substituted phosphoramide residue. HO- represents a hydroxyl group at the 5' end, and -OH represents a hydroxyl group at the 3' end.

(1-27) The oligonucleotide or a pharmaceutically acceptable salt thereof according to any one of (1-1) to (1-7), the oligonucleotide comprising a modified oligonucleotide having a chemical modification pattern represented by any one of the following Formulas (1a) to (22a):

(1a) 5'- HO-MsLsMsLsMsFsXFsMsMsdCsDpmMsFsMpmFsMsFsMsFsMsLsMsLsM-
OH -3'

(2a) 5’- HO-MsLsMsLsMsFsXFsMsMsdCsDpmMsFsMpmFsMsFsMsFsMsMsMsLsM-
OH -3’

(3a) 5'- HO-MsLsMsMsMsFsXFsMsMsdCsDpmMsFsMpmFsMsFsMsFsMsLsMsLsM-
OH -3'

(4a) 5'-HO-MsLsMsMsMsFsXFsMsMsdCsDpmMsFsMpmFsMsFsMsFsMsMsMsLsM-
OH -3'

(5a) 5'-HO-LsMsMsMsMsFsXFsMsMsdCsDpmMsFsMpmFsMsFsMsFsMsMsMsLsM-
OH -3'

(6a) 5'- HO-MsLsMsLsMsFsXFsMsMsdCsDrMpFsMrFsMsFsMsFsMsLsMsLsM-OH -
3'

(7a) 5'- HO-MsLsMsLsMsFsXFpMsMsdCsDrMpFsMrFsMsFsMsFsMsLsMsLsM-OH -
3'

(8a) 5'- HO-MsLsMsLsMsFsXFsMpMsdCsDrMsFsMrFsMsFsMsFsMsLsMsLsM-OH -
3'

(9a) 5’- HO-MsLsMsLpMsFsXFsMsMsdCsDrMpFsMrFsMsFsMsFsMsLpMsLsM-OH
-3’

(10a) 5'- HO-MsLsMsLpMsFsXFsMsMsdCsDrMpFsMrFsMpFsMsFsMsLpMsLsM-
OH -3'

(11a)   5’-   HO-MsLsMsLpMsFsXFsMsMsdCsDrMpFsMrFsMsFsMpFsMsLpMsLsM-
OH -3’

(12a) 5'- HO-MsLsMsLpMsFsXFsMsMsdCsDrMpFsMrFsMsFsMsFsMpLpMsLsM-
OH -3'

(13a) 5'- HO-MsLsMsLsMsFsXFsMsMsdCsDrMpFsMrFsMsFsMsFsMsMsMsLsM-
OH -3'

(14a) 5'- HO-MsLsMsLsMsFsXFsMsMsdCsDrMpFsMrFsMpFsMsFsMsMsMsLsM-

OH -3'

(15a) 5'- HO-MsLsMsLsMsFsXFsMsMsdCsDrMpFsMrFsMsFsMpFsMsMsMsLsM-
OH -3'

(16a) 5'- HO-MsLsMsLsMsFsXFsMsMsdCsDrMpFsMrFsMsFsMsFsMpMsMsLsM-
OH -3'

(17a) 5'- HO-MsLsMsLsMsFsXFsMsMsdCsDrMpFsMrFsMpFsMpFsMsMsMsLsM-
OH -3'

(18a) 5'- HO-MsLsMsLsMsFsXFsMsMsdCsDrMpFsMrFsMpFsMsFsMpMsMsLsM-
OH -3'

(19a) 5'- HO-MsLsMsLsMsFsXFsMsMsdCsDrMpFsMrFsMsFsMpFsMpMsMsLsM-
OH -3'

(20a) 5'- HO-MsLsMsLsMsFsXFsMsMsdCsDrMpFsMrFsMpFsMpFsMpMsMsLsM-
OH -3'

(21a) 5'- HO-MsLsMsLsMsFsXFsMsMsdCsDrMpFsMsFsMsFsMsFsMsLsMsLsM-OH
-3'

(22a) 5’- HO-MsLsMsLsMsFsXFsMsMsdCsDrMpFsMsFsMsFsMsFsMsMsMsLsM-
OH -3’

In the Formulas, D represents a 2'-deoxyribonucleoside residue, F represents a 2'-fluoro-2'-deoxyribonucleoside residue, L represents a bridged nucleoside residue, M represents a 2'-O-methyl- ribonucleoside residue, dC represents a target nucleoside residue, a 2'-deoxycytidine residue. X indicates that one nucleoside residue is missing from the target RNA. p represents a phosphate residue, s represents a thiophosphate residue, q represents an alkylphosphonate residue, an alkyl phosphate residue, or a substituted phosphoramide residue. HO- represents a hydroxyl group at the 5' end, and -OH represents a hydroxyl group at the 3' end.

(2-1) An oligonucleotide or a pharmaceutically acceptable salt thereof, which oligonucleotide induces site-specific editing that converts an editing target adenosine residue contained in a target RNA into an inosine residue,
wherein the oligonucleotide includes a structure represented by the following Formula (A),
a target-corresponding nucleoside residue corresponding to the editing target adenosine residue is represented by N⁶,
an oligonucleotide composed of nucleoside residues excluding N⁶ and X has a base sequence complementary to the target RNA, and
the base sequence corresponds to a base sequence of the target RNA, such that the target RNA contains a base corresponding to X, or contains a base at the position corresponding to X in the case of a deletion of X.

   (A) 5'-O¹-(N¹-L¹)-(N²-L2)-X-(N³-L³)ₚ-(N⁴-L⁴)-(N⁵-L⁵)-(N⁶-L⁶)-(N⁷-L⁷)-(N⁸-L⁸)-(N⁹-L⁹)-(N¹⁰-L¹⁰)-O²-O³-3'

In Formula (A), O¹ represents an oligonucleotide of 1 to 8 residues that forms a complementary pair with the corresponding oligonucleotide portion of the target RNA;
N¹ to N⁵ and N⁷ to N¹⁰ each represent a nucleoside residue that forms a complementary pair with the corresponding nucleoside residue of the target RNA;
N⁶ represents a 2'-deoxycytidine residue, a 2'-deoxyguanosine residue, a 2'-deoxyadenosine residue, or a derivative thereof;
O² and O³ each independently represent an oligonucleotide of 3 to 17 residues that forms a complementary pair with the corresponding oligonucleotide portion of the target RNA, and the total number of residues of O² and O³ is 6 to 20 residues;
X represents a deletion of one nucleoside residue with respect to the target RNA, or represents a nucleoside residue and a phosphorus-containing linking group that are not complementary to the corresponding nucleoside residue of the target RNA;
L¹, L², L³, L⁴, L⁵, L⁶, L⁸, and L⁹ each independently represent a phosphorus-containing linking group selected from the group consisting of a phosphoric residue and a thiophosphoric residue;
at least one of L¹, L², L³, L⁴, L⁵, L⁶, L⁸, or L⁹ represents a thiophosphoric residue;
L⁷ and L¹⁰ are each independently selected from the group consisting of a phosphoric residue, a thiophosphoric residue, an alkylphosphonic residue, an alkyl phosphate residue, and a substituted phosphoramide residue; at least one of L⁷ or L¹⁰ represents a phosphorus-containing linking group selected from the group consisting of an alkylphosphonic residue, an alkyl phosphate residue, and a substituted phosphoramide residue; and
p represents an integer of 1 to 4.

(2-2) The oligonucleotide or a pharmaceutically acceptable salt thereof according to (2-1), wherein all of L¹, L², L³, L⁴, L⁵, L⁶, L⁸, and L⁹ represent thiophosphoric residues, or 1 to 5 thereof represent phosphoric residues, and the remainder represent thiophosphoric residues.

(2-3) The oligonucleotide or a pharmaceutically acceptable salt thereof according to (2-1) or (2-2), wherein L⁷ and L¹⁰ are each independently a phosphorus-containing linking group selected from the group consisting of an alkylphosphonic residue, an alkyl phosphate residue, and a substituted phosphoramide residue.

(2-4) The oligonucleotide or a pharmaceutically acceptable salt thereof according to any one of (2-1) to (2-3), wherein L⁸ is a phosphoric residue.

(2-5) The oligonucleotide or a pharmaceutically acceptable salt thereof according to any one of (2-1) to (2-4), wherein O¹ includes an oligonucleotide in which 2'-O-alkylribonucleoside residues and bridged nucleoside residues are alternately linked.

(2-6) The oligonucleotide or a pharmaceutically acceptable salt thereof according to any one of (2-1) to (2-5), wherein O² includes an oligonucleotide in which 2'-deoxy-2'-fluoronucleoside residues and 2'-O-alkylribonucleoside residues are alternately linked.

(2-7) The oligonucleotide or a pharmaceutically acceptable salt thereof according to any one of (2-1) to (2-6), wherein O³ includes an oligonucleotide in which 2'-O-alkylribonucleoside residues and bridged nucleoside residues are alternately linked.

(2-8) The oligonucleotide or a pharmaceutically acceptable salt thereof according to any one of (2-1) to (2-7), the oligonucleotide comprising a modified oligonucleotide having a chemical modification pattern represented by any one of the following Formulas (1) to (20). [0049]

(1) 5'- HO-MsLsMsLsMsFsXFsMsMsdCsDqMsFsMqFsMsFsMsFsMsLsMsLsM-OH -
3'

(2) 5'- HO-MsLsMsLsMsFsXFsMsMsdCsDqMsFsMqFsMsFsMsFsMsMsMsLsM-OH -

3'

(3) 5’- HO-MsLsMsMsMsFsXFsMsMsdCsDqMsFsMqFsMsFsMsFsMsLsMsLsM-OH -
3’

(4) 5'- HO-MsLsMsMsMsFsXFsMsMsdCsDqMsFsMqFsMsFsMsFsMsMsMsLsM-OH
-3'

(5) 5'- HO-LsMsMsMsMsFsXFsMsMsdCsDqMsFsMqFsMsFsMsFsMsMsMsLsM-OH
-3'

(6) 5'- HO-MsLsMsLsMsFsXFsMsMsdCsDqMpFsMqFsMsFsMsFsMsLsMsLsM-OH -
3'

(7) 5’- HO-MsLsMsLsMsFsXFpMsMsdCsDqMpFsMqFsMsFsMsFsMsLsMsLsM-OH -
3’

(8) 5'- HO-MsLsMsLsMsFsXFsMpMsdCsDqMsFsMqFsMsFsMsFsMsLsMsLsM-OH -
3'

(9) 5'- HO-MsLsMsLpMsFsXFsMsMsdCsDqMpFsMqFsMsFsMsFsMsLpMsLsM-OH -
3'

(10) 5'- HO-MsLsMsLpMsFsXFsMsMsdCsDqMpFsMqFsMpFsMsFsMsLpMsLsM-
OH -3'

(11) 5'- HO-MsLsMsLpMsFsXFsMsMsdCsDqMpFsMqFsMsFsMpFsMsLpMsLsM-OH
-3'

(12) 5'- HO-MsLsMsLpMsFsXFsMsMsdCsDqMpFsMqFsMsFsMsFsMpLpMsLsM-
OH -3'

(13) 5'- HO-MsLsMsLsMsFsXFsMsMsdCsDqMpFsMqFsMsFsMsFsMsMsMsLsM-OH
-3'

(14) 5'- HO-MsLsMsLsMsFsXFsMsMsdCsDqMpFsMqFsMpFsMsFsMsMsMsLsM-
OH -3'

(15) 5'- HO-MsLsMsLsMsFsXFsMsMsdCsDqMpFsMqFsMsFsMpFsMsMsMsLsM-
OH -3'

(16) 5'- HO-MsLsMsLsMsFsXFsMsMsdCsDqMpFsMqFsMsFsMsFsMpMsMsLsM-
OH -3'

(17) 5'- HO-MsLsMsLsMsFsXFsMsMsdCsDqMpFsMqFsMpFsMpFsMsMsMsLsM-

OH -3'

(18) 5’- HO-MsLsMsLsMsFsXFsMsMsdCsDqMpFsMqFsMpFsMsFsMpMsMsLsM-
OH -3’

(19) 5'- HO-MsLsMsLsMsFsXFsMsMsdCsDqMpFsMqFsMsFsMpFsMpMsMsLsM-
OH -3'

(20) 5'- HO-MsLsMsLsMsFsXFsMsMsdCsDqMpFsMqFsMpFsMpFsMpMsMsLsM-
OH -3'

In the Formulas, D represents a 2'-deoxyribonucleoside residue, F represents a 2'-fluoro-2'-deoxyribonucleoside residue, L represents a bridged nucleoside residue, M represents a 2'-O-methyl- ribonucleoside residue, dC represents a target nucleoside residue, a 2'-deoxycytidine residue. X indicates that one nucleoside residue is missing from the target RNA. p represents a phosphate residue, s represents a thiophosphate residue, q represents an alkylphosphonate residue, an alkyl phosphate residue, or a substituted phosphoramide residue. HO- represents a hydroxyl group at the 5' end, and -OH represents a hydroxyl group at the 3' end.

(2-9) The oligonucleotide or a pharmaceutically acceptable salt thereof according to any one of (2-1) to (2-7), the oligonucleotide comprising a modified oligonucleotide having a chemical modification pattern represented by any one of the following Formulas (1a) to (20a)

(1a) 5'- HO-MsLsMsLsMsFsXFsMsMsdCsDpmMsFsMpmFsMsFsMsFsMsLsMsLsM-
OH -3'

(2a) 5'- HO-MsLsMsLsMsFsXFsMsMsdCsDpmMsFsMpmFsMsFsMsFsMsMsMsLsM-
OH -3'

(3a) 5'- HO-MsLsMsMsMsFsXFsMsMsdCsDpmMsFsMpmFsMsFsMsFsMsLsMsLsM-
OH -3'

(4a) 5’-HO-MsLsMsMsMsFsXFsMsMsdCsDpmMsFsMpmFsMsFsMsFsMsMsMsLsM-
OH -3’

(5a) 5’-HO-LsMsMsMsMsFsXFsMsMsdCsDpmMsFsMpmFsMsFsMsFsMsMsMsLsM-
OH -3’

(6a) 5’- HO-MsLsMsLsMsFsXFsMsMsdCsDrMpFsMrFsMsFsMsFsMsLsMsLsM-OH -
3’

(7a) 5’- HO-MsLsMsLsMsFsXFpMsMsdCsDrMpFsMrFsMsFsMsFsMsLsMsLsM-OH -
3’

(8a) 5'- HO-MsLsMsLsMsFsXFsMpMsdCsDrMsFsMrFsMsFsMsFsMsLsMsLsM-OH -
3'

(9a) 5'- HO-MsLsMsLpMsFsXFsMsMsdCsDrMpFsMrFsMsFsMsFsMsLpMsLsM-OH
-3'

(10a) 5'- HO-MsLsMsLpMsFsXFsMsMsdCsDrMpFsMrFsMpFsMsFsMsLpMsLsM-
OH -3'

(11a) 5'- HO-MsLsMsLpMsFsXFsMsMsdCsDrMpFsMrFsMsFsMpFsMsLpMsLsM-
OH -3'

(12a) 5'- HO-MsLsMsLpMsFsXFsMsMsdCsDrMpFsMrFsMsFsMsFsMpLpMsLsM-
OH -3'

(13a) 5'- HO-MsLsMsLsMsFsXFsMsMsdCsDrMpFsMrFsMsFsMsFsMsMsMsLsM-
OH -3'

(14a) 5'- HO-MsLsMsLsMsFsXFsMsMsdCsDrMpFsMrFsMpFsMsFsMsMsMsLsM-
OH -3'

(15a) 5'- HO-MsLsMsLsMsFsXFsMsMsdCsDrMpFsMrFsMsFsMpFsMsMsMsLsM-
OH -3'

(16a) 5'- HO-MsLsMsLsMsFsXFsMsMsdCsDrMpFsMrFsMsFsMsFsMpMsMsLsM-
OH -3'

(17a) 5’- HO-MsLsMsLsMsFsXFsMsMsdCsDrMpFsMrFsMpFsMpFsMsMsMsLsM-
OH -3’

(18a) 5'- HO-MsLsMsLsMsFsXFsMsMsdCsDrMpFsMrFsMpFsMsFsMpMsMsLsM-
OH -3'

(19a) 5'- HO-MsLsMsLsMsFsXFsMsMsdCsDrMpFsMrFsMsFsMpFsMpMsMsLsM-
OH -3'

(20a) 5'- HO-MsLsMsLsMsFsXFsMsMsdCsDrMpFsMrFsMpFsMpFsMpMsMsLsM-
OH -3'

In the Formulas, D represents a 2'-deoxyribonucleoside residue, F represents a 2'-fluoro-2'-deoxyribonucleoside residue, L represents a bridged nucleoside residue, M represents a 2'-O-methyl- ribonucleoside residue, dC represents a target nucleoside residue, a 2'-deoxycytidine residue. X indicates that one nucleoside residue is missing from the target RNA. p represents a phosphate residue, s represents a thiophosphate residue, q represents an alkylphosphonate residue, an alkyl phosphate residue, or a substituted phosphoramide residue. HO- represents a hydroxyl group at the 5' end, and -OH represents a hydroxyl group at the 3' end.

(2-10) The oligonucleotide or a pharmaceutically acceptable salt thereof according to any one of (2-1) to (2-7), the oligonucleotide comprising a modified oligonucleotide having a chemical modification pattern represented by any one of the following Formulas (1) to (22).

(1) 5'- HO-MsLsMsLsMsFsXFsMsMsdCsDqMsFsMqFsMsFsMsFsMsLsMsLsM-OH -
3'

(2) 5’- HO-MsLsMsLsMsFsXFsMsMsdCsDqMsFsMqFsMsFsMsFsMsMsMsLsM-OH -
3’

(3) 5’- HO-MsLsMsMsMsFsXFsMsMsdCsDqMsFsMqFsMsFsMsFsMsLsMsLsM-OH -
3’

(4) 5’- HO-MsLsMsMsMsFsXFsMsMsdCsDqMsFsMqFsMsFsMsFsMsMsMsLsM-OH
-3’

(5) 5’- HO-LsMsMsMsMsFsXFsMsMsdCsDqMsFsMqFsMsFsMsFsMsMsMsLsM-OH
-3’

(6) 5'- HO-MsLsMsLsMsFsXFsMsMsdCsDqMpFsMqFsMsFsMsFsMsLsMsLsM-OH -
3'

(7) 5'- HO-MsLsMsLsMsFsXFpMsMsdCsDqMpFsMqFsMsFsMsFsMsLsMsLsM-OH -
3'

(8) 5’- HO-MsLsMsLsMsFsXFsMpMsdCsDqMsFsMqFsMsFsMsFsMsLsMsLsM-OH -
3’

(9) 5'- HO-MsLsMsLpMsFsXFsMsMsdCsDqMpFsMqFsMsFsMsFsMsLpMsLsM-OH -

3'

(10) 5'- HO-MsLsMsLpMsFsXFsMsMsdCsDqMpFsMqFsMpFsMsFsMsLpMsLsM-
OH -3'

(11) 5'- HO-MsLsMsLpMsFsXFsMsMsdCsDqMpFsMqFsMsFsMpFsMsLpMsLsM-OH
-3'

(12) 5'- HO-MsLsMsLpMsFsXFsMsMsdCsDqMpFsMqFsMsFsMsFsMpLpMsLsM-
OH -3'

(13) 5'- HO-MsLsMsLsMsFsXFsMsMsdCsDqMpFsMqFsMsFsMsFsMsMsMsLsM-OH
-3'

(14) 5'- HO-MsLsMsLsMsFsXFsMsMsdCsDqMpFsMqFsMpFsMsFsMsMsMsLsM-
OH -3'

(15) 5’- HO-MsLsMsLsMsFsXFsMsMsdCsDqMpFsMqFsMsFsMpFsMsMsMsLsM-
OH -3’

(16) 5'- HO-MsLsMsLsMsFsXFsMsMsdCsDqMpFsMqFsMsFsMsFsMpMsMsLsM-
OH -3'

(17) 5'- HO-MsLsMsLsMsFsXFsMsMsdCsDqMpFsMqFsMpFsMpFsMsMsMsLsM-
OH -3'

(18) 5'- HO-MsLsMsLsMsFsXFsMsMsdCsDqMpFsMqFsMpFsMsFsMpMsMsLsM-
OH -3'

(19) 5'- HO-MsLsMsLsMsFsXFsMsMsdCsDqMpFsMqFsMsFsMpFsMpMsMsLsM-
OH -3'

(20) 5'- HO-MsLsMsLsMsFsXFsMsMsdCsDqMpFsMqFsMpFsMpFsMpMsMsLsM-
OH -3'

(21) 5'- HO-MsLsMsLsMsFsXFsMsMsdCsDqMpFsMsFsMsFsMsFsMsLsMsLsM-OH
-3'

(22) 5’- HO-MsLsMsLsMsFsXFsMsMsdCsDqMpFsMsFsMsFsMsFsMsMsMsLsM-OH
-3’

In the Formulas, D represents a 2'-deoxyribonucleoside residue, F represents a 2'-fluoro-2'-deoxyribonucleoside residue, L represents a bridged nucleoside residue, M represents a 2'-O-methyl- ribonucleoside residue, dC represents a target nucleoside residue, a 2'-deoxycytidine residue. X indicates that one nucleoside residue is missing from the target RNA. p represents a phosphate residue, s represents a thiophosphate residue, q represents an alkylphosphonate residue, an alkyl phosphate residue, or a substituted phosphoramide residue. HO- represents a hydroxyl group at the 5' end, and -OH represents a hydroxyl group at the 3' end.

(2-11) The oligonucleotide or a pharmaceutically acceptable salt thereof according to any one of (2-1) to (2-7), the oligonucleotide comprising a modified oligonucleotide having a chemical modification pattern represented by any one of the following Formulas (1a) to (22a)

(1a) 5'- HO-MsLsMsLsMsFsXFsMsMsdCsDpmMsFsMpmFsMsFsMsFsMsLsMsLsM-
OH -3'

(2a) 5'- HO-MsLsMsLsMsFsXFsMsMsdCsDpmMsFsMpmFsMsFsMsFsMsMsMsLsM-
OH -3'

(3a) 5’- HO-MsLsMsMsMsFsXFsMsMsdCsDpmMsFsMpmFsMsFsMsFsMsLsMsLsM-
OH -3’

(4a) 5’-HO-MsLsMsMsMsFsXFsMsMsdCsDpmMsFsMpmFsMsFsMsFsMsMsMsLsM-
OH -3’

(5a) 5’-HO-LsMsMsMsMsFsXFsMsMsdCsDpmMsFsMpmFsMsFsMsFsMsMsMsLsM-
OH -3’

(6a) 5'- HO-MsLsMsLsMsFsXFsMsMsdCsDrMpFsMrFsMsFsMsFsMsLsMsLsM-OH -
3'

(7a) 5'- HO-MsLsMsLsMsFsXFpMsMsdCsDrMpFsMrFsMsFsMsFsMsLsMsLsM-OH -
3'

(8a) 5’- HO-MsLsMsLsMsFsXFsMpMsdCsDrMsFsMrFsMsFsMsFsMsLsMsLsM-OH -
3’

(9a) 5’- HO-MsLsMsLpMsFsXFsMsMsdCsDrMpFsMrFsMsFsMsFsMsLpMsLsM-OH
-3’

(10a) 5'- HO-MsLsMsLpMsFsXFsMsMsdCsDrMpFsMrFsMpFsMsFsMsLpMsLsM-
OH -3'

(11a) 5'- HO-MsLsMsLpMsFsXFsMsMsdCsDrMpFsMrFsMsFsMpFsMsLpMsLsM-
OH -3'

(12a) 5'- HO-MsLsMsLpMsFsXFsMsMsdCsDrMpFsMrFsMsFsMsFsMpLpMsLsM-
OH -3'

(13a) 5'- HO-MsLsMsLsMsFsXFsMsMsdCsDrMpFsMrFsMsFsMsFsMsMsMsLsM-
OH -3'

(14a) 5'- HO-MsLsMsLsMsFsXFsMsMsdCsDrMpFsMrFsMpFsMsFsMsMsMsLsM-
OH -3'

(15a) 5'- HO-MsLsMsLsMsFsXFsMsMsdCsDrMpFsMrFsMsFsMpFsMsMsMsLsM-
OH -3'

(16a) 5’- HO-MsLsMsLsMsFsXFsMsMsdCsDrMpFsMrFsMsFsMsFsMpMsMsLsM-
OH -3’

(17a) 5’- HO-MsLsMsLsMsFsXFsMsMsdCsDrMpFsMrFsMpFsMpFsMsMsMsLsM-
OH -3’

(18a) 5'- HO-MsLsMsLsMsFsXFsMsMsdCsDrMpFsMrFsMpFsMsFsMpMsMsLsM-
OH -3'

(19a) 5'- HO-MsLsMsLsMsFsXFsMsMsdCsDrMpFsMrFsMsFsMpFsMpMsMsLsM-
OH -3'

(20a) 5'- HO-MsLsMsLsMsFsXFsMsMsdCsDrMpFsMrFsMpFsMpFsMpMsMsLsM-
OH -3'

(21a) 5’- HO-MsLsMsLsMsFsXFsMsMsdCsDrMpFsMsFsMsFsMsFsMsLsMsLsM-OH
-3’

(22a) 5'- HO-MsLsMsLsMsFsXFsMsMsdCsDrMpFsMsFsMsFsMsFsMsMsMsLsM-
OH -3'

In the Formulas, D represents a 2'-deoxyribonucleoside residue, F represents a 2'-fluoro-2'-deoxyribonucleoside residue, L represents a bridged nucleoside residue, M represents a 2'-O-methyl- ribonucleoside residue, dC represents a target nucleoside residue, a 2'-deoxycytidine residue. X indicates that one nucleoside residue is missing from the target RNA. p represents a phosphate residue, s represents a thiophosphate residue, q represents an alkylphosphonate residue, an alkyl phosphate residue, or a substituted phosphoramide residue. HO- represents a hydroxyl group at the 5' end, and -OH represents a hydroxyl group at the 3' end.

### EFFECTS OF THE INVENTION

One aspect of the present invention makes it possible to provide an oligonucleotide that is capable of inducing the editing activity of ADAR in a cell, and has excellent stability in a living organism.

### BRIEF DESCRIPTION OF THE DRAWINGS

Fig. 1A is a graph showing the editing rate for target RNA hACTB by an oligonucleotide in a cell-free system.
Fig. 1B is a graph showing the editing rate for target RNA hACTB by an oligonucleotide in a cell.
Fig. 2A is a graph showing the editing rate for target RNA hACTB by an oligonucleotide in a cell-free system.
Fig. 2B is a graph showing the editing rate for target RNA hACTB by an oligonucleotide in a cell.
Fig. 3A is a graph showing the editing rate for target RNA hACTB by an oligonucleotide in a cell-free system.
Fig. 3B is a graph showing the editing rate for target RNA hACTB by an oligonucleotide in a cell-free system.
Fig. 3C is a graph showing the editing rate for target RNA hACTB by an oligonucleotide in a cell.
Fig. 3D is a graph showing the editing rate for target RNA hACTB by an oligonucleotide in a cell-free system.
Fig. 3E is a graph showing the editing rate for target RNA hACTB by an oligonucleotide in a cell.
Fig. 4A is a graph showing the editing rate for target RNA hACTB by an oligonucleotide in a cell-free system.
Fig. 4B is a graph showing the editing rate for target RNA hACTB by an oligonucleotide in a cell-free system.
Fig. 4C is a graph showing the editing rate for target RNA hACTB by an oligonucleotide in a cell.
Fig. 4D is a graph showing the editing rate for target RNA hACTB by an oligonucleotide in a cell.
Fig. 5 is a graph showing the editing rate for target RNA hACTB by an oligonucleotide in a cell.
Fig. 6A is a graph showing the editing rate for target RNA hACTB by an oligonucleotide in a cell-free system.
Fig. 6B is a graph showing the editing rate for target RNA hACTB by an oligonucleotide in a cell.
Fig. 7A is a graph showing the editing rate for target RNA hACTB by an oligonucleotide in a cell-free system.
Fig. 7B is a graph showing the editing rate for target RNA hACTB by an oligonucleotide in a cell.
Fig. 8A is a graph showing the editing rate for target RNA hACTB by an oligonucleotide in a cell-free system.
Fig. 8B is a graph showing the editing rate for target RNA hACTB by an oligonucleotide in a cell.
Fig. 9A is a graph showing the editing rate for target RNA hACTB by an oligonucleotide in a cell-free system.
Fig. 9B is a graph showing the editing rate for target RNA hACTB by an oligonucleotide in a cell.
Fig. 10A is a graph showing the editing rate for target RNA hACTB by an oligonucleotide in a cell-free system.
Fig. 10B is a graph showing the editing rate for target RNA hACTB by an oligonucleotide in a cell.
Fig. 11A is a graph showing the editing rate for target RNA hACTB by an oligonucleotide in a cell-free system.
Fig. 11B is a graph showing the editing rate for target RNA hACTB by an oligonucleotide in a cell.
Fig. 11C is a graph showing the editing rate for target RNA hACTB by an oligonucleotide in a cell-free system.
Fig. 11D is a graph showing the editing rate for target RNA hACTB by an oligonucleotide in a cell.
Fig. 12A is a graph showing the editing rate for target RNA A1AT by an oligonucleotide in a cell-free system.
Fig. 12B is a graph showing the editing rate for target RNA A1AT by an oligonucleotide in a cell.
Fig. 13A is a graph showing the editing rate for target RNA mGAPDH by an oligonucleotide in a cell-free system.
Fig. 13B is a graph showing the editing rate for target RNA mGAPDH by an oligonucleotide in a cell.
Fig. 14 is a graph showing the editing rate for target RNA mGAPDH by an oligonucleotide in a cell.

### DETAILED DESCRIPTION

The term "step" as used herein comprises not only an independent step but also a step not clearly distinguishable from another step as long as the intended purpose of the step is achieved. If multiple substances correspond to a component in a composition, the content of the component in the composition means the total amount of the multiple substances present in the composition unless otherwise specified. Furthermore, with respect to an upper limit and a lower limit of numerical ranges described herein, the numerical values exemplified as the numerical range can be freely selected and combined.

Additionally, the term "oligonucleotide" as used herein may be understood as an oligonucleotide molecule or an oligonucleotide residue, depending on the context. Furthermore, in the oligonucleotide, nucleoside residues constituting the oligonucleotide may be linked by a natural phosphoric residue, or may be linked by a phosphorus-containing linking group such as a thiophosphoric residue, an alkylphosphonic residue, an alkyl phosphate residue, or a substituted phosphoramide residue (including an N-sulfonyl substituted phosphoramide residue and an N-alkyl substituted phosphoramide residue). Herein, linking groups that link nucleoside residues such as ordinary phosphoric residues, thiophosphoric residues, alkylphosphonic residues, alkyl phosphate residues, and substituted phosphoramide residues are collectively referred to as phosphorus-containing linking groups.

Below, embodiments of the present invention are described in detail. However, the embodiments described below exemplify an oligonucleotide or a pharmaceutically acceptable salt thereof for embodying the technical idea of the present invention, and the present invention is not limited to the oligonucleotide or a pharmaceutically acceptable salt thereof described below.

### Target-editing oligonucleotide

The oligonucleotide that induces site-specific editing for a target RNA (hereinafter, the oligonucleotide is also referred to as a target-editing oligonucleotide or a target-editing guide RNA) includes: a first oligonucleotide that identifies a target RNA; and a second oligonucleotide linked to the 5' side of the first oligonucleotide. The first oligonucleotide may be composed of: a target-corresponding nucleoside residue corresponding to an adenosine residue in the target RNA; an oligonucleotide containing 3 to 6 residues, linked to the 5' side of the target-corresponding nucleoside residue, and having a base sequence complementary to the target RNA; and an oligonucleotide containing 10 to 24 residues, linked to the 3' side of the target-corresponding nucleoside residue, and having a base sequence complementary to the target RNA. In the oligonucleotide linked to the 3' side of the target-corresponding nucleoside residue in the first oligonucleotide, at least one phosphorus-containing linking group selected from the group consisting of a phosphorus-containing linking group linking a 1st nucleoside residue and a 2nd nucleoside residue and a phosphorus-containing linking group linking a 4th nucleoside residue and a 5th nucleoside residue, counting in the 3' direction from the target-corresponding nucleoside residue, may be at least one selected from the group consisting of an alkylphosphonic residue, an alkyl phosphate residue, and a substituted phosphoramide residue. At the 3' end of the second oligonucleotide, a nucleoside residue corresponding to a nucleoside residue of the target RNA may be deleted, or, at the 3' end, the second oligonucleotide may have a nucleoside residue that does not form a complementary pair with the nucleoside residue of the target RNA. The second oligonucleotide may contain 2 to 10 residues, and nucleoside residues at least other than at the 3' end of the second oligonucleotide may form a double-stranded structure complementary to the target RNA. At least one of the first oligonucleotide or the second oligonucleotide may include a phosphorothioate bond.

The second oligonucleotide constituting the target-editing oligonucleotide has a base sequence in which a nucleoside residue does not form a complementary pair with one nucleoside residue of the target RNA, and nucleoside residues corresponding to others than that one nucleoside residue form a double-stranded structure complementary to the target RNA. That is, the second oligonucleotide has a base sequence that forms an incomplete double-stranded structure with the target RNA. The target-editing guide RNA in which a short-chain second oligonucleotide having such a characteristic base sequence is on the 5' side of the first oligonucleotide that identifies the target RNA can preferentially induce the editing activity of ADAR in a cell. This can be considered because, for example, the presence of a nucleoside residue that does not form a complementary pair works advantageously for the recruitment of ADAR (for example, ADAR1). Additionally, in the oligonucleotide linked to the 3' side of the target-corresponding nucleoside residue, at least one phosphorus-containing linking group selected from the group consisting of a phosphorus-containing linking group linking a 1st nucleoside residue and a 2nd nucleoside residue and a phosphorus-containing linking group linking a 4th nucleoside residue and a 5th nucleoside residue, counting in the 3' direction from the target-corresponding nucleoside residue, is at least one selected from the group consisting of an alkylphosphonic residue, an alkyl phosphate residue, and a substituted phosphoramide residue. Thus, the target-editing nucleotide has excellent stability in a living organism, and can exhibit excellent site-specific editing activity for the target RNA.

The target-editing oligonucleotide of the present embodiment is shorter than a conventional target-editing oligonucleotide, and allows the production cost to be simply reduced accordingly. Additionally, the target-editing oligonucleotide of the present embodiment makes chemical synthesis easier, and makes it easier to apply, to the target-editing oligonucleotide, a modified nucleotide such as an existing artificial nucleic acid used in nucleic acid drug development or the like. This makes it possible to provide a further highly functional target-editing oligonucleotide by increasing intracellular degradation resistance, increasing cell introduction efficiency, or the like. Furthermore, the fact that the target-editing oligonucleotide is constituted by the minimum number of residues necessary for editing induction makes it possible to inhibit off-target editing at positions other than the adenosine residue, which is an editing target, while maintaining specificity for the target RNA.

The target-editing oligonucleotide may, for example, preferentially recruit, to the target RNA, ADAR1 among ADARs that catalyze target editing, whereby site-specific editing for the target RNA may be efficiently induced. ADAR is an enzyme that converts an adenosine residue in double-stranded RNA into an inosine residue by a hydrolytic deamination reaction, and is widely present in mammalian cells. The inosine residue is similar in structure to a guanosine residue, and thus, is translated as a guanosine residue during translation of RNA information, with the result that the RNA information is edited. When such RNA editing occurs in a portion encoding an amino acid, amino acid substitution or the like occurs even though there is no DNA mutation on the genome, and the function of the target gene can be controlled.

When introduced into a mammalian cell, the target-editing oligonucleotide may preferentially recruit ADAR1 existing in the cell to the target RNA to induce site-specific editing for the target RNA. Here, being preferential to ADAR1 may mean, for example, that in vitro, the ratio of the editing inducing activity for ADAR1 to the editing inducing activity for ADAR2 exceeds 1, and preferably may mean 1.5 or more, 2 or more, or 5 or more. Additionally, the editing inducing activity for ADAR1 and ADAR2 in a cell refers to a case where the editing induction efficiency for the same target editing site is higher in ADAR1 when an ADAR1 expression plasmid and an ADAR2 expression plasmid constructed using the same expression vector are transfected into cells under the same conditions and expressed.

The first oligonucleotide contained in the target-editing oligonucleotide identifies the target RNA. That is, the first oligonucleotide contains a base sequence complementary to a base sequence of the target RNA, and may form a complementary strand with the target RNA. The target RNA is not particularly limited as long as it contains an adenosine residue to be edited, may be either cellular RNA or viral RNA, and is usually an mRNA precursor (including pre-mRNA) or mRNA that encodes a protein. The editing site in the target RNA may be present in a non-translated region, a splice region, an exon, an intron, or any region affecting the stability, structure, or function of RNA. The target RNA may also contain a mutation to be corrected or altered. Alternatively, the target RNA may be mutated such that its sequence encodes a phenotype different from the natural type. Additionally, the residue linked to the 5' side of the adenosine residue which is an editing target in the target RNA is not particularly limited, but may preferably be a cytidine residue or a uridine residue.

The target RNA is preferably an RNA encoding a protein. Specific examples of the protein to be encoded include phosphorylated proteins involved in signal transduction, such as a serotonin receptor, glutamate receptor, membrane potential-dependent potassium channel, STAT3, NFkBIA, and MAPK14.

The target-editing oligonucleotide can be applied to, for example, the treatment of genetic diseases. The genetic disease may be, for example, a disease that can be treated by converting an adenosine residue in a target RNA into an inosine residue. The genetic disease may also be, for example, a genetic disease caused by a mutation from a guanosine residue to an adenosine residue in a gene. Examples of genetic diseases include cystic fibrosis, albinism, alpha-1 antitrypsin deficiency, Alzheimer's disease, amyotrophic lateral sclerosis, asthma, beta-thalassemia, CADASIL syndrome, Charcot-Marie-Tooth disease, chronic obstructive pulmonary disease (COPD), distal spinal muscular atrophy (DSMA), Duchenne/Becker muscular dystrophy, dystrophic epidermolysis bullosa, Epidermolysis bullosa, Fabry disease, Factor V Leiden related disorders, familial adenomatous, polyposis, galactosemia, Gaucher disease, glucose-6-phosphate dehydrogenase deficiency, hemophilia, hereditary hemochromatosis, Hunter syndrome, Huntington's disease, Hurler syndrome, inflammatory bowel disease (IBD), hereditary multi-agglutination syndrome, Leber congenital amaurosis, Lesch-Nyhan syndrome, Lynch syndrome, Marfan syndrome, mucopolysaccharidosis, muscular dystrophy, myotonic dystrophy types I and II, neurofibromatosis, Niemann-Pick disease types A, B and C, NY-eso1 related pancreatic cancer, Parkinson's disease, Peutz-Jeghers syndrome, phenylketonuria, Pompe disease, primary ciliary dyskinesia, prothrombin mutation related diseases such as prothrombin G20210A mutation, pulmonary hypertension, retinitis pigmentosa, Sandhoff disease, severe combined immunodeficiency syndrome (SCID), sickle cell anemia, spinal muscular atrophy, Stargardt disease, Tay-Sachs disease, Usher syndrome, X-linked immunodeficiency, and various forms of cancer (for example, BRCA1 and 2 related breast cancer and ovarian cancer).

Specific examples of genetic diseases include citrullinemia type I (ASS1; known mutations include Gly390Arg c.1168G>A (rs121908641)), citrullinemia type II (SLC25A13; known mutations include IVS11+1G>A (rs80338722)), hemophilia (Factor V Leiden: known mutations include Arg506Gln c.1601G>A (rs6025)), primary hyperoxaluria (AGXT; known mutations include G170R c.508G>A (rs121908529)), distal myopathy (GNE; known mutations include R266Q c.797G>A (rs121908622)), cystic fibrosis (CFTR; known mutations include W1282X c.3846G>A (rs77010898)), homocystinuria (CBS; known mutations include Asp444Asn c.1330G>A (rs28934891)), Hurler syndrome: mucopolysaccharidosis (IDUA (SLC26A1; known mutations include Gly51Asp c.152G>A (rs794726877))), Alexander disease (GFAP; known mutations include R239H, c.716G>A (rs59565950)), retinitis pigmentosa (EYS; known mutations include W2940X, c.7919G>A (rs527236066)), phenylketonuria (PAH; known mutations include R261Q c.782G>A (rs5030849)), hemochromatosis (HFE; known mutations include C282Y c.845G>A (rs1800562)), and Gilbert syndrome (UGT1A1; known mutations include G71R c.211.G>A (rs4148323)). Note that the names in parentheses are target gene names and examples of gene mutations thereof.

The c.1168G>A mutation (rs121908641) in the ASS1 gene, causative of citrullinemia type I, is a mutation from G to A at the 1524th nucleotide of Homo sapiens argininosuccinate synthase 1 (ASS1), transcript variant 1, mRNA (GenBank Accession No: NM_000050.4). In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 1500 to 1540 of the ASS1 mRNA, preferably completely complementary to a region of 20 to 29 consecutive bases contained in positions 1504 to 1534, and more preferably completely complementary to a region of 20 to 25 consecutive bases contained in positions 1510 to 1534.

The c.1601G>A mutation (rs6025) in the Factor V gene, causative of hemophilia (thrombosis), is a mutation from G to A at the 1696th nucleotide of Homo sapiens coagulation factor V (F5), mRNA (GenBank Accession No: NM_000130.5). In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 1672 to 1712 of the F5 mRNA, preferably completely complementary to a region of 20 to 29 consecutive bases contained in positions 1676 to 1706, and more preferably completely complementary to a region of 20 to 25 consecutive bases contained in positions 1682 to 1706.

Abnormalities in post-transcriptional modification of the GRIA2 gene can cause amyotrophic lateral sclerosis (ALS). An abnormality in A-to-I RNA editing at the 2135th adenosine of Homo sapiens glutamate ionotropic receptor AMPA type subunit 2 (GRIA2), transcript variant 1, mRNA (GenBank Accession No: NM_000826.4) may correspond to the abnormality in the post-transcriptional modification. In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 2111 to 2151 of the GRIA2 mRNA, preferably completely complementary to a region of 20 to 29 consecutive bases contained in positions 2115 to 2145, and more preferably completely complementary to a region of 20 to 25 consecutive bases contained in positions 2121 to 2145.

The c.1561G>A mutation (rs137852959) in the PANK2 gene, causative of pantothenate kinase-associated neurodegeneration, is a mutation from G to A at the 1728th nucleotide of Homo sapiens pantothenate kinase 2 (PANK2), transcript variant 1, mRNA (GenBank Accession No: NM_153638.3). In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 1704 to 1744 in the PANK2 mRNA, preferably completely complementary to a region of 20 to 29 consecutive bases contained in positions 1708 to 1738, and more preferably completely complementary to a region of 20 to 25 consecutive bases contained in positions 1714 to 1738.

The c.1330G>A mutation (rs28934891) in the CBS gene, causative of homocystinuria, is a mutation from G to A at the 1575th nucleotide of Homo sapiens cystathionine beta-synthase (CBS), transcript variant 1, mRNA (GenBank Accession No: NM_000071.2). In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 1551 to 1591 of the CBS mRNA, preferably completely complementary to a region of 20 to 29 consecutive bases contained in positions 1555 to 1585, and more preferably completely complementary to a region of 20 to 25 consecutive bases contained in positions 1561 to 1585.

The c.413G>A mutation (rs74315342) in the NPHS2 gene, causative of focal segmental glomerulosclerosis, is a mutation from G to A at the 497th nucleotide of Homo sapiens NPHS2 stomatin family member, podocin (NPHS2), transcript variant 1, mRNA (GenBank Accession No: NM_014625.4). In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 473 to 513 of the NPHS2 mRNA, preferably completely complementary to a region of 20 to 29 consecutive bases contained in positions 477 to 507, and more preferably completely complementary to a region of 20 to 25 consecutive bases contained in positions 483 to 507.

The c.1096G>A mutation (rs28929474) in the SERPINA1 gene, causative of alpha-1 antitrypsin deficiency, is a mutation from G to A at the 1143rd nucleotide of Homo sapiens serpin family A member 1 (SERPINA1), transcript variant 1, mRNA (GenBank Accession No: NM_000295.5). In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 1119 to 1159 of the SERPINA1 mRNA, preferably completely complementary to a region of 20 to 29 consecutive bases contained in positions 1123 to 1153, and more preferably completely complementary to a region of 20 to 25 consecutive bases contained in positions 1129 to 1153.

The c.782G>A mutation (rs5030849) in the PAH gene, causative of phenylketonuria, is a mutation from G to A at the 896th nucleotide of Homo sapiens phenylalanine hydroxylase (PAH), transcript variant 1, mRNA (GenBank Accession No: NM_000277.3). In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 872 to 912 of the PAH mRNA, preferably completely complementary to a region of 20 to 29 consecutive bases contained in positions 876 to 906, and more preferably completely complementary to a region of 20 to 25 consecutive bases contained in positions 882 to 906.

The c.940+1G>A mutation (rs765249238) of the SLCO2A1 gene, causative of pachydermoperiostosis, is a mutation from G to A at the 81034th nucleotide on the pre-mRNA of Homo sapiens solute carrier organic anion transporter family member 2A1 (SLCO2A1), mRNA (GenBank Accession No: NM_005630.3) (the editing target is located on the (-) strand at position 133948892 of chromosome 3). In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 81010 to 81050 of the SLCO2A1 pre-mRNA, preferably completely complementary to a region of 20 to 29 consecutive bases contained in positions 81014 to 81044, and more preferably completely complementary to a region of 20 to 25 consecutive bases contained in positions 81020 to 81044.

The c.716G>A mutation (rs59565950) in the GFAP gene, causative of Alexander disease, is a mutation from G to A at the 2168th nucleotide of Homo sapiens glial fibrillary acidic protein (GFAP), transcript variant 1, mRNA (GenBank Accession No: NM_002055.5). In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 2144 to 2184 of the GFAP mRNA, preferably completely complementary to a region of 20 to 29 consecutive bases contained in positions 2148 to 2178, and more preferably completely complementary to a region of 20 to 25 consecutive bases contained in positions 2154 to 2178.

The c.508G>A mutation (rs121908529) of the AGXT gene, causative of primary hyperoxaluria, is a mutation from G to A at the 550th nucleotide of Homo sapiens alanine-glyoxylate and serine-pyruvate aminotransferase (AGXT), mRNA (GenBank Accession No: NM_000030.3). In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 526 to 566 of the AGXT mRNA, preferably completely complementary to a region of 20 to 29 consecutive bases contained in positions 530 to 560, and more preferably completely complementary to a region of 20 to 25 consecutive bases contained in positions 536 to 560.

The c.211G>A mutation (rs4148323) of the UGT1A1 gene, causative of Gilbert syndrome, is a mutation from G to A at the 229th nucleotide of Homo sapiens UDP glucuronosyltransferase family 1 member A1 (UGT1A1), mRNA (GenBank Accession No: NM_000463.3). In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 205 to 245 of the UGT1A1 mRNA, preferably completely complementary to a region of 20 to 29 consecutive bases contained in positions 209 to 239, and more preferably completely complementary to a region of 20 to 25 consecutive bases contained in positions 215 to 239.

The c.7919G>A mutation (rs527236066) of the EYS gene, causative of retinitis pigmentosa, is a mutation from G to A at the 8478th nucleotide of Homo sapiens eyes shut homolog (EYS), transcript variant 1, mRNA (GenBank Accession No: NM_001142800.2). In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 8454 to 8494 of the EYS mRNA, preferably completely complementary to a region of 20 to 29 consecutive bases contained in positions 8458 to 8488, and more preferably completely complementary to a region of 20 to 25 consecutive bases contained in positions 8464 to 8488.

The c.797G>A mutation (rs121908622) of the GNE gene, causative of distal myopathy, is a mutation from G to A at the 924th nucleotide of Homo sapiens glucosamine (UDP-N-acetyl)-2-epimerase/N-acetylmannosamine kinase (GNE), transcript variant 1, mRNA (GenBank Accession No: NM_001128227.3). In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 900 to 940 of the GNE mRNA, preferably completely complementary to a region of 20 to 29 consecutive bases contained in positions 904 to 934, and more preferably completely complementary to a region of 20 to 25 consecutive bases contained in positions 910 to 934.

The c.845G>A mutation (rs1800562) of the HFE gene, causative of hemochromatosis, is a mutation from G to A at the 1005th nucleotide of Homo sapiens homeostatic iron regulator (HFE), transcript variant 1, mRNA (GenBank Accession No: NM_000410.3). In one aspect, the oligonucleotide targets the adenosine residue. For example, in one aspect, the oligonucleotide may have a nucleotide sequence that is other than the target-corresponding nucleoside residue, and is completely complementary to a region of 20 to 40 consecutive bases contained in positions 981 to 1021 of the HFE mRNA, preferably completely complementary to a region of 20 to 29 consecutive bases contained in positions 985 to 1015, and more preferably completely complementary to a region of 20 to 25 consecutive bases contained in positions 991 to 1015.

The first oligonucleotide is composed of: a target-corresponding nucleoside residue corresponding to an adenosine residue as an editing target in the target RNA; a 3'-side oligonucleotide containing 10 to 24 residues, linked to the 3' side of the target-corresponding nucleoside residue, and having a base sequence complementary to the corresponding base sequence of the target RNA; and a 5'-side oligonucleotide containing 3 to 6 residues, linked to the 5' side of the target-corresponding nucleoside residue, and having a base sequence complementary to the corresponding base sequence of the target RNA. The oligonucleotides linked to the 3' side and the 5' side of the target-corresponding nucleoside residue, respectively, form a double-stranded structure with the target RNA to form a complementary strand as a whole (hereinafter, also referred to as a first complementary strand), thereby identifying the target RNA and the editing target site in the target RNA. Herein, the complementary base sequence includes, in addition to a base sequence forming a Watson-Crick base pair, a base sequence forming a thermodynamically stable non-Watson-Crick wobble base pair such as a G-U base pair. All of the base pairs forming the first complementary strand, excluding the target-corresponding nucleotide residue, may be Watson-Crick base pairs, or at least a part of the first complementary strand may be wobble base pairs. In a case where the first complementary strand contains a wobble base pair, the number thereof may be 1 or 2. Hereinafter, a region composed of three residues, i.e., the target-corresponding nucleoside residue, one residue on the 5' side thereof, and one residue on the 3' side thereof, is conveniently referred to as a counter region, and a region of the first oligonucleotide other than the counter region may be referred to as a non-counter region.

In one aspect, regarding the 3'-side oligonucleotide containing 10 to 24 residues, and linked to the 3' side of the target-corresponding nucleoside residue in the first oligonucleotide, the 1st nucleoside residue on the 3' side of the target-corresponding nucleoside residue may be a nucleoside residue that does not form a complementary pair with the corresponding nucleoside residue of the target RNA, and the other nucleoside residues on the 3' side may form a complementary pair with the corresponding nucleoside residues of the target RNA to form a double-stranded structure with the target RNA. Specifically, for example, in a case where a guanosine residue is linked to the 5' side of an adenosine residue serving as an editing target of the target RNA, the 1st nucleoside residue on the 3' side of the target-corresponding nucleoside residue may be a 2'-deoxyinosine residue.

The target-corresponding nucleoside residue is a nucleoside residue corresponding to the adenosine residue serving as the editing target, and is, for example, a cytidine residue, a guanosine residue, an adenosine residue, or a derivative thereof. The target-corresponding nucleoside residue is preferably a base that does not form a base pair with the adenosine residue serving as the editing target, more preferably a cytidine residue or a derivative thereof, and still more preferably a cytidine residue. In one aspect, the target-corresponding nucleoside residue may be an N-alkylpyrimidine nucleoside residue, preferably an N-alkylcytidine residue, an N-alkyl-2'-deoxycytidine residue, an N-alkyluridine residue, or an N-alkyl-2'-deoxyuridine residue. The alkyl group in the N-alkylpyrimidine nucleoside residue may be a C₁₋₆ or C₁₋₄ alkyl group, and preferably a methyl group or an ethyl group. In some of the cases where the target-corresponding nucleoside residue is an N-alkylpyrimidine nucleoside residue, the editing-inducing activity is increased. Additionally, in the N-alkylpyrimidine nucleoside residue, the sugar moiety may be further modified, and a phosphorus-containing linking group (for example, a phosphoric residue) linked to an adjacent nucleoside residue may be further modified. Modifications of the sugar moiety and the phosphoric residue are described below.

In one aspect, the target-corresponding nucleoside residue may be a 2'-deoxycytidine residue. In the 2'-deoxycytidine residue, a phosphoric residue linked to the 2'-deoxycytidine residue may be further modified.

In the first oligonucleotide, the number of residues of the oligonucleotide linked to the 3' side of the target-corresponding nucleoside residue and having a base sequence complementary to the target RNA may be 10 or more and 24 or less, preferably 11 or more, 12 or more, or 13 or more, and preferably 22 or less, 20 or less, 18 or less, 16 or less, or 15 or less. In one aspect, the number of residues of the oligonucleotide having a base sequence complementary to the target RNA may be 12 or more and 20 or less, 12 or more and 18 or less, 12 or more and 16 or less, 14 or more and 18 or less, or 14 or more and 16 or less. In one aspect, the number of residues of the oligonucleotide having a base sequence complementary to the target RNA may be 14. In a case where the number of residues is within the above range, the selectivity for ADAR1 tends to be further increased.

In the first oligonucleotide, the 3'-side oligonucleotide linked to the 3' side of the target-corresponding nucleoside residue may have a complementary base sequence containing no mismatch base pair with respect to the target RNA. In this case, it is preferable that a guanosine residue is not linked to the 5' side of the adenosine residue serving as the editing target in the target RNA. That is, in a case where a base linked to the 5' side of the adenosine residue serving as the editing target of the target RNA is an adenosine residue, a cytidine residue, or a uridine residue, it is preferable that, in the first oligonucleotide, the oligonucleotide linked to the 3' side of the target-corresponding nucleoside residue has a complementary base sequence containing no mismatch base pair with respect to the target RNA.

In the first oligonucleotide, the 3'-side oligonucleotide linked to the 3' side of the target-corresponding nucleoside residue may optionally contain a base noncomplementary to a base sequence of the target RNA. For example, in a target RNA in which a guanosine residue is linked to the 5' side of an adenosine residue serving as an editing target, the editing-inducing activity may decrease. Even in this case, the presence of the first oligonucleotide having a base sequence containing a base noncomplementary to the guanosine residue can increase the editing-inducing activity. The base noncomplementary to the guanosine residue may be, for example, a guanosine residue. That is, in a target-editing oligonucleotide for a target RNA in which a guanosine residue is linked to the 5' side of an adenosine residue serving as an editing target, a guanosine residue may be linked to the 3' side of the target-corresponding nucleoside residue.

In the first oligonucleotide, the 3'-side oligonucleotide linked to the 3' side of the target-corresponding nucleoside residue may optionally form a non-Watson-Crick type wobble base pair with the corresponding nucleoside residue of the target RNA, and the 3'-side oligonucleotide linked to the 3' side of the target-corresponding nucleoside residue may optionally contain a 2'-deoxynucleoside residue. Thereby, the editing target activity is increased in some cases.

For example, in a case where a cytidine residue is linked to the 5' side of the adenosine residue serving as the editing target in the target RNA, a nucleoside residue having a hypoxanthine residue as a base may be linked to the 3' side of the target-corresponding nucleoside residue in the first oligonucleotide. That is, the cytidine residue linked to the 5' side of the adenosine residue serving as the editing target and the nucleoside residue having a hypoxanthine residue as a base in the first oligonucleotide may form a wobble base pair. The nucleoside residue having a hypoxanthine residue as a base may be an inosine residue or a 2'-deoxyinosine residue, and is preferably a 2'-deoxyinosine residue. Furthermore, in the nucleoside residue having a hypoxanthine residue as a base, the sugar moiety may be further modified, and a phosphorus-containing linking group (for example, a phosphoric residue) linked to an adjacent nucleoside residue may be further modified. Modifications of the sugar moiety and the phosphoric residue are described below.

For example, in a case where a uridine residue is linked to the 5' side of the adenosine residue serving as the editing target in the target RNA, a nucleoside residue having an adenyl group as a base may be linked to the 3' side of the target-corresponding nucleoside residue in the first oligonucleotide. That is, the uridine residue linked to the 5' side of the adenosine residue serving as the editing target and the nucleoside residue having an adenyl group as a base in the first oligonucleotide may form a base pair. The nucleoside residue having an adenyl group as a base may be an adenosine residue or a 2'-deoxyadenosine residue, and is preferably a 2'-deoxyadenosine residue. Furthermore, in the nucleoside residue having an adenyl group as a base, the sugar moiety may be further modified, and a phosphorus-containing linking group (for example, a phosphoric residue) linked to an adjacent nucleoside residue may be further modified. Modifications of the sugar moiety and the phosphoric residue are described below.

For example, in a case where an adenosine residue is linked to the 5' side of the adenosine residue serving as the editing target in the target RNA, a nucleoside residue having a pyrimidinyl group as a base may be linked to the 3' side of the target-corresponding nucleoside residue in the first oligonucleotide. That is, the adenosine residue linked to the 5' side of the adenosine residue serving as the editing target and the nucleoside residue having a pyrimidinyl group as a base in the first oligonucleotide may form a base pair. The nucleoside residue having a pyrimidyl group as a base may be a thymidine residue or a 2'-deoxyuridine residue. Furthermore, in the nucleoside residue having a pyrimidinyl group as a base, the sugar moiety may be further modified, and a phosphorus-containing linking group (for example, a phosphoric residue) linked to an adjacent nucleoside residue may be further modified. Modifications of the sugar moiety and the phosphoric residue are described below.

For example, in a case where a guanosine residue is linked to the 5' side of the adenosine residue serving as the editing target in the target RNA, a nucleoside residue having a hypoxanthine residue as a base may be linked to the 3' side of the target-corresponding nucleoside residue in the first oligonucleotide. The nucleoside residue having a hypoxanthine residue may be a 2'-deoxyinosine residue. Furthermore, in the nucleoside residue having a hypoxanthine residue, the sugar moiety may be further modified, and a phosphorus-containing linking group (for example, a phosphoric residue) linked to an adjacent nucleoside residue may be further modified. Modifications of the sugar moiety and the phosphoric residue are described below.

In the first oligonucleotide, the number of residues of the 5'-side oligonucleotide linked to the 5' side of the target-corresponding nucleoside residue and having a base sequence complementary to the target RNA may be 3 or more and 6 or less, and is preferably 3 or more and 5 or less, 3 or more and 4 or less, or 3. In a case where the number of residues is within the above range, the selectivity for ADAR1 tends to be further increased.

The second oligonucleotide and the target RNA form a double-stranded structure (hereinafter also referred to as a second complementary strand) containing one or more nucleoside residues that do not form a complementary base pair. The number of residues of the second oligonucleotide may be 2 or more and 10 or less, or 4 or more and 8 or less. The number of residues of the second oligonucleotide is, for example, 3 or more, preferably 4 or more or 5 or more, and is, for example, 10 or less, preferably 9 or less, 8 or less, or 7 or less. In one aspect, the number of residues of the second oligonucleotide may be 6. Allowing the number of residues to be within the above range tends to enhance the editing induction further. The number of nucleoside residues that do not form a complementary base pair in the second complementary strand is, for example, 3 or less, or 2 or less, and preferably 1.

The nucleoside residue that does not form a complementary base pair in the second complementary strand may be a nucleoside residue that exists, without forming a base pair, for the reason that, in one of the target RNA and the second oligonucleotide, a nucleoside residue corresponding to the nucleoside residue in the other is deleted. Additionally, the nucleoside residue that does not form a complementary base pair in the second complementary strand may be a nucleoside residue that exists in both the target RNA and the second oligonucleotide for the reason that, in the target RNA and the second oligonucleotide, a base pair composed of corresponding nucleoside residues is a mismatch base pair (noncomplementary base pair) having nucleobases noncomplementary to each other. In one embodiment, the nucleoside residue that does not form a complementary base pair may be a nucleoside residue that exists in one of the target RNA and the second oligonucleotide, and does not form a base pair, and may be preferably a nucleoside residue that exists in the target RNA, and does not form a base pair. This tends to enhance the editing induction further. Here, the mismatch base pair means a combination in which nucleobases possessed by two corresponding nucleoside residues in the target RNA and the second oligonucleotide do not form a stable base pair.

The position of the nucleoside residue that does not form a complementary base pair in the second complementary strand may be on the target RNA, and may be the 1st residue on the 3' side from the binding position of the first complementary strand and the second complementary strand. Additionally, the position of the nucleoside residue that does not form a complementary base pair in the second complementary strand may be on the second oligonucleotide, and may be the 1st residue on the 5' side from the binding position of the first complementary strand and the second complementary strand.

The nucleoside residue that does not form a complementary base pair in the second complementary strand may be derived from the fact that a nucleoside residue corresponding to the 1st nucleoside residue on the 3' side from the binding position of the first complementary strand and the second complementary strand in the target RNA is deleted from the second oligonucleotide. Additionally, the nucleoside residue that does not form a complementary base pair in the second complementary strand may be derived from a mismatch base pair formed from: the 1st nucleoside residue on the 3' side from the binding position of the first complementary strand and the second complementary strand in the target RNA; and the nucleoside residue at the 3' end of the second oligonucleotide.

In a case where the nucleoside residue corresponding to the nucleoside residue of the target RNA is deleted in the second oligonucleotide, the second oligonucleotide may have a base sequence complementary to the target RNA except for the portion where the nucleoside residue is deleted. Additionally, in a case where the second oligonucleotide has a nucleoside residue that does not form a complementary pair with the nucleoside residue corresponding to the nucleoside residue of the target RNA, the second oligonucleotide may have a base sequence complementary to the target RNA except for the nucleoside residue that does not form a complementary pair.

In one aspect of the target-editing oligonucleotide, the first oligonucleotide that forms a first complementary strand with the target RNA includes: a cytidine residue as a target-corresponding nucleoside residue; a 3'-side oligonucleotide containing 12 to 18 or 14 to 16 residues, linked to the 3' side of the target-corresponding nucleotide residue, and having a base sequence complementary to a base sequence of the target RNA; and a 5'-side oligonucleotide containing 3 residues, linked to the 5' side of the target-corresponding nucleoside residue, and having a base sequence complementary to a base sequence of the target RNA. The second oligonucleotide includes an oligonucleotide containing 4 to 8 residues, in which oligonucleotide a nucleoside residue corresponding to the 1st residue on the 3' side from the binding position of the first complementary strand and the second complementary strand in the target RNA is deleted, and which oligonucleotide has a base sequence complementary to the 2nd and subsequent residues.

The nucleotide constituting the target-editing oligonucleotide may be selected from any of a natural ribonucleotide (RNA), natural deoxyribonucleotide (DNA), RNA/DNA chimera, and modified nucleotide which is a modified product thereof. The nucleotides constituting the target-editing oligonucleotide can be linked to each other by a phosphorus-containing linking group such as a phosphodiester bond bound to a hydroxyl group of a sugar moiety of a nucleoside. The internucleoside bond by the phosphorus-containing linking group can be formed using a 2'-position hydroxyl group, a 3'-position hydroxyl group, or a 5'-position hydroxyl group of the sugar moiety. The nucleosides constituting the target-editing oligonucleotide can be linked by forming a natural 3'-5' phosphodiester bond by a phosphoric residue. Additionally, the nucleosides constituting the target-editing oligonucleotide can be linked by forming a non-natural 3'-5' phosphorus-containing diester bond by a phosphorus-containing linking group other than a phosphoric residue. Here, the non-natural phosphorus-containing diester bond means that the phosphodiester bond portion is modified. At least one of the nucleosides constituting the target-editing oligonucleoside may be a modified nucleoside. Examples of the modified nucleoside include modified nucleosides in which a sugar moiety is modified, modified nucleosides in which a base is modified, and combinations thereof.

Examples of the modification of the sugar moiety include 2'-O-alkylated ribonucleotides of D-ribofuranose (for example, 2'-O-methylated, 2'-O-ethylated, 2'-O-aminoethylated, 2'-O-propylated, 2'-O-allylated, 2'-O-methoxyethylated, 2'-O-butylated, 2'-O-pentylated, and 2'-O-propargylated); bridged ribonucleotides in which the 2' position and 4' position of D-ribofuranose are bridged (for example, 2'-O,4'-C-ethylenated, 2'-O,4'-C-methylenated, 2'-O,4'-C-propylenated, 2'-O,4'-C-tetramethylenated, 2'-O,4'-C-pentamethylenated, 2'-S,4'-C-methylenated, 2'-deoxy-2'-C,4'-C-methyleneoxymethylenated form of D-ribofuranose, S-cEt (2',4'-constrained ethyl), and AmNA); 3'-deoxy-3'-amino-2'-deoxy-D-ribofuranose; 3'-deoxy-3'-amino-2'-deoxy-2'-fluoro-D-ribofaranose; 2'-deoxy-2'-fluoro-D-ribofuranose; and D-deoxyribose.

Examples of the modification of the phosphodiester bond portion include a phosphorothioate bond (including an optical isomer derived from the asymmetry of the phosphorus atom), an alkyl phosphate bond, an alkylphosphonate bond, an alkylthiophosphonate bond, a phosphorodithioate bond, and a phosphoramidate bond.

Here, the phosphorothioate bond is formed when thiophosphoric acid forms ester the respective bonds with hydroxyl groups of sugar moieties of two nucleosides. That is, the phosphorothioate bond can be formed by a thiophosphoric residue.

The alkyl phosphate bond is formed when an alkyl phosphate forms the respective ester bonds with hydroxyl groups of sugar moieties of two nucleosides. That is, the alkyl phosphate bond can be formed by an alkyl phosphate residue.

The alkyl phosphonate bond is formed when alkylphosphonic acid forms the respective ester bonds with hydroxyl groups of sugar moieties of two nucleosides. That is, the alkylphosphonate bond can be formed by an alkylphosphonic residue.

The alkyl thiophosphonate bond is formed when alkylthiophosphonic acid forms the respective ester bonds with hydroxyl groups of sugar moieties of two nucleosides. That is, the alkyl thiophosphonate bond can be formed by an alkylthiophosphonic residue.

The phosphorodithioate bond is formed when dithiophosphoric acid forms the respective ester bonds with hydroxyl groups of sugar moieties of two nucleosides. That is, the phosphorodithioate bond can be formed by a dithiophosphoric residue.

The phosphoramidate bond is formed by a phosphoric amide which may have a substituent forms the respective ester bonds with hydroxyl groups of sugar moieties of two nucleosides. That is, the phosphoramidate bond can be formed by a phosphoramide residue optionally having a substituent. The substituent in the phosphoric amide is a substituent on a nitrogen atom, and examples thereof include an alkyl group, an alkylsulfonyl group, and an imino group optionally having a substituent. Specific examples of the alkyl group and the alkyl group portion of the alkylsulfonyl group include: C₁₋₆ alkyl groups such as methyl, ethyl, propyl, isopropyl, cyclopropyl, butyl, isobutyl, t-butyl, cyclobutyl, pentyl, and hexyl; and a substituted alkyl group such as a 2-methoxyethyl group. Specific examples of the imino group optionally having a substituent include a 1,3-dimethylimidazolidin-2-ylidene group.

The alkyl group in the alkyl phosphate, alkylphosphonic acid, alkylthiophosphonic acid, and phosphoric amide may be linear, branched, cyclic, or a combination thereof. The number of carbon atoms of the alkyl group may be, for example, 1 to 6, and preferably 1 to 3 or 1. Specific examples of the alkyl group include methyl, ethyl, 2-methoxyethyl, propyl, 3-methoxypropyl, an isopropyl, cyclopropyl, butyl, isobutyl, t-butyl, cyclobutyl, pentyl, and hexyl. Additionally, the alkyl group may further have a substituent. Examples of the substituent in the alkyl group include: a halogen atom such as a fluorine atom and a chlorine atom; and a C₁₋₃ alkoxy group.

Specific examples of the alkyl phosphate include methyl phosphate, ethyl phosphate, propyl phosphate, isopropyl phosphate, butyl phosphate, 2-methoxyethyl phosphate, cyclopropyl phosphate, and cyclobutyl phosphate. Specific examples of the alkylphosphonic acid include methylphosphonic acid, ethylphosphonic acid, propylphosphonic acid, isopropylphosphonic acid, butylphosphonic acid, and 3-methoxypropylphosphonic acid. Specific examples of the alkylthiophosphonic acid include methylthiophosphonic acid, ethylthiophosphonic acid, propylthiophosphonic acid, and isopropylthiophosphonic acid. Specific examples of the substituted phosphoramide include N-methylphosphoramide, N-ethylphosphoramide, and N-methanesulfonylphosphoramide (mesylphosphoramide).

Examples of the modification of the base moiety include: halogenation; C₁₋₆ or C₁₋₄ alkylation such as methylation, ethylation, propylation, isopropylation, cyclopropylation, butylation, isobutylation, s-butylation, t-butylation, and cyclobutylation; hydroxylation; amination; deamination; and demethylation. Specific examples thereof include: 5-methylation, 5-fluorination, 5-bromination, 5-iodination, and N4-methylation of cytosine; 5-demethylation (uracil), 5-fluorination, 5-bromination, and 5-iodination of thymine; N6-methylation and 8-bromination of adenine; and N2-methylation and 8-bromination of guanine.

In the oligonucleotide linked to the 3' side of the target-corresponding nucleoside residue in the target-editing oligonucleotide, at least one phosphorus-containing linking group selected from the group consisting of a phosphorus-containing linking group linking a 1st nucleoside residue and a 2nd nucleoside residue and a phosphorus-containing linking group linking a 4th nucleoside residue and a 5th nucleoside residue, counting in the 3' direction from the target-corresponding nucleoside residue, may be at least one selected from the group consisting of an alkylphosphonic residue, an alkyl phosphate residue, and a substituted phosphoramide residue, preferably at least one selected from the group consisting of an alkylphosphonic residue and an alkyl phosphate residue, and more preferably at least one selected from the group consisting of a methylphosphonic residue, an ethyl phosphate residue, and a 2-methoxyethyl phosphate residue. Additionally, the phosphorus-containing linking group linking the 1st nucleoside residue and the 2nd nucleoside residue and the phosphorus-containing linking group linking the 4th nucleoside residue and the 5th nucleoside residue, counting in the 3' direction from the target-corresponding nucleoside residue, may be each independently at least one selected from the group consisting of an alkylphosphonic residue, an alkyl phosphate residue, and a substituted phosphoramide residue, preferably at least one selected from the group consisting of an alkylphosphonic residue and an alkyl phosphate residue, and more preferably at least one selected from the group consisting of a methylphosphonic residue, an ethyl phosphate residue, and a 2-methoxyethyl phosphate residue. Here, the 1st nucleoside residue counting in the 3' direction from the target-corresponding nucleoside residue is the 1st residue counting without including the target-corresponding nucleoside residue itself, that is, the nucleoside residue adjacent to the 3' side of the target-corresponding oligonucleoside residue.

Herein, counting in the 3' direction from the target-corresponding nucleoside residue, the position of the phosphorus-containing linking group linking the target-corresponding nucleoside residue and the 1st nucleoside residue is referred to as internucleoside 0 in some cases, and the position of the phosphorus-containing linking group linking the 4th nucleoside residue and the 5th nucleoside residue is referred to as internucleoside +4 in some cases. Additionally, counting in the 5' direction from the target-corresponding nucleoside residue, the position of the phosphorus-containing linking group linking the 1st nucleoside residue and the target-corresponding nucleoside residue is referred to as internucleoside -1 in some cases, and the position of the phosphorus-containing linking group linking the 7th nucleoside residue and the 6th nucleoside residue is referred to as internucleoside -7 in some cases. Other internucleosides are referred to in the same manner in some cases. Additionally, in a case where the second oligonucleotide has, at the 3' end, a nucleoside residue that does not form a complementary base pair with a nucleoside residue of the target RNA, the nucleoside residue is not considered in specifying the position of the phosphorus-containing linking group.

In the target-editing oligonucleotide, at least one phosphorus-containing linking group selected from the group consisting of: a phosphorus-containing linking group (+2) linking the 2nd nucleoside residue and the 3rd nucleoside residue counting, a phosphorus-containing linking group (+6) linking the 6th nucleoside residue and the 7th nucleoside residue, a phosphorus-containing linking group (+8) linking the 8th nucleoside residue and the 9th nucleoside residue, a phosphorus-containing linking group (+10) linking the 10th nucleoside residue and the 11th nucleoside residue, and a phosphorus-containing linking group (+11) linking the 11th nucleoside residue and the 12th nucleoside residue, in the 3' direction from the target-corresponding nucleoside residue; and a phosphorus-containing linking group (-2) linking the 2nd nucleoside residue and the 1st nucleoside residue, a phosphorus-containing linking group (-3) linking the 3rd nucleoside residue and the 2nd nucleoside residue, and a phosphorus-containing linking group (-6) linking the 6th nucleoside residue and the 5th nucleoside residue, counting in the 5' direction from the target-corresponding nucleoside residue, may be a phosphoric residue. In the first oligonucleotide, preferably, at least one phosphorus-containing linking group selected from the group consisting of a phosphorus-containing linking group at internucleoside +2, a phosphorus-containing linking group at internucleoside +8, a phosphorus-containing linking group at internucleoside +10, a phosphorus-containing linking group at internucleoside +11, and a phosphorus-containing linking group at internucleoside -6 may be a phosphoric residue. In the first oligonucleotide, at least the phosphorus-containing linking group at internucleoside +2 may be more preferably a phosphoric residue. That is, in the first oligonucleotide, at least the 2nd nucleoside residue and the 3rd nucleoside residue counting in the 3' direction from the target-corresponding nucleoside residue may be linked by a phosphodiester bond. The number of phosphoric residues linking the nucleoside residues constituting the first oligonucleotide may be, for example, 0 or more and 8 or less, and preferably 4 or less. The number of phosphoric residues may be 1 or more.

In the target-editing oligonucleotide, some nucleoside residues may be linked by a phosphorothioate bond. In the target-editing oligonucleotide, a phosphorus-containing linking group other than a phosphoric residue, an alkylphosphonic residue, an alkyl phosphate residue, and a substituted phosphoramide residue that link nucleoside residues may be a thiophosphoric residue. Additionally, in the target-editing oligonucleotide, all phosphorus-containing linking groups other than the phosphorus-containing linking groups at internucleosides +1, +2, +4, +8, +10, +11, -2, -3, or -6 may be thiophosphoric residues, and all phosphorus-containing linking groups other than at least one of the phosphorus-containing linking groups at internucleoside +1 or +4 and at least one selected from the group consisting of the phosphorus-containing linking groups at internucleosides +8, +10, +11 or -6 may be thiophosphoric residues.

The counter region of the first oligonucleotide may contain a phosphorothioate bond. At least one of the phosphorus-containing linking group linking the target-corresponding nucleoside residue and the nucleoside residue on the 3' side thereof or the phosphorus-containing linking group linking the target-corresponding nucleoside residue and the nucleoside residue on the 5' side thereof may be a thiophosphoric residue. Both of the phosphorus-containing linking groups may be thiophosphoric residues. That is, the first oligonucleotide may have a partial structure in which the target-corresponding nucleoside residue and the nucleoside residue on the 3' side thereof are linked via a phosphorothioate bond, may have a partial structure in which the target-corresponding nucleoside residue and the nucleoside residue on the 5' side thereof are linked via a phosphorothioate bond, or may have a partial structure in which the target-corresponding nucleoside residue is linked to the nucleoside residue on the 3' side thereof and the nucleoside residue on the 5' side thereof via the respective phosphorothioate bonds.

The target-editing oligonucleotide may contain at least one modified nucleotide residue selected from the group consisting of a 2'-O-alkylribonucleoside residue, a 2'-deoxy-2'-fluororibonucleoside residue, a bridged nucleoside residue, and a 2'-deoxyribonucleoside residue.

In the target-editing oligonucleotide, an oligonucleoside residue linked to the 3' side of the target-corresponding nucleoside residue may be a 2'-deoxynucleoside residue. Additionally, an oligonucleoside residue linked to the 5' side of the target-corresponding nucleoside residue may be a 2'-O-alkylribonucleoside residue or a 2'-deoxynucleoside residue, and preferably a 2'-O-alkylnucleoside residue.

In the first oligonucleotide, the 3' side oligonucleotide linked to the 3' side of the target-corresponding nucleoside residue may have a base sequence in which two types of modified nucleoside residues selected from the group consisting of a 2'-deoxy-2'-fluoronucleoside residue, a 2'-O-alkylribonucleoside residue, and a bridged nucleoside residue are alternately linked. That is, the 3' side oligonucleotide linked to the 3' side of the target-corresponding nucleoside residue may have a base sequence in which 2'-deoxy-2'-fluoronucleoside residues and 2'-O-alkylribonucleoside residues are alternately linked, may have a base sequence in which bridged nucleoside residues and 2'-O-alkylribonucleoside residues are alternately linked, or may have a base sequence in which 2'-deoxy-2'-fluoronucleoside residues and bridged nucleoside residues are alternately linked. Having a base sequence in which two types of modified nucleoside residues used herein are alternately linked means both that the subject oligonucleotide (here, the 3' side oligonucleotide of the first oligonucleotide applies) has a base sequence in which two types of modified nucleoside residues are partially alternately linked, and that the entire subject oligonucleotide has a base sequence in which two types of modified nucleoside residues are alternately linked, and is used with the same meaning in the following description herein.

In the 3' side oligonucleotide of the first oligonucleotide, the 3rd nucleoside residue counting in the 3' direction from the target-corresponding nucleoside residue may be a modified nucleoside residue selected from the group consisting of a 2'-deoxy-2'-fluoronucleoside residue, a 2'-O-alkylribonucleoside residue, and a bridged nucleoside residue, and preferably a 2'-deoxy-2'-fluoronucleoside residue. Additionally, in the 3' side oligonucleotide, the 3rd nucleoside residue counting in the 3' direction from the target-corresponding nucleoside residue may be a modified nucleoside residue selected from the group consisting of a 2'-deoxy-2'-fluoronucleoside residue, a 2'-O-alkylribonucleoside residue, and a bridged nucleoside residue, and the 4th nucleoside residue may be a modified nucleoside residue different from the 3rd modified nucleoside residue.

The first oligonucleotide may have at least one bridged nucleoside residue at the 10th and subsequent nucleoside residues, preferably the 11th and subsequent nucleoside residues, counting in the 3' direction from the target-corresponding nucleoside residue. In the bridged nucleoside residue, the 2' position and the 4' position of D-ribofuranose may be bridged therebetween. The bridged nucleoside residue may include at least one of a 2'-O,4'-C-methylene bridged-nucleoside (LNA) or a 2'-O,4'-C-ethylene-bridged nucleoside (ENA). Additionally, in the bridged nucleoside residue, the base moiety may be further modified, and a phosphorus-containing linking group (for example, a phosphoric residue) linked to an adjacent nucleoside residue may be further modified.

The first oligonucleotide may have at least one kind of bridged nucleoside residue at least one nucleoside residue selected from the group consisting of the 5th, 4th, 3rd, 2nd, and 1st nucleoside residues counting in the 5' direction from the 3' end. Preferably, the first oligonucleotide may have at least one kind of bridged nucleoside residue at at least one nucleoside residue selected from the group consisting of the 5th, 3rd, and 1st nucleoside residues counting in the 5' direction from the 3' end. Here, the 5th counting in the 5' direction from the 3' end means the 5th when the nucleoside residue adjacent to the 5' side of the nucleoside residue at the 3' end is counted as the 1st.

In the first oligonucleotide, an oligonucleotide composed of the 10th and subsequent nucleoside residues, preferably the 11th and subsequent nucleoside residues, counting in the 3' direction from the target-corresponding nucleoside residue, may have a base sequence in which 2'-O-alkylribonucleoside residues and bridged nucleoside residues are alternately linked. The number of repetitions of the linkage between the 2'-O-alkylribonucleoside residue and the bridged nucleoside residue may be, for example, 1 or more and 3 or less, and preferably 2. In the base sequence in which 2'-O-alkylribonucleoside residues and bridged nucleoside residues are alternately linked, the former residues and the latter residues may be alternately linked in the 3' direction from the 2'-O-alkylribonucleoside residue, or may be alternately linked in the 3' direction from the bridged nucleoside residue. Additionally, in the 2'-O-alkylribonucleoside residue or the bridged nucleoside residue, the base moiety may be further modified, and a phosphorus-containing linking group (for example, a phosphoric residue) linked to an adjacent nucleoside residue may be further modified. Here, for the number of repetitions of the linkage between the 2'-O-alkylribonucleoside residue and the bridged nucleoside residue, a pair of a 2'-O-alkylribonucleoside residue and a bridged nucleoside residue linked therebetween is regarded as one unit. That is, the number of repetitions for 3 residues alternately linked is 1, and the number of repetitions for 4 residues alternately linked is 2.

The 5'-side oligonucleotide linked to the 5' side of the target-corresponding nucleoside residue of the first oligonucleotide may have a base sequence in which 2'-O-alkylribonucleoside residues are consecutive. The base sequence in which 2'-O-alkylribonucleoside residues are consecutive may be adjacent to the target-corresponding nucleoside residue. The number of residues in the base sequence in which 2'-O-alkylribonucleoside residues are consecutive may be, for example, 2 or 3. Additionally, the 5' side oligonucleotide may have a base sequence in which a 2'-O-alkylribonucleoside residue and a 2'-deoxy-2'-fluoronucleoside residue or a bridged nucleoside residue are linked.

The 5'-side oligonucleotide in the first oligonucleotide may be constituted such that part of the nucleoside residues are linked by a phosphorothioate bond, or may be constituted such that all the nucleoside residues are linked by a phosphorothioate bond.

In at least 1 residue, at least 3 residues, or all the residues in the second oligonucleotide, at least one of a sugar moiety or a phosphodiester bond may be modified, at least a sugar moiety may be modified, at least a phosphodiester bond may be modified, or a sugar moiety and a phosphodiester bond may be modified. The modification of the sugar moiety in the second oligonucleotide may be, for example, 2'-O-alkylation, 2'-deoxy-2'-fluorination, crosslinking between the 2' position and the 4' position, or 2'-deoxylation (DNA formation). In a case where the second oligonucleotide has a plurality of modified nucleoside residues, the plurality of modified nucleotide residues may be arranged consecutively, or may be arranged apart.

The second oligonucleotide may have a base sequence in which 2'-O-alkylribonucleoside residues are consecutive. The second oligonucleotide may have a base sequence in which two kinds of residues selected from the group consisting of a 2'-deoxy-2'-fluoronucleoside residue, a 2'-O-alkylribonucleoside residue, and a bridged nucleoside residue are alternately linked; may have a base sequence in which 2'-O-alkylribonucleoside residues and 2'-deoxy-2'-fluoronucleoside residues are alternately linked; or may have a base sequence in which 2'-O-alkylribonucleoside residues and bridged nucleoside residues are alternately linked. Furthermore, from the viewpoint of ADAR1 selectivity, the second oligonucleotide is as follows: the 2nd nucleoside residue counting in the 5' direction from the nucleoside residue at the 3' end of the second oligonucleotide may be one selected from the group consisting of a 2'-deoxy-2'-fluoronucleoside residue, a 2'-O-alkylribonucleoside residue, and a bridged nucleoside residue, or may be a 2'-O-alkylribonucleoside residue. Here, the nucleoside residue at the 3' end of the second oligonucleotide means a nucleoside residue located farthest from the 5' end among the nucleoside residues forming complementary pairs with nucleoside residues of the target RNA. Accordingly, the 2nd nucleoside residue counting in the 5' direction from the nucleoside residue at the 3' end of the second oligonucleotide refers to the 2nd nucleoside residue counting in the 5' direction when the nucleoside residue adjacent in the 5' direction to the oligonucleoside residue at the 3' end is counted as the 1st, not including the nucleoside residue at the 3' end itself, among the nucleoside residues forming complementary pairs with nucleoside residues of the target RNA.

The second oligonucleotide may have at least one bridged nucleoside residue at the 2nd and subsequent nucleoside residues, preferably the 3rd and subsequent nucleoside residues, counting in the 5' direction from the nucleoside residue at the 3' end of the second oligonucleotide. In the bridged nucleoside residue, the 2' position and the 4' position of D-ribofuranose may be bridged therebetween. The bridged nucleoside residue may include at least one of a 2'-O,4'-C-methylene bridged-nucleoside (LNA) or a 2'-O,4'-C-ethylene-bridged nucleoside (ENA). Additionally, in the bridged nucleoside residue, at least one of a base portion or a phosphate ester bond portion may be further modified.

In the second oligonucleotide, an oligonucleotide composed of the 2nd and subsequent, preferably the 3rd and subsequent nucleoside residues counting in the 5' direction from the nucleoside residue at the 3' end of the second oligonucleotide may have a base sequence in which 2'-O-alkylribonucleoside residues and bridged nucleoside residues are alternately linked. The number of repetitions of the linkage between the 2'-O-alkylribonucleoside residue and the bridged nucleoside residue may be, for example, 1 or more and 3 or less, and preferably 2. In the base sequence in which 2'-O-alkylribonucleoside residues and bridged nucleoside residues are alternately linked, the former residues and the latter residues may be alternately linked in the 5' direction from the 2'-O-alkylribonucleoside residue, or may be alternately linked in the 5' direction from the bridged nucleoside residue. Additionally, in the 2'-O-alkylribonucleoside residue or the bridged nucleoside residue, at least one of a base portion or a phosphate ester bond portion may be further modified.

The second oligonucleotide may be constituted such that part of the nucleoside residues are linked by a phosphorothioate bond, or may be constituted such that all the nucleoside residues are linked by a phosphorothioate bond. Additionally, in the second oligonucleotide, a phosphorus-containing linking group linking a part of nucleoside residues may be at least one selected from the group consisting of an alkylphosphonic residue, an alkyl phosphate residue, and a substituted phosphoramide residue. In the second oligonucleotide, at least one phosphorus-containing linking group selected from the group consisting of a phosphorus-containing linking group linking a 1st nucleoside residue and a 2nd nucleoside residue, a phosphorus-containing linking group linking a 3rd nucleoside residue and a 4th nucleoside residue, and a phosphorus-containing linking group linking a 5th nucleoside residue and a 6th nucleoside residue, counting in the 5' direction from the nucleoside residue at the 3' end of the second oligonucleotide, may be at least one selected from the group consisting of an alkylphosphonic residue, an alkyl phosphate residue, and a substituted phosphoramide residue.

In one aspect, the target-editing oligonucleotide may contain a modified oligonucleotide having a chemical modification pattern represented by any one of the following Formulas (1) to (22), or may be composed of a modified oligonucleotide having a chemical modification pattern represented by any one of the following Formulas (1) to (22).

(1) 5'- HO-MsLsMsLsMsFsXFsMsMsdCsDqMsFsMqFsMsFsMsFsMsLsMsLsM-OH -
3'

(2) 5’- HO-MsLsMsLsMsFsXFsMsMsdCsDqMsFsMqFsMsFsMsFsMsMsMsLsM-OH -
3’

(3) 5’- HO-MsLsMsMsMsFsXFsMsMsdCsDqMsFsMqFsMsFsMsFsMsLsMsLsM-OH -
3’

(4) 5’- HO-MsLsMsMsMsFsXFsMsMsdCsDqMsFsMqFsMsFsMsFsMsMsMsLsM-OH
-3’

(5) 5’- HO-LsMsMsMsMsFsXFsMsMsdCsDqMsFsMqFsMsFsMsFsMsMsMsLsM-OH
-3’

(6) 5'- HO-MsLsMsLsMsFsXFsMsMsdCsDqMpFsMqFsMsFsMsFsMsLsMsLsM-OH -
3'

(7) 5'- HO-MsLsMsLsMsFsXFpMsMsdCsDqMpFsMqFsMsFsMsFsMsLsMsLsM-OH -
3'

(8) 5'- HO-MsLsMsLsMsFsXFsMpMsdCsDqMsFsMqFsMsFsMsFsMsLsMsLsM-OH -
3'

(9) 5'- HO-MsLsMsLpMsFsXFsMsMsdCsDqMpFsMqFsMsFsMsFsMsLpMsLsM-OH -
3'

(10) 5'- HO-MsLsMsLpMsFsXFsMsMsdCsDqMpFsMqFsMpFsMsFsMsLpMsLsM-

OH -3'

(11) 5'- HO-MsLsMsLpMsFsXFsMsMsdCsDqMpFsMqFsMsFsMpFsMsLpMsLsM-OH
-3'

(12) 5'- HO-MsLsMsLpMsFsXFsMsMsdCsDqMpFsMqFsMsFsMsFsMpLpMsLsM-
OH -3'

(13) 5'- HO-MsLsMsLsMsFsXFsMsMsdCsDqMpFsMqFsMsFsMsFsMsMsMsLsM-OH
-3'

(14) 5’- HO-MsLsMsLsMsFsXFsMsMsdCsDqMpFsMqFsMpFsMsFsMsMsMsLsM-
OH -3’

(15) 5'- HO-MsLsMsLsMsFsXFsMsMsdCsDqMpFsMqFsMsFsMpFsMsMsMsLsM-
OH -3'

(16) 5'- HO-MsLsMsLsMsFsXFsMsMsdCsDqMpFsMqFsMsFsMsFsMpMsMsLsM-
OH -3'

(17) 5'- HO-MsLsMsLsMsFsXFsMsMsdCsDqMpFsMqFsMpFsMpFsMsMsMsLsM-
OH -3'

(18) 5'- HO-MsLsMsLsMsFsXFsMsMsdCsDqMpFsMqFsMpFsMsFsMpMsMsLsM-
OH -3'

(19) 5'- HO-MsLsMsLsMsFsXFsMsMsdCsDqMpFsMqFsMsFsMpFsMpMsMsLsM-
OH -3'

(20) 5'- HO-MsLsMsLsMsFsXFsMsMsdCsDqMpFsMqFsMpFsMpFsMpMsMsLsM-
OH -3'

(21) 5'- HO-MsLsMsLsMsFsXFsMsMsdCsDqMpFsMsFsMsFsMsFsMsLsMsLsM-OH
-3'

(22) 5’- HO-MsLsMsLsMsFsXFsMsMsdCsDqMpFsMsFsMsFsMsFsMsMsMsLsM-OH
-3’

In the Formulas, D represents a 2'-deoxyribonucleoside residue, F represents a 2'-fluoro-2'-deoxyribonucleoside residue, L represents a bridged nucleoside residue, M represents a 2'-O-methyl- ribonucleoside residue, dC represents a target nucleoside residue, a 2'-deoxycytidine residue. X indicates that one nucleoside residue is missing from the target RNA. p represents a phosphate residue, s represents a thiophosphate residue, q represents an alkylphosphonate residue, an alkyl phosphate residue, or a substituted phosphoramide residue. HO- represents a hydroxy group at the 5' end, and -OH represents a hydroxyl group at the 3' end.

In one aspect, the target-editing oligonucleotide may contain a modified oligonucleotide having a chemical modification pattern represented by any one of the following Formulas (1a) to (22a), or may be composed of a modified oligonucleotide having a chemical modification pattern represented by any one of the following Formulas (1a) to (22a).

(1a) 5'- HO-MsLsMsLsMsFsXFsMsMsdCsDpmMsFsMpmFsMsFsMsFsMsLsMsLsM-
OH -3'

(2a) 5'- HO-MsLsMsLsMsFsXFsMsMsdCsDpmMsFsMpmFsMsFsMsFsMsMsMsLsM-
OH -3'

(3a) 5'- HO-MsLsMsMsMsFsXFsMsMsdCsDpmMsFsMpmFsMsFsMsFsMsLsMsLsM-
OH -3'

(4a) 5’-HO-MsLsMsMsMsFsXFsMsMsdCsDpmMsFsMpmFsMsFsMsFsMsMsMsLsM-
OH -3’

(5a) 5’-HO-LsMsMsMsMsFsXFsMsMsdCsDpmMsFsMpmFsMsFsMsFsMsMsMsLsM-
OH -3’

(6a) 5’- HO-MsLsMsLsMsFsXFsMsMsdCsDrMpFsMrFsMsFsMsFsMsLsMsLsM-OH -
3’

(7a) 5'- HO-MsLsMsLsMsFsXFpMsMsdCsDrMpFsMrFsMsFsMsFsMsLsMsLsM-OH -
3'

(8a) 5’- HO-MsLsMsLsMsFsXFsMpMsdCsDrMsFsMrFsMsFsMsFsMsLsMsLsM-OH -
3’

(9a) 5'- HO-MsLsMsLpMsFsXFsMsMsdCsDrMpFsMrFsMsFsMsFsMsLpMsLsM-OH
-3'

(10a) 5'- HO-MsLsMsLpMsFsXFsMsMsdCsDrMpFsMrFsMpFsMsFsMsLpMsLsM-
OH -3'

(11a) 5'- HO-MsLsMsLpMsFsXFsMsMsdCsDrMpFsMrFsMsFsMpFsMsLpMsLsM-

OH -3’

(12a) 5'- HO-MsLsMsLpMsFsXFsMsMsdCsDrMpFsMrFsMsFsMsFsMpLpMsLsM-
OH -3'

(13a) 5'- HO-MsLsMsLsMsFsXFsMsMsdCsDrMpFsMrFsMsFsMsFsMsMsMsLsM-
OH -3'

(14a) 5'- HO-MsLsMsLsMsFsXFsMsMsdCsDrMpFsMrFsMpFsMsFsMsMsMsLsM-
OH -3'

(15a) 5'- HO-MsLsMsLsMsFsXFsMsMsdCsDrMpFsMrFsMsFsMpFsMsMsMsLsM-
OH -3'

(16a) 5'- HO-MsLsMsLsMsFsXFsMsMsdCsDrMpFsMrFsMsFsMsFsMpMsMsLsM-
OH -3'

(17a) 5'- HO-MsLsMsLsMsFsXFsMsMsdCsDrMpFsMrFsMpFsMpFsMsMsMsLsM-
OH -3'

(18a) 5'- HO-MsLsMsLsMsFsXFsMsMsdCsDrMpFsMrFsMpFsMsFsMpMsMsLsM-
OH -3'

(19a) 5'- HO-MsLsMsLsMsFsXFsMsMsdCsDrMpFsMrFsMsFsMpFsMpMsMsLsM-
OH -3'

(20a) 5'- HO-MsLsMsLsMsFsXFsMsMsdCsDrMpFsMrFsMpFsMpFsMpMsMsLsM-
OH -3'

(21a) 5’- HO-MsLsMsLsMsFsXFsMsMsdCsDrMpFsMsFsMsFsMsFsMsLsMsLsM-OH
-3’

(22a) 5'- HO-MsLsMsLsMsFsXFsMsMsdCsDrMpFsMsFsMsFsMsFsMsMsMsLsM-
OH -3'

In the Formulas, D represents a 2'-deoxyribonucleoside residue, F represents a 2'-fluoro-2'-deoxyribonucleoside residue, L represents a bridged nucleoside residue, M represents a 2'-O-methyl- ribonucleoside residue, dC represents a target nucleoside residue, a 2'-deoxycytidine residue. X indicates that one nucleoside residue is missing from the target RNA. p represents a phosphate residue, s represents a thiophosphate residue, q represents an alkylphosphonate residue, an alkyl phosphate residue, or a substituted phosphoramide residue. HO- represents a hydroxyl group at the 5' end, and -OH represents a hydroxyl group at the 3' end.

In one aspect, the target-editing oligonucleotide is an oligonucleotide or a pharmaceutically acceptable salt thereof, which oligonucleotide induces site-specific editing that converts an editing target adenosine residue contained in a target RNA into an inosine residue, and the oligonucleotide may include a structure represented by the following Formula (A), or may be composed of a structure represented by the following Formula (A).

(A) 5'-O¹-(N¹-L¹)-(N² -L²)-X-(N³-L³)p-(N⁴-L⁴)-(N⁵-L⁵)-(N⁶-L⁶)-(N⁷-L⁷ (N⁸-L⁸)-(N⁹-L⁹)-(N¹⁰-L¹⁰)-O²-O³-3'

In Formula (A), the target-corresponding nucleoside residue corresponding to the editing target adenosine residue may be represented by N⁶. An oligonucleotide composed of nucleoside residues excluding N⁶ and X has a base sequence complementary to the target RNA. The base sequence corresponds to a base sequence of the target RNA, such that the target RNA contains a base corresponding to X, or contains a base at the position corresponding to X in the case of a deletion of X.

In Formula (A), O¹ may represent an oligonucleotide of 1 to 8 residues that forms a complementary pair with the corresponding oligonucleotide portion of the target RNA. N¹ to N⁵ and N⁷ to N¹⁰ may each represent a nucleoside residue that forms a complementary pair with the corresponding nucleoside residue of the target RNA. N⁶ may represent a 2'-deoxycytidine residue, a 2'-deoxyguanosine residue, a 2'-deoxyadenosine residue, or a derivative thereof. O² and O³ may each independently represent an oligonucleotide of 3 to 17 residues that forms a complementary pair with the corresponding oligonucleotide portion of the target RNA. The total number of residues of O² and O³ may be 6 to 20. X may represent a deletion of one nucleoside residue with respect to the target RNA, or represent a nucleoside residue and a phosphorus-containing linking group, which nucleoside residue is not complementary to the corresponding nucleoside residue of the target RNA. L¹, L², L³, L⁴, L⁵, L⁶, L⁸, and L⁹ may each independently represent a phosphorus-containing linking group selected from the group consisting of a phosphoric residue and a thiophosphoric residue. At least one of L¹, L², L³, L⁴, L⁵, L⁶, L⁸, or L⁹ may be a thiophosphoric residue. L⁷ and L¹⁰ may each be independently selected from the group consisting of a phosphoric residue, a thiophosphoric residue, an alkylphosphonic residue, an alkyl phosphate residue, and a substituted phosphoramide residue. At least one of L⁷ or L¹⁰ may represent a phosphorus-containing linking group selected from the group consisting of an alkylphosphonic residue, an alkyl phosphate residue, and a substituted phosphoramide residue. p may represent an integer of 1 to 4. In Formula (A), 5'- and -3' represent the 5' side and the 3' side of the oligonucleotide represented by Formula (A), respectively.

The target-editing oligonucleotide has the structure represented by Formula (A), thus having excellent stability in a living organism, and being capable of more effectively inducing the editing activity of ADAR in a cell.

In this regard, in the structure represented by Formula (A), the oligonucleotide represented by (N³-L³)-(N⁴-L⁴)-(N⁵-L⁵)-(N⁶-L⁶)-(N⁷-L⁷)-(N⁸-L⁸)-(N⁹-L⁹)-(N¹⁰-L¹⁰)-O²-O³ from the 5' side to the 3' side may correspond to a first oligonucleotide that identifies a target RNA, and the oligonucleotide represented by O¹-(N¹-L¹)-(N²-L²)-X from the 5' side to the 3' side may correspond to a second oligonucleotide linked to the 5' side of the first oligonucleotide.

In Formula (A), L¹, L², L³, L⁴, L⁵, L⁶, L⁸, and L⁹ may all represent a thiophosphoric residue, or 1 to 5, preferably 1 to 3, or 1 thereof may represent a phosphoric residue, and the remainder may represent a thiophosphoric residue. Additionally, in a case where any of L¹, L ², L³, L ⁴, L⁵, L⁶, L⁸, and L⁹ represents a phosphoric residue, at least L⁸ may represent a phosphoric residue.

O¹ may preferably represent an oligonucleotide of 1 to 6 residues, 2 to 6 residues, or 4 residues. Additionally, O¹ may include an oligonucleotide in which two kinds of residues selected from the group consisting of a 2'-deoxy-2'-fluoronucleoside residue, a 2'-O-alkylribonucleoside residue, and a bridged nucleoside residue are alternately linked, or may include an oligonucleotide in which 2'-O-alkylribonucleoside residues and bridged nucleoside residues are alternately linked.

O¹ may be constituted such that part of the nucleoside residues are linked by a phosphorothioate bond, or may be constituted such that all the nucleoside residues are linked by a phosphorothioate bond. Additionally, in O¹, a phosphorus-containing linking group linking a part of nucleoside residues may include at least one selected from the group consisting of an alkylphosphonic residue, an alkyl phosphate residue, and a substituted phosphoramide residue.

O² may preferably represent an oligonucleotide of 3 to 12 residues, 3 to 10 residues, 3 to 8 residues, or 3 to 6 residues. Additionally, O² may include an oligonucleotide in which two kinds of residues selected from the group consisting of a 2'-deoxy-2'-fluoronucleoside residue, a 2'-O-alkylribonucleoside residue, and a bridged nucleoside residue are alternately linked, or may include an oligonucleotide in which 2'-deoxy-2'-fluoronucleoside residues and 2'-O-alkylribonucleoside residues are alternately linked.

O² may be constituted such that part of the nucleoside residues are linked by a phosphorothioate bond, or may be constituted such that all the nucleoside residues are linked by a phosphorothioate bond. Additionally, in O², a phosphorus-containing linking group linking a part of nucleoside residues may include at least one selected from the group consisting of an alkylphosphonic residue, an alkyl phosphate residue, and a substituted phosphoramide residue.

O³ may preferably represent an oligonucleotide of 3 to 12 residues, 3 to 10 residues, 3 to 8 residues, or 3 to 6 residues. Additionally, O³ may include an oligonucleotide in which two kinds of residues selected from the group consisting of a 2'-deoxy-2'-fluoronucleoside residue, a 2'-O-alkylribonucleoside residue, and a bridged nucleoside residue are alternately linked, or may include an oligonucleotide in which 2'-O-alkylribonucleoside residues and bridged nucleoside residues are alternately linked.

O³ may be constituted such that part of the nucleoside residues are linked by a phosphorothioate bond, or may be constituted such that all the nucleoside residues are linked by a phosphorothioate bond. Additionally, in O², a phosphorus-containing linking group linking a part of nucleoside residues may include at least one selected from the group consisting of an alkylphosphonic residue, an alkyl phosphate residue, and a substituted phosphoramide residue.

The oligonucleotide including the structure represented by Formula (A) may include a modified oligonucleotide having a chemical modification pattern represented by any one of the above Formulas (1) to (20).

The target-editing oligonucleotide can be synthesized according to a method described in known literature (for example, see Nucleic Acids Reserch, 12, 4539 (1984)), using a commercially available synthesizer (for example, Model 392 based on the phosphoramidite method by PerkinElmer, Inc.). As the phosphoramidite reagent to be used, a commercially available reagent may be used, or a reagent suitably synthesized according to a method described in known literature may be used. After coupling the phosphoramidite reagent, a phosphorothioate bond can be introduced by allowing a reagent such as sulfur, tetraethylthiuram disulfide (TETD, Applied Biosystems, Inc.), Beaucage reagent (Glen Research), xanthan hydride, phenylacetyl disulfide, or [(N,N-dimethylaminomethylidene)amino]-3H-1,2,4-dithiazoline-3-thione (DDTT) to react (see, for example, Tetrahedron Letters, 32, 3005 (1991), J. Am. Chem. Soc., 112, 1253 (1990), and PCT/WO98/54198). Additionally, by using an alkylphosphonamidite reagent, an alkylphosphoramidite reagent, or the like instead of the phosphoramidite reagent, an alkylphosphonate bond, an alkylphosphonate bond, or the like can be introduced. As the alkylphosphonamidite reagent and the alkylphosphoramidite reagent, commercially available reagents may be used, or reagents suitably synthesized according to a method described in known literature may be used.

The target-editing oligonucleotide (hereinafter, also simply referred to as the "oligonucleotide") may be used in the form of a pharmaceutically acceptable salt. The "pharmaceutically acceptable salt" is a salt of the oligonucleotide. Examples of such salts include: alkali metal salts such as sodium salts, potassium salts, and lithium salts; alkaline earth metal salts such as calcium salts and magnesium salts; metal salts such as aluminium salts, iron salts, zinc salts, copper salts, nickel salts, and cobalt salts; inorganic salts such as ammonium salts; amine salts such as organic salts including t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts, and tris(hydroxymethyl)aminomethane salts; hydrohalic acid salts such as hydrofluorides, hydrochlorides, hydrobromides, and hydroiodides; inorganic acid salts such as nitrates, perchlorates, sulfates, and phosphates; lower alkanesulfonates such as methanesulfonates, trifluoromethanesulfonates, and ethanesulfonates; arylsulfonates such as benzenesulfonates and p-toluenesulfonates; organic acid salts such as acetates, malates, fumarates, succinates, citrates, tartrates, oxalates, and maleates; and amino acid salts such as glycine salts, lysine salts, arginine salts, ornithine salts, glutamates, and aspartates. The form of the pharmaceutically acceptable salt of the oligonucleotide is preferably an alkali metal salt of the oligonucleotide, and more preferably a sodium salt. These salts can be produced by known methods.

Additionally, the oligonucleotide and a pharmaceutically acceptable salt thereof exist in the form of a solvate (for example, a hydrate) in some cases, and may be such a solvate.

In some cases, the oligonucleotide and a pharmaceutically acceptable salt thereof exist in the form of an optical isomer derived from the asymmetry of the phosphorus atom, and the oligonucleotide of the present invention encompasses such an optical isomer. Such an optical isomer can be synthesized by a known method (for example, Org. Lett., 114, 967 (2009), Bioorganic & Medicinal Chemistry Letters, 8, 2359 (1998), and the like).

The oligonucleotide and a pharmaceutically acceptable salt thereof may have a delivery molecule such as GalNAc, cholesterol, or a fatty acid bound at the 5' end or the 3' end via a linker, a phosphodiester bond (including a phosphorothioate bond), or the like. Examples of such delivery molecules and linkers are described in Bioconjugate Chem. 2019, 30, 366. The oligonucleotide to which a delivery molecule is bound and a pharmaceutically acceptable salt thereof can be produced by a known production method. The oligonucleotide and a pharmaceutically acceptable salt thereof that are herein described also encompass a form in which such a delivery molecule is bound.

When used for the treatment of a disease, the oligonucleotide, a pharmaceutically acceptable salt thereof, or a solvate thereof can be administered alone or mixed with a suitable, pharmaceutically acceptable excipient, diluent, or the like, orally in the form of a tablet, capsule, granule, powder, syrup, or the like, or parenterally in the form of an injection, suppository, patch, external preparation, or the like.

These formulations are produced by a well-known method using additives such as excipients (for example, sugar derivatives such as lactose, sucrose, glucose, mannitol, and sorbitol; starch derivatives such as corn starch, potato starch, α starch, and dextrin; cellulose derivatives such as crystalline cellulose; gum arabic; dextran; organic excipients such as pullulan; silicate derivatives such as light anhydrous silicic acid, synthetic aluminium silicate, calcium silicate, and magnesium aluminometasilicate; phosphates such as calcium hydrogen phosphate; carbonates such as calcium carbonate; and inorganic excipients such as sulfates such as calcium sulfate), lubricants (for example, stearic acid; metal stearates such as calcium stearate and magnesium stearate; talc; colloidal silica; waxes such as beeswax and spermaceti; boric acid; adipic acid; sulfates such as sodium sulfate; glycol; fumaric acid; sodium benzoate; DL-leucine; lauryl sulfates such as sodium lauryl sulfate and magnesium lauryl sulfate; silicas such as anhydrous silicic acid and silicic acid hydrate; and the above starch derivatives), binders (for example, hydroxypropyl cellulose, hydroxypropyl methylcellulose, polyvinylpyrrolidone, macrogol, and compounds similar to the above excipients), disintegrants (for example, cellulose derivatives such as low-substituted hydroxypropyl cellulose, carboxymethyl cellulose, carboxymethyl cellulose calcium, and internally crosslinked sodium carboxymethyl cellulose; chemically modified starches/celluloses such as carboxymethyl starch, sodium carboxymethyl starch, and crosslinked polyvinylpyrrolidone), emulsifiers (for example, colloidal clays such as bentonite and veegum; metal hydroxides such as magnesium hydroxide and aluminium hydroxide; anionic surfactants such as sodium lauryl sulfate and calcium stearate; cationic surfactants such as benzalkonium chloride; and nonionic surfactants such as polyoxyethylene alkyl ethers, polyoxyethylene sorbitan fatty acid esters, and sucrose fatty acid esters), stabilizers (para-hydroxybenzoate esters such as methylparaben and propylparaben; alcohols such as chlorobutanol, benzyl alcohol, and phenylethyl alcohol; benzalkonium chloride; phenols such as phenol and cresol; thimerosal; dehydroacetic acid; sorbic acid and the like), corrigents (for example, commonly used sweeteners, acidulants, flavors, and the like), and diluents.

A therapeutic agent containing the oligonucleotide, a pharmaceutically acceptable salt thereof, or a solvate thereof may contain the oligonucleotide in an amount of 0.1 to 250 µmoles/ml, and preferably 1 to 50 µmoles/ml. Additionally, the therapeutic agent may be constituted by combining the oligonucleotide, a pharmaceutically acceptable salt thereof, or a solvate thereof in a predetermined amount, a carbohydrate or polyhydric alcohol at 0.02 to 10% w/v, and a pharmaceutically acceptable surfactant at 0.01 to 0.4% w/v.

As the carbohydrate, at least one of a monosaccharide or a disaccharide is particularly preferable. Examples of these carbohydrates and polyhydric alcohols include glucose, galactose, mannose, lactose, maltose, mannitol, and sorbitol. These may be used singly or in combination.

Additionally, preferable examples of the surfactant include polyoxyethylene sorbitan mono- to tri-esters, alkylphenyl polyoxyethylenes, sodium taurocholate, sodium cholate, and polyhydric alcohol esters. Among these, polyoxyethylene sorbitan mono-to tri-esters are particularly preferable, and here, particularly preferable esters are oleate, laurate, stearate, and palmitate. These may be used singly or in combination.

More preferably, the therapeutic agent containing the oligonucleotide, a pharmaceutically acceptable salt thereof, or a solvate thereof may contain pharmaceutically acceptable neutral salt, for example, sodium chloride, potassium chloride, and/or calcium chloride at 0.03 M to 0.09 M.

More preferably, the therapeutic agent including the oligonucleotide, a pharmaceutically acceptable salt thereof, or a solvate thereof can contain a pharmaceutically acceptable buffer at 0.002 M to 0.05 M. Examples of preferable buffers include sodium citrate, sodium glycinate, sodium phosphate, and tris(hydroxymethyl)aminomethane. These buffers may be used singly or in combination.

Furthermore, the above-mentioned therapeutic agent may be supplied in a solution state. However, for the purpose of stabilizing the oligonucleotide to prevent a decrease in the therapeutic effect, such as when the agent needs to be stored for a certain period, it is usually preferable to freeze-dry the agent. In this case, the agent may be subjected to reconstructed with a dissolution liquid (such as distilled water for injection) when used, that is, may be brought into the state of a liquid to be administered, and used. Accordingly, the therapeutic agent of the present invention also encompasses the agent in a freeze-dried state for use by being reconstructed with a dissolution liquid so that each component falls within a predetermined concentration range. For the purpose of promoting the solubility of the freeze-dried product, the agent may contain an amino acid such as albumin or glycine.

When administered to a human, the oligonucleotide, a pharmaceutically acceptable salt thereof, or a solvate thereof may be administered by subcutaneous injection, intravenous infusion, or intravenous injection once or in several installments, for example, at a dose of approximately 0.01 mg/kg to 100 mg/kg (body weight), preferably 0.1 mg/kg to 20 mg/kg (body weight), per day for an adult. The dose and the frequency of administration may be suitably changed depending on the type of a disease, symptoms, age, administration method, and the like.

### Method of treating disease

The method of treating a disease includes a step of administering the above-mentioned therapeutic agent to a subject. The method of treating a disease may include a step of administering a pharmacologically effective amount of the above-mentioned therapeutic agent to a subject. As used herein, "treatment" may be any treatment performed for a disease, and examples thereof include therapy, improvement, suppression of progression (prevention of exacerbation), prevention, and the like of the disease (suitably, treatment or prevention). The subject of treatment may be a warm-blooded animal including a human, a non-human warm-blooded animal, or a human.

### Method of site-specific editing of target RNA

The method of site-specific editing of a target RNA includes a step of bringing a target RNA into contact with a target-editing oligonucleotide, which is an oligonucleotide that induces site-specific editing of the target RNA, in the presence of adenosine deaminase. By the target-editing oligonucleotide forming a partial double strand with the target RNA and recruiting adenosine deaminase, an adenosine residue contained in the target RNA can be site-specifically converted to an inosine residue. The method of site-specific editing of a target RNA may further include a step of preparing the target-editing oligonucleotide.

The method of site-specific editing of a target RNA can be performed, for example, by introducing the above-described target-editing oligonucleotide into a eukaryotic cell having the target RNA. As a method of introducing the target-editing oligonucleotide into a eukaryotic cell, any of various techniques used in nucleic acid drugs can be suitably selected and applied. The method of site-specific editing of a target RNA may be performed in vitro or in vivo.

As another aspect, the present invention also encompasses the use of the target-editing oligonucleotide in the manufacture of a pharmaceutical composition used for the treatment of a disease (for example, a genetic disease), the use of the target-editing oligonucleotide in the treatment of a disease (for example, a genetic disease), and the target-editing oligonucleotide for use in the treatment of a disease (for example, a genetic disease).

### EXAMPLES

Below, the present invention is specifically described with reference to Examples, but the present invention is not limited to these Examples.

### Reference Example 6

### Synthesis of 5'-O-(4,4'-dimethoxytrityl)-2'-deoxy-4-N-benzoyladenosine-3'-O-(ethyl-N,N-diisopropyl)phosphonamidite

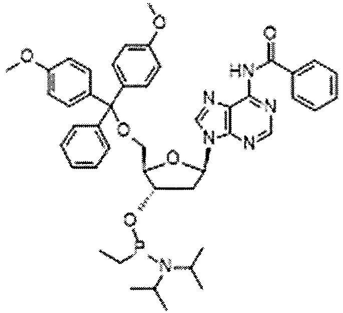

After 5'-O-(4,4'-dimethoxytrityl)-2'-deoxy-4-N-benzoyladenosine (1.85 g, 2.81 mmol) was azeotropically dehydrated with anhydrous acetonitrile/anhydrous toluene (1/1), the resultant material was dissolved in anhydrous dichloromethane (16 ml). To the resultant solution, tetrazole N,N-diisopropylamine salt (0.48 g) was added. Under ice cooling, a solution of P-ethyl-N,N,N',N'-tetraisopropylphosphonamidite *(*Tetrahedron Lett. 1989, 30, 2445-24482, 1.26 g, 5.62 mmol) in anhydrous dichloromethane (3 ml) was added. The resultant mixture was stirred for 50 minutes under ice cooling, and then stirred at room temperature overnight. The solvent was concentrated under reduced pressure (to an approximately 1/2 volume), and then diluted with toluene. The resultant solution was purified using silica gel chromatography (hexane (containing 1% triethylamine):ethyl acetate (containing 1% triethylamine) = 90:10 -> 50/50 -> 30/70) to yield the title compound in the form of a colorless solid (1.41 g, 1.73 mmol, 61%).

¹H-NMR (400 MHz, CDCl₃): 0.9-1.20 (15H, m), 1.42-1.70 (2H, m), 2.55-2.72 (1H, m), 2.85 (1H, m), 3.29-3.56 (4H, m), 3.77 (6H, s), 4.28 (1H, m), 4.65 (1H, m), 6.49 (1H, m), 6.78 (4H, m), 7.17-7.32 (7H, m), 7.39 (2H, m), 7.53 (2H, m), 7.61 (1H, m), 8.02 (2H, m), 8.19 (1H, m), 8.76 (1H, m), 9.03 (1H, m).
FAB-MAS (mNBA): 817 (M+H)⁺

### Reference Example 7

### Synthesis of 5'-O-(4,4'-dimethoxytrityl)-2'-deoxy-4-N-benzoyladenosine-3'-O-(n-propyl-N,N-diisopropyl)phosphonamidite

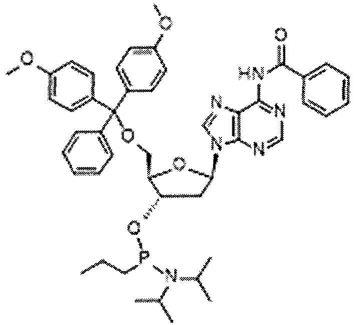

P-propyl-N,N,N',N'-tetraisopropylphosphonamidite was synthesized as follows. Bis(diisopropylamino)chlorophosphine (5.18 g, 0.0194 mol) was suspended in anhydrous diethyl ether (340 ml). Under ice/acetone bath cooling, a 2 M diethyl ether solution of n-propylmagnesium chloride (12 ml, 0.024 mol) was added dropwise. The resultant mixture was stirred for 1 hour under ice/acetone bath cooling, and then stirred at room temperature for 1.5 hours. At room temperature, n-hexane (350 ml) was added, and insoluble matter was removed by filtration through Celite. Then, the solvent was concentrated under reduced pressure, and the resultant residue was purified using NH silica gel chromatography (n-hexane, 100%) to yield the title compound in the form of a colorless oil (4.36 g, 0.0176 mol, 82%).

¹H-NMR (400 MHz, CDCl₃): 1.00 (3H, t, J=7.3 Hz), 1.13 (24H, dd, J1=40.3 Hz, J2=6.7 Hz), 1.33-1.47 (2H, m), 1.58-1.67 (2H, d, m), 3.33-3.58 (4H, m).

After 5'-O-(4,4'-dimethoxytrityl)-2'-deoxy-4-N-benzoyladenosine (1.93 g, 2.93 mmol) was azeotropically dehydrated with anhydrous acetonitrile/anhydrous toluene (1/1), the resultant material was dissolved in anhydrous dichloromethane (17 ml). To the resultant solution, tetrazole N,N-diisopropylamine salt (0.5 g) was added. Under ice cooling, a solution of P-propyl-N,N,N',N'-tetraisopropylphosphonamidite (1.45 g, 5.28 mmol) in anhydrous dichloromethane (3 ml) was added. The resultant mixture was stirred for 50 minutes under ice cooling, and then stirred at room temperature overnight. The solvent was concentrated under reduced pressure (to an approximately 1/2 volume), and then diluted with toluene. The resultant solution was purified using silica gel chromatography (hexane (containing 1% triethylamine):ethyl acetate (containing 1% triethylamine) = 90:10 -> 50/50 -> 25/75) to yield the title compound in the form of a colorless solid (1.89 g, 2.27 mmol, 76%).

¹H-NMR (400 MHz, CDCl₃): 0.95-1.70 (19H, m), 2.51-2.72 (1H, m), 2.85 (1H, m), 3.29-3.57 (4H, m), 3.77 (6H, s), 4.28 (1H, m), 4.64 (1H, m), 6.49 (1H, m), 6.78 (4H, m), 7.17-7.30 (7H, m), 7.39 (2H, m), 7.53 (2H, m), 7.60 (1H, m), 8.02 (2H, m), 8.18 (1H, m), 8.76 (1H, m), 9.03 (1H, m).
FAB-MAS (mNBA): 831 (M+H)⁺

### Reference Example 8

### Synthesis of 5'-O-(4,4'-dimethoxytrityl)-2'-deoxy-4-N-benzoyladenosine-3'-O-(isopropyl-N,N-diisopropyl)phosphonamidite

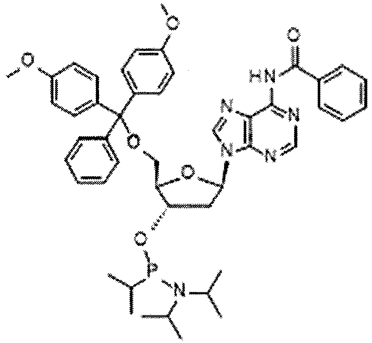

P-isopropyl-N,N,N',N'-tetraisopropylphosphonamidite was synthesized as follows. Bis(diisopropylamino)chlorophosphine (5.27 g, 0.0198 mol) was suspended in anhydrous diethyl ether (350 ml). Under ice/acetone bath cooling, a 1 M diethyl ether solution of isopropylmagnesium chloride (24 ml, 0.024 mol) was added dropwise. The resultant mixture was stirred for 1 hour under ice/acetone bath cooling, and then stirred at room temperature for 1 hour. At room temperature, n-hexane (350 ml) was added, and insoluble matter was removed by filtration through Celite. Then, the solvent was concentrated under reduced pressure, and the resultant residue was purified using NH silica gel chromatography (n-hexane, 100%) to yield the title compound in the form of a colorless solid (3.73 g, 0.0136 mol, 69%).

¹H-NMR (400 MHz, CDCl₃): 1.03 (6H, dd, J1=17.1 Hz, J2=6.7 Hz), 1.17 (24H, dd, J1=40.3 Hz, J2=6.7 Hz), 2.19 (1H, m), 3.26-3.61 (4H, m).

A mixture of 5'-O-(4,4'-dimethoxytrityl)-2'-deoxy-4-N-benzoyladenosine (3 g, 4.56 mmol), 4,5-dicyanoimidazole (0.54 g), and anhydrous dichloromethane (25 ml) was stirred at room temperature for 10 minutes. Then, a solution of P-isopropyl-N,N,N',N'-tetraisopropylphosphonamidite (1.91 g, 6.96 mmol) in anhydrous dichloromethane (5 ml) was added, and the resultant mixture was stirred at room temperature overnight. The solvent was concentrated under reduced pressure (to an approximately 1/2 volume), and then diluted with toluene. The resultant solution was purified using NH silica gel chromatography (hexane (containing 1% triethylamine):ethyl acetate (containing 1% triethylamine) = 90:10 -> 65/35 -> 40/60), and then repurified using silica gel chromatography (hexane (containing 1% triethylamine):ethyl acetate (containing 1% triethylamine) = 90:10 -> 25/75) to yield the title compound in the form of a colorless solid (306 mg, 0.37 mmol, 8%).

¹H-NMR (400 MHz, CDCl₃): 0.86-1.20 (18H, m), 1.94 (1H, m), 2.58-2.77 (1H, m), 2.87 (1H, m), 3.29-3.58 (4H, m), 3.77 (6H, s), 4.28-4.38 (1H, m), 4.66 (1H, m), 6.49 (1H, m), 6.78 (4H, m), 7.16-7.35 (7H, m), 7.39 (2H, m), 7.53 (2H, m), 7.60 (1H, m), 8.02 (2H, m), 8.18 (1H, m), 8.75 (1H, m), 9.01 (1H, m).
FAB-MAS (mNBA): 831 (M+H)⁺

### Reference Example 9

### Synthesis of 5'-O-(4,4'-dimethoxytrityl)-2'-deoxy-4-N-benzoyladenosine-3'-O-(n-butyl-N,N-diisopropyl)phosphonamidite

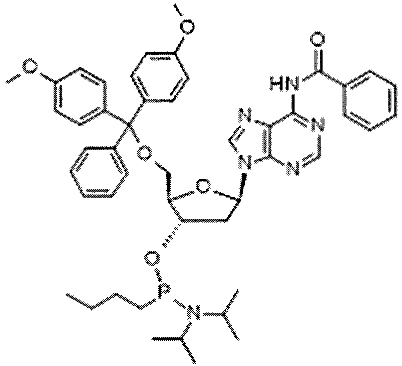

In anhydrous dichloromethane (12 ml), 5'-O-(4,4'-dimethoxytrityl)-2'-deoxy-4-N-benzoyladenosine (1.500 g, 2.28 mmol) was suspended. Tetrazole N,N-diisopropylamine salt (0.391 g) was added at room temperature, and the resultant mixture was stirred at the same temperature for 10 minutes. Then, a separately prepared solution of P-n-butyl-N,N,N',N'-tetraisopropylphosphonamidite (Tetraheron Lett. 1989, 30, 2445-2448; 0.987 g, 3.42 mmol) in anhydrous dichloromethane (3 ml) was added, and the resultant mixture was stirred at room temperature for 21 hours. After the reaction solution was evaporated under reduced pressure, the residue was purified using silica gel chromatography (hexane containing 1% triethylamine: ethyl acetate containing 1% triethylamine = 60:40 -> 40/60) to yield the title compound in the form of a white foamy solid (1.689 g, 2.00 mmol, 88%).

¹H-NMR (500 MHz, CDCl₃): 0.88-0.98 (4H, m), 1.03-1.95 (18H, m), 2.54-2.73 (1H, m), 2.80-2.89 (1H, m), 3.27-3.58 (4H, m), 3.77 (6H, s), 4.24-4.33 (1H, m), 4.57-4.70 (1H, m), 6.46-6.53 (1H, m), 6.74-6.81 (4H, m), 7.16-7.32 (6H, m), 7.35-7.42 (2H, m), 7.50-7.56 (2H, m), 7.58-7.63 (1H, m), 7.99-8.05 (2H, m), 8.18 (1H, d, J=9.3 Hz), 8.75 (1H, d, J=3.9 Hz), 8.97 (1H, d, J=5.9 Hz).
ESI-MAS: 843 (M-H)⁺

### Reference Example 10

### Synthesis of 5'-O-(4,4'-dimethoxytrityl)-2'-deoxy-4-N-benzoyladenosine-3'-O-(3-methoxypropyl-N,N-diisopropyl)phosphonamidite

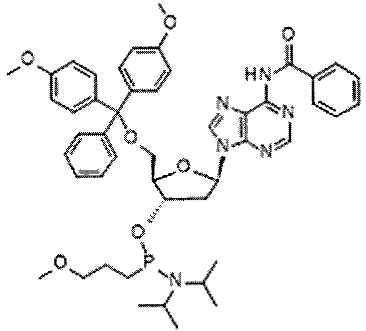

Under a nitrogen stream, 5'-O-(4,4'-dimethoxytrityl)-2'-deoxy-4-N-benzoyladenosine (1.5 g, 2.3 mmol) and 4,5-dicyanoimidazole (340 mg, 2.6 mmol) were dissolved in anhydrous dichloromethane (10 ml), and the resultant mixture was stirred for 10 minutes. A separately prepared solution of P-3-methoxypropyl-N,N,N',N'-tetraisopropylphosphonamidite *(*Nucleic Acids Research (2019), 47(11), 5465-5479; 870 mg, 2.9 mmol) in anhydrous dichloromethane (6 ml) was added dropwise at room temperature, and the resultant mixture was stirred overnight. The reaction solvent was distilled off under reduced pressure, and the resultant residue was purified using silica gel chromatography (hexane containing 1% triethylamine: ethyl acetate containing 1% triethylamine = 2:3) to yield the title compound in the form of a white solid (907 mg, 1.13 mmol, 46%).

¹H-NMR (400MHz, CDCl₃):1.06-1.19 (12H, m), 1.49-1.70 (4H, m), 2.53-2.61 and 2.65-2.72 (1H, each m), 2.83-2.90 (1H, m), 3.29-3.58 (10H, m), 3.77 (7H, s), 4.24-4.31 (1H, m), 4.60-4.69 (1H, m), 6.46-6.51 (1H, m), 6.76-6.80 (4H, m), 7.19-7.31 (6H, m), 7.37-7.41 (2H, m), 7.51-7.56 (2H, m), 7.58-7.64 (1H, m), 8.00-8.04 (2H, m), 8.18 (1H, d, J=7.3Hz), 8.75 (1H, d, J=3.7Hz), 8.96 (1H, d, J=4.3Hz).
ESI-MAS: 778 (M-NiPr₂+OH)⁺

### Reference Example 11

### Synthesis of 5'-O-(4,4'-dimethoxytrityl)-2'-O-methyl-4-N-benzoyladenosine-3'-O-(ethyl-N,N-diisopropyl)phosphonamidite

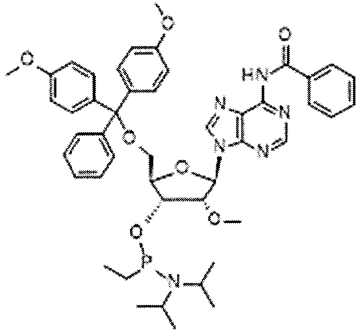

Under a nitrogen stream, 5'-O-(4,4'-dimethoxytrityl)-2'-O-methyl-4-N-benzoyladenosine (1.2 g, 1.7 mmol) and 4,5-dicyanoimidazole (227 mg, 1.9 mmol) were dissolved in anhydrous dichloromethane (20 ml). A separately prepared solution of P-ethyl-N,N,N',N'-tetraisopropylphosphonamidite (619 mg, 2.4 mmol) in anhydrous dichloromethane (5 ml) was added dropwise at room temperature, and the resultant mixture was stirred overnight. The reaction solvent was distilled off under reduced pressure, and the resultant residue was purified using silica gel chromatography (hexane containing 1% triethylamine:ethyl acetate containing 1% triethylamine = 1:1) to yield the title compound in the form of a white solid (1.0 g, 1.18 mmol, 70%).

¹H-NMR (400 MHz, CDCl₃):0.84-0.92 (4H, m), 0.97-1.09 (6H, m), 1.15 (2H, d, J=6.7Hz), 1.56-1.67 (1H, m), 1.70-1.80 (1H, m), 3.32-3.40 (1H, m), 3.44-3.59 (6H, m), 3.78 (7H, s), 4.31-4.39 (1H, m), 4.46-4.54 (1H, m), 4.62-4.67 (1H, m), 6.18 and 6.21 (1H, each d, J=6.1 and 3.7Hz), 6.78-6.82 (4H, m), 7.20-7.36 (8H, m), 7.42-7.47 (2H, m), 7.51-7.55 (2H, m), 7.59-7.64 (1H, m), 8.00-8.04 (2H, m), 8.18 and 8.28 (1H, each s), 8.73 and 8.76 (1H, each s), 8.96 and 9.00 (1H, each s).
ESI-MAS: 764 (M-NiPr₂+OH)⁺

### Reference Example 12

### Synthesis of 5'-O-(4,4'-dimethoxytrityl)-2'-O-methyl-4-N-benzoyladenosine-3'-O-(n-propyl-N,N-diisopropyl)phosphonamidite

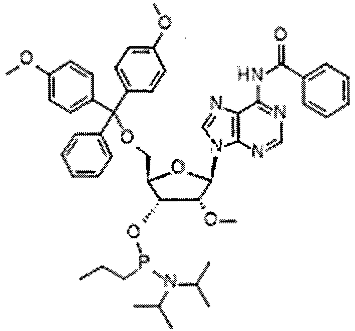

Under a nitrogen stream, 5'-O-(4,4'-dimethoxytrityl)-2'-O-methyl-4-N-benzoyladenosine (1.2 g, 1.7 mmol) and 4,5-dicyanoimidazole (227 mg, 1.9 mmol) were dissolved in anhydrous dichloromethane (20 ml), and the resultant mixture was stirred for 10 minutes. A separately prepared solution of P-n-propyl-N,N,N',N'-tetraisopropylphosphonamidite (700 mg, 2.6 mmol) in anhydrous dichloromethane (5 ml) was added dropwise at room temperature, and the resultant mixture was stirred overnight. The reaction solvent was distilled off under reduced pressure, and the resultant residue was purified using silica gel chromatography (hexane containing 1% triethylamine:ethyl acetate containing 1% triethylamine = 1:1) to yield the title compound in the form of a white solid (1.09 g, 1.27 mmol, 74%).

¹H-NMR (400 MHz, CDCl₃):0.88 and 0.94 (3H, each t, J=6.7 and 7.3Hz), 1.00-1.09 (12H, m), 1.15 (2H, d, J=6.7Hz), 1.41-1.52 (2H, m), 1.58-1.66 (1H, m), 1.71-1.81 (1H, m), 3.33-3.40 (1H, m), 3.44-3.61 (6H, m), 3.78 (6H, s), 4.30-4.41 (1H, m), 4.45-4.54 (1H, m), 4.59-4.68 (1H, m), 6.17 (1H, d, J=6.1Hz), 6.21 (1H, d, J=3.7Hz), 6.78-6.82 (4H, m), 7.20-7.36 (6H, m), 7.41-7.47 (2H, m), 7.51-7.56 (2H, m), 7.59-7.64 (1H, m), 8.00-8.04 (2H, m), 8.18 and 8.26 (1H, each s), 8.73 and 8.76 (1H, each s), 8.96 and 9.00 (1H, each s).
ESI-MAS: 778 (M-NiPr₂+OH)⁺

### Reference Example 13

### Synthesis of 5'-O-(4,4'-dimethoxytrityl)-2'-O-methyl-4-N-benzoyladenosine-3'-O-(isopropyl-N,N-diisopropyl)phosphonamidite

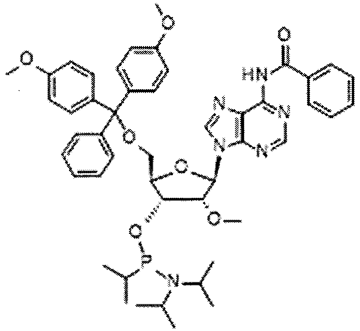

Under a nitrogen stream, 5'-O-(4,4'-dimethoxytrityl)-2'-O-methyl-4-N-benzoyladenosine (1.9 g, 2.8 mmol) and 4,5-dicyanoimidazole (364 mg, 3.1 mmol) were dissolved in anhydrous dichloromethane (10 ml), and the resultant mixture was stirred for 10 minutes. A separately prepared solution of P-isopropyl-N,N,N',N'-tetraisopropylphosphonamidite (1.1 g, 4.2 mmol) in anhydrous dichloromethane (6 ml) was added dropwise at room temperature, and the resultant mixture was stirred overnight. The reaction solvent was distilled off under reduced pressure, and the resultant residue was purified using silica gel chromatography (hexane containing 1% triethylamine:ethyl acetate containing 1% triethylamine = 2:1 -> 1:1) to yield the title compound in the form of a white solid (310 mg, 0.36 mmol, 13%).

¹H-NMR (400 MHz, CDCl₃):0.86-1.23 (18H, m), 1.96-2.06 (1H, m), 3.34-3.56 (8H, m), 3.78 and 3.80 (6H, each s), 4.13-4.40 (1H, m), 4.43-4.56 (1H, m), 4.61-4.71 (1H, m), 6.01-6.22 (1H, m), 6.78-6.82 (4H, m), 7.20-7.36 (6H, m), 7.39-7.56 (4H, m), 7.58-7.64 (1H, m), 7.96-8.05 (2H, m), 8.16 and 8.27 (1H, each s), 8.73 and 8.75 (1H, each s), 8.99 and 9.05 (1H, each s).
ESI-MAS: 778 (M-NiPr₂+OH)⁺

### Reference Example 14

### Synthesis of 5'-O-(4,4'-dimethoxytrityl)-2'-O-methyl-4-N-benzoyladenosine-3'-O-(n-butyl-N,N-diisopropyl)phosphonamidite

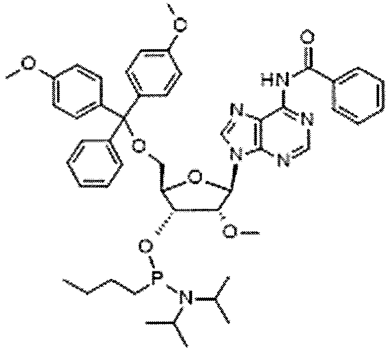

Under a nitrogen stream, 5'-O-(4,4'-dimethoxytrityl)-2'-O-methyl-4-N-benzoyladenosine (1.5 g, 2.2 mmol) and 4,5-dicyanoimidazole (285 mg, 2.4 mmol) were dissolved in anhydrous dichloromethane (12 ml), and the resultant mixture was stirred for 10 minutes. A separately prepared solution of P-n-butyl-N,N,N',N'-tetraisopropylphosphonamidite (825 mg, 2.9 mmol) in anhydrous dichloromethane (5 ml) was added dropwise at room temperature, and the resultant mixture was stirred overnight. The reaction solvent was distilled off under reduced pressure, and the resultant residue was purified using silica gel chromatography (hexane containing 1% triethylamine:ethyl acetate containing 1% triethylamine = 1:1) to yield the title compound in the form of a white solid (1.3 g, 1.49 mmol, 68%).

¹H-NMR (400 MHz, CDCl₃):0.88 and 0.92 (3H, each t, J=7.0 and 7.3Hz), 1.03-1.16 (12H, m), 1.26-1.46 (4H, m), 1.56-1.79 (2H, m), 3.32-3.60 (7H, m), 3.78 (6H, s), 4.30-4.39 (1H, m), 4.46-4.54 (1H, m), 4.60-4.66 (1H, m), 6.18 and 6.21 (1H, each d, J=6.1 and 3.7Hz), 6.78-6.82 (4H, m), 7.20-7.36 (6H, m), 7.41-7.47 (2H, m), 7.49-7.56 (2H, m), 7.59-7.63 (1H, m), 8.01-8.04 (2H, m), 8.18 and 8.27 (1H, each s), 8.73 and 8.75 (1H, each s), 8.99 and 9.03 (1H, each s).
ESI-MAS: 792 (M-NiPr₂+OH)⁺

### Reference Example 15

### Synthesis of 5'-O-(4,4'-dimethoxytrityl)-2'-O-methyl-4-N-benzoyladenosine-3'-O-(3-methoxypropyl-N,N-diisopropyl)phosphonamidite

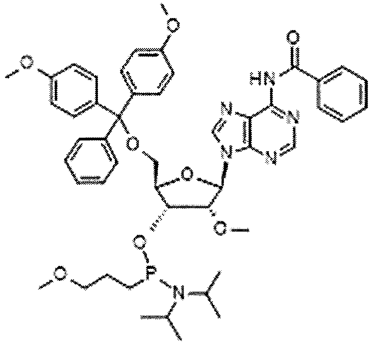

Under a nitrogen stream, 5'-O-(4,4'-dimethoxytrityl)-2'-O-methyl-4-N-benzoyladenosine (1.0 g, 1.5 mmol) and 4,5-dicyanoimidazole (190 mg, 1.7 mmol) were dissolved in anhydrous dichloromethane (5 ml), and the resultant mixture was stirred for 10 minutes. A separately prepared solution of P-3-methoxypropyl-N,N,N',N'-tetraisopropylphosphonamidite (590 mg, 2.0 mmol) in anhydrous dichloromethane (4 ml) was added dropwise at room temperature, and the resultant mixture was stirred overnight. The reaction solvent was distilled off under reduced pressure, and the resultant residue was purified using silica gel chromatography (hexane containing 1% triethylamine:ethyl acetate containing 1% triethylamine = 2:3) to yield the title compound in the form of a white solid (1.05 g, 1.18 mmol, 79%).

¹H-NMR (400 MHz, CDCl₃): 1.03-1.18 (12H, m), 1.25-1.32 (3H, m), 1.54-1.82 (3H, m), 3.28-3.61 (13H, m), 3.78 (6H, s), 4.29-4.40 (1H, m), 4.49-4.54 (1H, m), 4.60-4.69 (1H, m), 6.16 and 6.19 (1H, each d, J=6.7 and 3.7 Hz), 6.77-6.83 (4H, m), 7.20-7.36 (6H, m), 7.40-7.47 (2H, m), 7.50-7.55 (2H, m), 7.59-7.64 (1H, m), 8.00-8.04 (2H, m), 8.17 and 8.25 (1H, each s), 8.73 and 8.75 (1H, each s), 8.95 and 8.99 (1H, each s).
ESI-MAS: 808 (M-NiPr₂+OH)⁺

### Reference Example 16

### Synthesis of 5'-O-(4,4'-dimethoxytrityl)-2'-deoxy-4-N-benzoyladenosine-3'-O-(n-propyl-N,N-diisopropyl)phosphoramidite

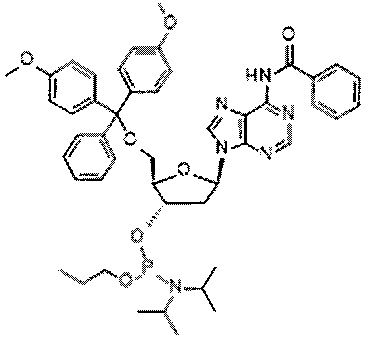

Under a nitrogen stream, 5'-O-(4,4'-dimethoxytrityl)-2'-deoxy-4-N-benzoyladenosine (1.2 g, 1.8 mmol) and 4,5-dicyanoimidazole (240 mg, 2.0 mmol) were dissolved in anhydrous dichloromethane (10 ml), and the resultant mixture was stirred for 15 minutes. A separately prepared solution of n-propyl-N,N,N',N'-tetraisopropylphosphoramidite *(*Nucleic Acids Research (2018), 46(8), 4013-4021; 630 mg, 2.2 mmol) in anhydrous dichloromethane (3 ml) was added dropwise at room temperature, and the resultant mixture was stirred overnight. The reaction solvent was distilled off under reduced pressure, and the resultant residue was purified using silica gel chromatography (hexane containing 1% triethylamine: ethyl acetate containing 1% triethylamine = 1:1) to yield the title compound in the form of a white solid (920 mg, 1.09 mmol, 60%).

¹H-NMR (400 MHz, CDCl₃): 0.87 and 0.93 (3H, each t, J=7.3 Hz), 1.10-1.26 (12H, m), 1.50-1.66 (2H, m), 2.62-2.76 (1H, m), 2.85-2.95 (1H, m), 3.32-3.75 (6H, m), 3.77 (6H, s), 4.31-4.39 (1H, m), 4.73-4.81 and 5.07-5.12 (1H, each m), 6.52 (1H, t, J=6.7 Hz), 6.75-6.80 (4H, m), 7.18-7.30 (7H, m), 7.38-7.41 (2H, m), 7.50-7.55 (2H, m), 7.58-7.63 (1H, m), 8.01-8.04 (2H, m), 8.20 (1H, d, J=7.9 Hz), 8.74 (0H, s), 8.75 (1H, s), 9.02 (1H, s).
ESI-MAS: 764 (M-NiPr₂+OH)⁺

### Reference Example 17

### Synthesis of 5'-O-(4,4'-dimethoxytrityl)-2'-deoxy-4-N-benzoyladenosine-3'-O-(n-butyl-N,N-diisopropyl)phosphoramidite

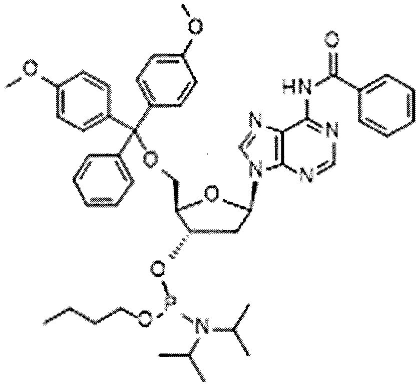

Under a nitrogen stream, 5'-O-(4,4'-dimethoxytrityl)-2'-deoxy-4-N-benzoyladenosine (1.3 g, 2.0 mmol) and 4,5-dicyanoimidazole (260 mg, 2.2 mmol) were dissolved in anhydrous dichloromethane (10 ml), and the resultant mixture was stirred for 10 minutes. A separately prepared solution of n-butyl-N,N,N',N'-tetraisopropylphosphoramidite *(*Nucleic Acids Research (2018), 46(8), 4013-4021; 720 mg, 2.4 mmol) in anhydrous dichloromethane (4 ml) was added dropwise at room temperature, and the resultant mixture was stirred overnight. The reaction solvent was distilled off under reduced pressure, and the resultant residue was purified using silica gel chromatography (hexane containing 1% triethylamine: ethyl acetate containing 1% triethylamine = 1:1) to yield the title compound in the form of a white solid (968 mg, 1.12 mmol, 56%).

¹H-NMR (400 MHz, CDCl₃): 0.86-0.93 (3H, m), 1.09-1.28 (12H, m), 1.31-1.42 (2H, m), 1.47-1.61 (2H, m), 2.62-2.75 (1H, m), 2.86-2.96 (1H, m), 3.31-3.69 (5H, m), 3.73-3.81 (7H, m), 4.31-4.39 (1H, m), 4.72-4.82 and 5.06-5.12 (1H, each m), 6.52 (1H, t, J=6.7 Hz), 6.76-6.80 (4H, m), 7.17-7.30 (7H, m), 7.38-7.41 (2H, m), 7.51-7.55 (2H, m), 7.58-7.63 (1H, m), 8.00-8.04 (2H, m), 8.20 (1H, d, J=6.1 Hz), 8.74 (0H, s), 8.75 (1H, s), 8.98 (1H, s).
ESI-MAS: 778 (M-NiPr₂+OH)⁺

### Reference Example 18

### Synthesis of 5'-O-(4,4'-dimethoxytrityl)-2'-deoxy-4-N-benzoyladenosine-3'-O-(2-methoxyethyl-N,N-diisopropyl)phosphoramidite

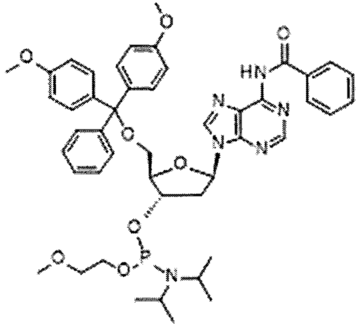

Under a nitrogen stream, 5'-O-(4,4'-dimethoxytrityl)-2'-deoxy-4-N-benzoyladenosine (1.5 g, 2.3 mmol) and 4,5-dicyanoimidazole (300 mg, 2.5 mmol) were dissolved in anhydrous dichloromethane (10 ml), and the resultant mixture was stirred for 10 minutes. A separately prepared solution of 2-methoxyethyl-N,N,N',N'-tetraisopropylphosphoramidite (J. Am. Chem. Soc. (1990), 112(21), 7475-82; 916 mg, 3.0 mmol) in anhydrous dichloromethane (5 ml) was added dropwise at room temperature, and the resultant mixture was stirred overnight. The reaction solvent was distilled off under reduced pressure, and the resultant residue was purified using silica gel chromatography (hexane containing 1% triethylamine: ethyl acetate containing 1% triethylamine = 1:1) to yield the title compound in the form of a white solid (1.2 g, 1.39 mmol, 62%).

¹H-NMR (400 MHz, CDCl₃): 1.11-1.20 (11H, m), 2.65-2.77 (1H, m), 2.87-2.96 (1H, m), 3.30 and 3.33 (3H, each s), 3.35-3.49 (3H, m), 3.53-3.82 (12H, m), 4.30-4.40 (1H, m), 4.73-4.82 (1H, m), 6.52 (1H, t, J=6.4 Hz), 6.75-6.80 (4H, m), 7.18-7.30 (7H, m), 7.38-7.42 (2H, m), 7.50-7.54 (2H, m), 7.59-7.62 (1H, m), 8.01-8.04 (2H, m), 8.20 (1H, d, J=5.5 Hz), 8.75 (1H, s), 8.98 (1H, s).
ESI-MAS: 780 (M-NiPr₂+OH)⁺

### Reference Example 19

### Synthesis of 5'-O-(4,4'-dimethoxytrityl)-2'-deoxy-4-N-benzoyladenosine-3'-O-(cyclopropyl-N,N-diisopropyl)phosphoramidite

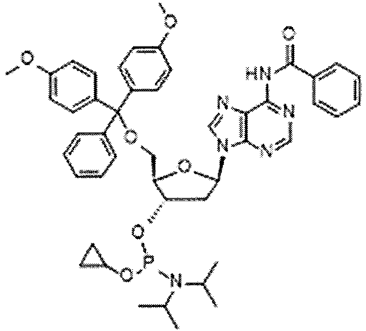

Cyclopropyl-N,N,N',N'-tetraisopropylphosphoramidite was synthesized as follows. Under a nitrogen stream, cyclopropanol (1.0 g, 20.4 mmol) was mixed with THF (10 ml), and triethylamine (4.7 ml, 40.8 mmol) was added. Then, a separately prepared solution of P-chloro-N,N,N',N'-tetraisopropylphosphonamidite (3.7 g, 17 mmol) in THF (15 ml) was added dropwise under ice cooling. Then, the resultant mixture was raised to room temperature, and stirred for 3 hours. The reaction solvent was distilled off under reduced pressure, and the resultant residue was purified using NH silica gel chromatography (eluted with hexane) to yield the title compound in the form of a colorless oil (2.8 g, 9.7 mmol, 57%).

¹H-NMR (400 MHz, CDCl₃): 0.47-0.52 (2H, m), 0.64-0.68 (2H, m), 1.15 (12H, d, J=6.7 Hz), 1.17 (12H, d, J=6.7 Hz), 3.33-3.39 (1H, m), 3.46-3.57 (4H, m).

Under a nitrogen stream, 5'-O-(4,4'-dimethoxytrityl)-2'-deoxy-4-N-benzoyladenosine (1.5 g, 2.3 mmol) and 4,5-dicyanoimidazole (300 mg, 2.5 mmol) were dissolved in anhydrous dichloromethane (10 ml), and the resultant mixture was stirred for 10 minutes. A separately prepared solution of cyclopropyl-N,N,N',N'-tetraisopropylphosphoramidite (860 mg, 3.0 mmol) in anhydrous dichloromethane (5 ml) was added dropwise at room temperature, and the resultant mixture was stirred overnight. The reaction solvent was distilled off under reduced pressure, and the resultant residue was purified using silica gel chromatography (hexane containing 1% triethylamine:ethyl acetate containing 1% triethylamine = 1:1) to yield the title compound in the form of a white solid (1.65 g, 1.95 mmol, 85%).

¹H-NMR (400 MHz, CDCl₃): 0.44-0.48 and 0.52-0.57 (2H, each m), 0.58-0.61 and 0.68-0.71 (2H, each m), 1.11-1.26 (12H, m), 2.62-2.74 (1H, m), 2.84-2.95 (1H, m), 3.26-3.45 (3H, m), 3.55-3.65 (2H, m), 3.74-3.80 (6H, m), 4.32-4.38 (1H, m), 4.72-4.79 (1H, m), 6.51 (1H, t, J=6.7 Hz), 6.76-6.81 (4H, m), 7.18-7.31 (7H, m), 7.38-7.41 (2H, m), 7.50-7.56 (2H, m), 7.58-7.64 (1H, m), 8.00-8.04 (2H, m), 8.19 (1H, d, J=9.2 Hz), 8.75 (1H, d, J=1.8 Hz), 8.97 (1H, brs).
ESI-MAS: 762 (M-NiPr₂+OH)⁺

### Reference Example 20

### Synthesis of 5'-O-(4,4'-dimethoxytrityl)-2'-deoxy-4-N-benzoyladenosine-3'-O-(cyclobutyl-N,N-diisopropyl)phosphoramidite

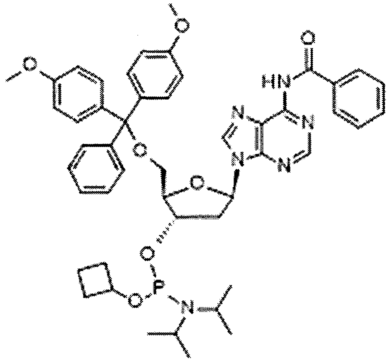

Under a nitrogen stream, 5'-O-(4,4'-dimethoxytrityl)-2'-deoxy-4-N-benzoyladenosine (1.5 g, 2.3 mmol) and 4,5-dicyanoimidazole (300 mg, 2.5 mmol) were dissolved in anhydrous dichloromethane (10 ml), and the resultant mixture was stirred for 10 minutes. A separately prepared solution of cyclobutyl-N,N,N',N'-tetraisopropylphosphoramidite (Bioorg. Med. Chem. Lett. (2012), 22(18), 5924-5929; 860 mg, 3.0 mmol) in anhydrous dichloromethane (5 ml) was added dropwise at room temperature, and the resultant mixture was stirred overnight. The reaction solvent was distilled off under reduced pressure, and the resultant residue was purified using NH silica gel chromatography (hexane containing 1% triethylamine:ethyl acetate containing 1% triethylamine = 1:1) to yield the title compound in the form of a white solid (1.63 g, 1.9 mmol, 82%).

¹H-NMR (400 MHz, CDCl₃): 1.09-1.20 (12H, m), 1.41-1.51 (1H, m), 1.59-1.69 (1H, m), 1.91-2.27 (4H, m), 2.62-2.77 (1H, m), 2.86-2.96 (1H, m), 3.30-3.45 (2H, m), 3.52-3.65 (2H, m), 3.77 (6H, s), 4.14-4.40 (2H, m), 4.68-4.78 (1H, m), 6.49-6.55 (1H, m), 6.75-6.81 (4H, m), 7.18-7.31 (7H, m), 7.38-7.43 (2H, m), 7.50-7.56 (2H, m), 7.59-7.63 (1H, m), 8.02 (2H, d, J=7.3 Hz), 8.20 (1H, d, J=7.3 Hz), 8.75 (1H, s), 8.99 (1H, s). ESI-MAS: 776 (M-NiPr₂+OH)⁺

### Reference Example 21

### Synthesis of 5'-O-(4,4'-dimethoxytrityl)-2'-O-methoxy-4-N-benzoyladenosine-3'-O-(n-propyl-N,N-diisopropyl)phosphoramidite

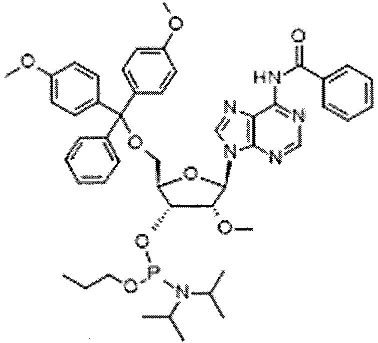

Under a nitrogen stream, 5'-O-(4,4'-dimethoxytrityl)-2'-O-methoxy-4-N-benzoyladenosine (1.3 g, 1.9 mmol) and 4,5-dicyanoimidazole (246 mg, 2.1 mmol) were dissolved in anhydrous dichloromethane (10 ml), and the resultant mixture was stirred for 10 minutes. A separately prepared solution of n-propyl-N,N,N',N'-tetraisopropylphosphoramidite (660 mg, 2.1 mmol) in anhydrous dichloromethane (4 ml) was added dropwise at room temperature, and the resultant mixture was stirred overnight. The reaction solvent was distilled off under reduced pressure, and the resultant residue was purified using silica gel chromatography (hexane containing 1% triethylamine:ethyl acetate containing 1% triethylamine = 1:1) to yield the title compound in the form of a white solid (983 mg, 1.16 mmol, 59%).

¹H-NMR (400 MHz, CDCl₃): 0.80-0.98 (3H, m), 1.07-1.26 (12H, m), 1.43-1.49 (1H, m), 1.56-1.64 (1H, m), 3.34-3.69 (9H, m), 3.78 (6H, s), 4.39-4.47 (1H, m), 4.58-4.66 (2H, m), 6.16-6.21 (1H, m), 6.78-6.82 (4H, m), 7.20-7.28 (3H, m), 7.30-7.36 (4H, m), 7.42-7.46 (2H, m), 7.50-7.55 (2H, m), 7.59-7.64 (1H, m), 8.00-8.05 (2H, m), 8.22 (1H, d, J=12.2 Hz), 8.73 (1H, t, J=3.4 Hz), 8.98 (1H, s).
ESI-MAS: 794 (M-NiPr₂+OH)⁺

### Reference Example 22

### Synthesis of 5'-O-(4,4'-dimethoxytrityl)-2'-O-methoxy-4-N-benzoyladenosine-3'-O-(n-butyl-N,N-diisopropyl)phosphoramidite

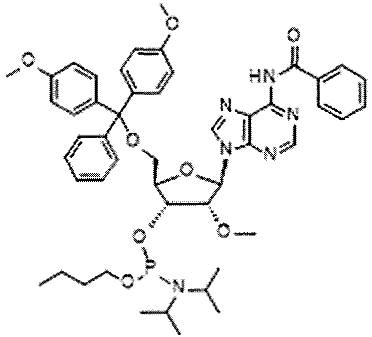

Under a nitrogen stream, 5'-O-(4,4'-dimethoxytrityl)-2'-O-methoxy-4-N-benzoyladenosine (1.3 g, 1.9 mmol) and 4,5-dicyanoimidazole (246 mg, 2.1 mmol) were dissolved in anhydrous dichloromethane (10 ml), and the resultant mixture was stirred for 10 minutes. A separately prepared solution of n-butyl-N,N,N',N'-tetraisopropylphosphoramidite (700 mg, 2.1 mmol) in anhydrous dichloromethane (4 ml) was added dropwise at room temperature, and the resultant mixture was stirred overnight. The reaction solvent was distilled off under reduced pressure, and the resultant residue was purified using silica gel chromatography (hexane containing 1% triethylamine:ethyl acetate containing 1% triethylamine = 3:2) to yield the title compound in the form of a white solid (1.05 g, 1.18 mmol, 62%).

¹H-NMR (400 MHz, CDCl₃): 0.83 and 0.88 (3H, each t, J=each 7.3 Hz), 1.05-1.26 (12H, m), 1.33-1.47 (2H, m), 1.54-1.60 (2H, m), 3.35-3.46 (2H, m), 3.48 and 3.52 (3H, each s), 3.54-3.71 (3H, m), 3.78 (7H, s), 4.38-4.48 (1H, m), 4.56-4.67 (2H, m), 6.19 (1H, t, J=4.9 Hz), 6.77-6.82 (4H, m), 7.19-7.29 (3H, m), 7.30-7.36 (4H, m), 7.42-7.47 (2H, m), 7.51-7.55 (2H, m), 7.59-7.63 (1H, m), 8.02 (2H, d, J=7.3 Hz), 8.22 (1H, d, J=10.4 Hz), 8.72-8.75 (1H, m), 8.99 (1H, s).
ESI-MAS: 808 (M-NiPr₂+OH)⁺

### Reference Example 23

### Synthesis of 5'-O-(4,4'-dimethoxytrityl)-2'-O-methoxy-4-N-benzoyladenosine-3'-O-(2-methoxyethyl-N,N-diisopropyl)phosphoramidite

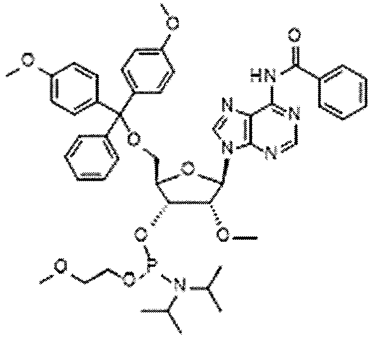

Under a nitrogen stream, 5'-O-(4,4'-dimethoxytrityl)-2'-O-methoxy-4-N-benzoyladenosine (1.5 g, 2.2 mmol) and 4,5-dicyanoimidazole (286 mg, 2.4 mmol) were dissolved in anhydrous dichloromethane (10 ml), and the resultant mixture was stirred for 10 minutes. A separately prepared solution of 2-methoxyethyl-N,N,N',N'-tetraisopropylphosphoramidite (809 mg, 2.6 mmol) in anhydrous dichloromethane (5 ml) was added dropwise at room temperature, and the resultant mixture was stirred overnight. The reaction solvent was distilled off under reduced pressure, and the resultant residue was purified using silica gel chromatography (hexane containing 1% triethylamine:ethyl acetate containing 1% triethylamine = 1:1). The resultant crude purified product was dissolved in ethyl acetate, washed with water, and then dried over anhydrous sodium sulfate. After filtration, the filtrate solvent was distilled off under reduced pressure, and diethyl ether was added to the resultant residue to yield the title compound in the form of a white solid (1.2 g, 1.34 mmol, 61%).

¹H-NMR (400 MHz, CDCl₃): 1.07-1.26 (12H, m), 3.21 (3H, s), 3.29 (3H, s), 3.34-3.68 (11H, m), 3.78 (6H, s), 3.82-4.21 (1H, m), 4.37-4.48 (1H, m), 4.59-4.67 (2H, m), 6.18-6.20 (1H, m), 6.78-6.82 (4H, m), 7.20-7.28 (3H, m), 7.31-7.35 (4H, m), 7.42-7.46 (2H, m), 7.51-7.55 (2H, m), 7.59-7.64 (1H, m), 8.02 (2H, t, J=4.3 Hz), 8.23 (1H, d, J=6.1 Hz), 8.74 (1H, d, J=1.2 Hz), 8.99 (1H, s).
ESI-MAS: 810 (M-NiPr₂+OH)⁺

### Reference Example 24

### Synthesis of 5'-O-(4,4'-dimethoxytrityl)-2'-O-methoxy-4-N-benzoyladenosine-3'-O-(cyclopropyl-N,N-diisopropyl)phosphoramidite

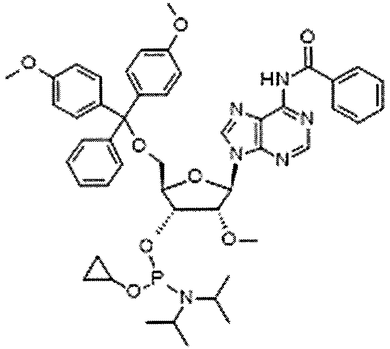

Under a nitrogen stream, 5'-O-(4,4'-dimethoxytrityl)-2'-O-methoxy-4-N-benzoyladenosine (1.5 g, 2.2 mmol) and 4,5-dicyanoimidazole (286 mg, 2.4 mmol) were dissolved in anhydrous dichloromethane (10 ml), and the resultant mixture was stirred for 10 minutes. A separately prepared solution of cyclopropyl-N,N,N',N'-tetraisopropylphosphoramidite (761 mg, 2.6 mmol) in anhydrous dichloromethane (5 ml) was added dropwise at room temperature, and the resultant mixture was stirred overnight. Water (10 ml) was added to the reaction solution, and the resultant mixture was extracted with dichloromethane. The organic layer was washed with saturated brine and dried over anhydrous sodium sulfate. After filtration, the filtrate solvent was distilled off under reduced pressure, and the resultant residue was purified using silica gel chromatography (hexane containing 1% triethylamine: ethyl acetate containing 1% triethylamine = 1:1) to yield the title compound in the form of a white solid (1.32 g, 1.51 mmol, 69%).

¹H-NMR (400 MHz, CDCl₃): 0.40-0.69 (4H, m), 1.07-1.23 (12H, m), 3.22-3.47 (2H, m), 3.47 (3H, s), 3.49 (3H, s), 3.50-3.67 (3H, m), 3.78 (6H, s), 4.38-4.44 (1H, m), 4.57-4.66 (2H, m), 6.17 (1H, dd, J=11.6, 4.9 Hz), 6.78-6.84 (4H, m), 7.19-7.28 (3H, m), 7.30-7.36 (4H, m), 7.42-7.46 (2H, m), 7.51-7.55 (2H, m), 7.59-7.64 (1H, m), 8.01-8.05 (2H, m), 8.21 (1H, d, J=12.2 Hz), 8.73 (1H, d, J=1.8 Hz), 9.00 (1H, s).
ESI-MAS: 792 (M-NiPr₂+OH)⁺

### Reference Example 25

### Synthesis of 5'-O-(4,4'-dimethoxytrityl)-2'-O-methoxy-4-N-benzoyladenosine-3'-O-(cyclobutyl-N,N-diisopropyl)phosphoramidite

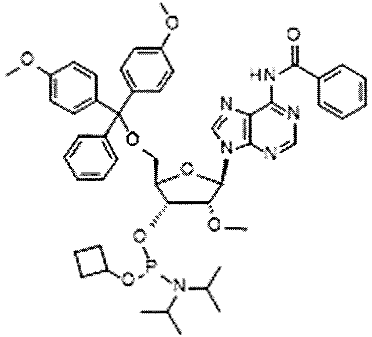

Under a nitrogen stream, 5'-O-(4,4'-dimethoxytrityl)-2'-O-methoxy-4-N-benzoyladenosine (1.5 g, 2.2 mmol) and 4,5-dicyanoimidazole (286 mg, 2.4 mmol) were dissolved in anhydrous dichloromethane (10 ml), and the resultant mixture was stirred for 10 minutes. A separately prepared solution of cyclobutyl-N,N,N',N'-tetraisopropylphosphoramidite (800 mg, 2.6 mmol) in anhydrous dichloromethane (5 ml) was added dropwise at room temperature, and the resultant mixture was stirred overnight. Water (10 ml) was added to the reaction solution, and the resultant mixture was extracted with dichloromethane. The organic layer was washed with saturated brine and dried over anhydrous sodium sulfate. After filtration, the filtrate solvent was distilled off under reduced pressure, and the resultant residue was purified using silica gel chromatography (hexane containing 1% triethylamine: ethyl acetate containing 1% triethylamine = 1:1) to yield the title compound in the form of a white solid (1.36 g, 1.53 mmol, 69%).

¹H-NMR (400 MHz, CDCl₃): 1.05-1.22 (12H, m), 1.38-1.48 (1H, m), 1.57-1.93 (2H, m), 1.99-2.25 (3H, m), 3.33-3.42 (1H, m), 3.47-3.66 (6H, m), 3.78 (6H, s), 4.11-4.66 (4H, m), 6.18 (1H, t, J=5.2 Hz), 6.78-6.84 (4H, m), 7.19-7.27 (3H, m), 7.30-7.36 (4H, m), 7.42-7.47 (2H, m), 7.50-7.56 (2H, m), 7.58-7.64 (1H, m), 8.22 (1H, d, J=12.2 Hz), 8.74 (1H, d, J=1.2 Hz), 8.99 (1H, s).
ESI-MAS: 806 (M-NiPr₂+OH)⁺

### Reference Example 26

### Synthesis of 5'-O-(4,4'-dimethoxytrityl)-2'-deoxy-2'-fluoro-4-N-benzoyladenosine-3'-O-(ethyl-N,N-diisopropyl)phosphoramidite

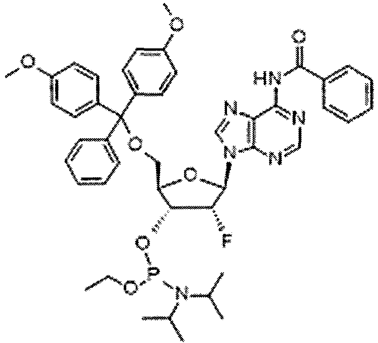

In anhydrous dichloromethane (13 ml), 5'-O-(4,4'-dimethoxytrityl)-2'-deoxy-2'-fluoro-4-N-benzoyladenosine (1.24 g, 1.83 mmol) was dissolved. Under ice cooling, N,N-diisopropylethylamine (0.19 ml), 1H-tetrazole (154 mg), and a solution of ethyl-N,N,N',N'-tetraisopropylphosphorodiamidite (J. Org. Chem. 1995, 60, 925-930; 0.92 g, 3.33 mmol) in anhydrous dichloromethane (2 ml) were added. The resultant mixture was stirred overnight under ice cooling (allowed to warm to room temperature naturally). The reaction mixture was directly purified using NH silica gel chromatography (hexane:ethyl acetate = 70:30 -> 15/85), and then repurified using diol silica gel chromatography (hexane:ethyl acetate = 90:10 -> 35/65) to yield the title compound in the form of a colorless solid (1.25 g, 1.47 mmol, 80%).

¹H-NMR (400 MHz, CDCl₃): 1.05-1.26 (15H, m), 3.49 (1H, m), 3.53-3.70 (5H, m), 3.77 (6H, s), 4.39 (1H, br), 4.79-5.02 (1H, m), 5.60-5.78 (1H, m), 6.28-6.36 (1H, m), 6.78 (4H, m), 7.16-7.32 (7H, m), 7.39 (2H, m), 7.53 (2H, m), 7.62 (1H, m), 8.02 (2H, m), 8.25 (1H, m), 8.77 (1H, m), 8.98 (1H, br).
FAB-MAS (mNBA): 851 (M+H)⁺

### Reference Example 27

### Synthesis of 5'-O-(4,4'-dimethoxytrityl)-2'-deoxy-2'-fluoro-2-N-isobutyrylguanosine-3'-O-(ethyl-N,N-diisopropyl)phosphoramidite

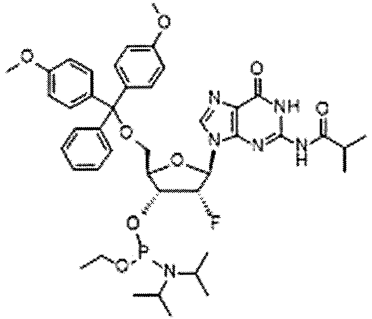

In anhydrous dichloromethane (10 ml), 5'-O-(4,4'-dimethoxytrityl)-2'-deoxy-2'-fluoro-2-N-isobutyrylguanosine (1.17 g, 1.78 mmol) was dissolved. Under ice cooling, N,N-diisopropylethylamine (0.18 ml), 1H-tetrazole (150 mg), and a solution of ethyl-N,N,N',N'-tetraisopropylphosphorodiamidite (0.89 g, 3.22 mmol) in anhydrous dichloromethane (2 ml) were added. The resultant mixture was stirred overnight under ice cooling (allowed to warm to room temperature naturally). The reaction mixture was directly purified using diol silica gel chromatography (hexane:ethyl acetate = 80:20 -> 20/80) to yield the title compound in the form of a colorless solid (1.19 g, 1.43 mmol, 80%).

¹H-NMR (400 MHz, CDCl₃): 0.74-1.23 (21H, m), 1.62-1.76 (1H, m), 3.14 (1H, m), 3.44-3.68 (11H, m), 4.31 (1H, m), 4.73 (1H, m), 5.65-5.83 (1H, m), 6.00-6.11 (1H, m), 6.79 (4H, m), 7.20-7.26 (3H, m), 7.38 (4H, m), 7.49-7.59 (3H, m), 7.84 (1H, m), 11.90 (1H, br).
FAB-MAS (mNBA): 833 (M+H)⁺

### Reference Example 28

### Synthesis of 5'-O-(4,4'-dimethoxytrityl)-2'-deoxy-2'-fluoro-4-N-benzoylcytidine-3'-O-(ethyl-N,N-diisopropyl)phosphoramidite

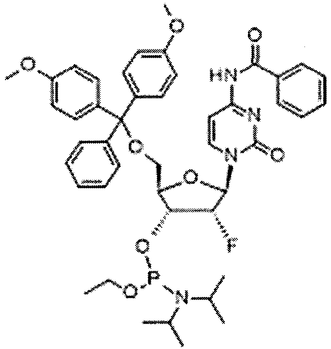

In anhydrous dichloromethane (12 ml), 5'-0-(4,4'-dimethoxytrityl)-2'-deoxy-2'-fluoro-4-N-benzoylcytidine (1.37 g, 2.1 mmol) was dissolved. Under ice cooling, N,N-diisopropylethylamine (0.21 ml), 1H-tetrazole (177 mg), and a solution of ethyl-N,N,N',N'-tetraisopropylphosphorodiamidite (1.05 g, 3.8 mmol) in anhydrous dichloromethane (2 ml) were added. The resultant mixture was stirred overnight under ice cooling (allowed to warm to room temperature naturally). The reaction mixture was directly purified using NH silica gel chromatography (hexane:ethyl acetate = 70:30 -> 15/85) to yield the title compound in the form of a colorless solid (1.59 g, 1.92 mmol, 91%).

¹H-NMR (400 MHz, CDCl₃): 1.00-1.24 (15H, m), 3.47-3.75 (6H, m), 3.83 (6H, s), 4.32 (1H, m), 4.42-4.70 (1H, m), 4.98-5.14 (1H, m), 6.13-6.18 (1H, m), 6.87 (4H, m), 7.10 (1H, br), 7.28-7.37 (7H, m), 7.40-7.47 (2H, m), 7.49-7.54 (2H, m), 7.61 (1H, m), 7.87 (2H, m), 8.61 (1H, br).
FAB-MAS (mNBA): 827 (M+H)⁺

### Reference Example 29

### Synthesis of 5'-O-(4,4'-dimethoxytrityl)-2'-deoxy-2'-fluorouridine-3'-O-(ethyl-N,N-diisopropyl)phosphoramidite

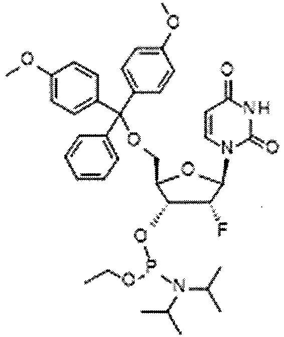

In anhydrous dichloromethane (14 ml), 5'-O-(4,4'-dimethoxytrityl)-2'-deoxy-2'-fluorouridine (1.58 g, 2.88 mmol) was dissolved. Under ice cooling, N,N-diisopropylethylamine (0.29 ml), 1H-tetrazole (242 mg), and a solution of ethyl-N,N,N',N'-tetraisopropylphosphorodiamidite (1.44 g, 5.21 mmol) in anhydrous dichloromethane (2 ml) were added. The resultant mixture was stirred overnight under ice cooling (allowed to warm to room temperature naturally). The reaction mixture was directly purified using NH silica gel chromatography (hexane:ethyl acetate = 50:50 -> 0/100 -> ethyl acetate / methanol = 95/5) to obtain the title compound in the form of a colorless solid (1.94 g, 2.68 mmol, 93%).

¹H-NMR (400 MHz, CDCl₃): 1.04-1.29 (15H, m), 3.48-3.80 (12H, m), 4.24 (1H, m), 4.48-4.73 (1H, m), 4.90-5.05 (1H, m), 5.19 (1H, m), 6.08 (1H, m), 6.84 (4H, m), 7.24-7.33 (7H, m), 7.36-7.42 (2H, m), 8.03 (1H, m), 8.26 (1H, br).
FAB-MAS (mNBA): 724 (M+H)⁺

### Reference Example 30

### Synthesis of 5'-O-(4,4'-dimethoxytrityl)-2'-deoxyinosine-3'-O-(ethyl-N,N-diisopropyl)phosphoramidite

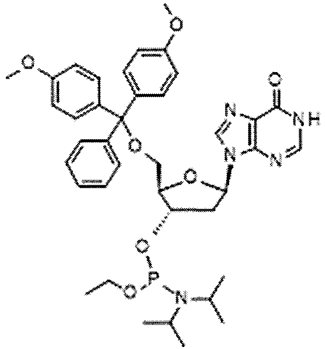

In anhydrous dichloromethane (20 ml), 5'-O-(4,4'-dimethoxytrityl)-2'-deoxyinosine (2.00 g, 3.61 mmol) was dissolved. To the resultant solution, dicyanoimidazole (426 mg, 3.61 mmol) was added. To the resultant mixture, ethyl-N,N,N',N'-tetraisopropylphosphorodiamidite (1.49 g, 5.42 mmol) was added dropwise, and the resultant mixture was stirred at room temperature overnight. After the reaction solution was concentrated under reduced pressure, the resultant residue was purified using silica gel chromatography (eluted with 1% triethylamine hexane solution:1% triethylamine ethyl acetate solution = 7:3 -> 0:10) to yield the title compound in the form of a colorless foamy solid (1.67 g, 2.29 mmol, 63%).

¹H-NMR (400 MHz, CDCl₃) 1.05-1.31 (15H, m), 2.58-2.79 (2H, m), 3.31-3.39 (2H, m), 3.51-3.73 (4H, m), 3.77 (6H, s), 4.30-4.39 (1H, m), 4.66-4.75 (1H, m), 6.42 (1H, t, J=7.0 Hz), 6.76-6.82 (4H, m), 7.15-7.26 (3H, m), 7.27-7.33 (4H, m), 7.38-7.44 (2H, m), 7.96-8.00 (2H, m).
ESI-MAS (hexafluoroisopropanol/Et₃N): 831 (M+Et₃N+H)⁺

### Reference Example 31

### Synthesis of 5'-O-(4,4'-dimethoxytrityl)-2'-deoxy-purine riboside-3'-O-(ethyl-N,N-diisopropyl)phosphoramidite

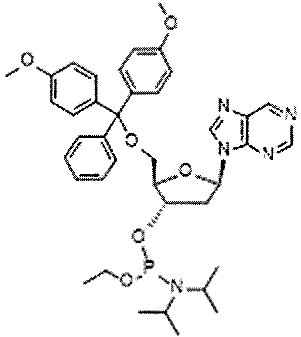

In anhydrous dichloromethane (20 ml), 5'-O-(4,4'-dimethoxytrityl)-2'-deoxy-purine riboside *(*Nucleic Acids Research (1986), 14(20), 8135-53; 2.00 g, 3.72 mmol) was dissolved, and diisopropylammonium tetrazole (699 mg, 4.09 mmol) was added thereto. To the resultant mixture, ethyl-N,N,N',N'-tetraisopropylphosphorodiamidite (1.54 g, 5.57 mmol) was added dropwise, and the resultant mixture was stirred at room temperature for 7 hours. After the reaction solution was concentrated under reduced pressure, the resultant residue was purified using silica gel chromatography (eluted with 1% triethylamine hexane solution:1% triethylamine ethyl acetate solution = 4:1 -> 0:10) to yield the title compound in the form of a colorless foamy solid (2.86 g, 3.72 mmol, 100%).

¹H-NMR (400 MHz, CDCl₃) 1.06-1.30 (15H, m), 2.61-2.96 (2H, m), 3.33-3.76 (6H, m), 3.78 (6H, s), 4.36 (1H, dd, J=16.0, 4.0 Hz), 4.72-4.82 (1H, m), 6.52-6.57 (1H, m), 6.75-6.80 (4H, m), 7.17-7.25 (3H, m), 7.27-7.32 (4H, m), 7.36-7.42 (2H, m), 8.27 (1H, d, J=8.0 Hz), 8.91 (1H, s), 9.12 (1H, s).
ESI-MAS (hexafluoroisopropanol/Et₃N): 714 (M+H)⁺

### Reference Example 1

Human β-actin (hACTB; GenBank accession No. NM_001101.5) was selected as a target RNA. The base sequence (SEQ ID NO: 1) shown in Table 1 was selected as the base sequence of a target-editing oligonucleotide. In Table 1, SEQ ID NO: 2 shows a region that contains an adenosine residue serving as an editing target in the target RNA, and corresponds to the target-editing oligonucleotide (SEQ ID NO: 1). The underlined portion corresponds to a target-corresponding nucleoside residue. It is considered that the target-editing oligonucleotide (SEQ ID NO: 1; the lower row), the base sequence of which is shown in Table 1 can take, for example, the following structure with the target RNA (SEQ ID NO: 2; the upper row).

Furthermore, the base sequences shown in Table 1 (SEQ ID NOs: 126 and 127) were selected as base sequences of the target-editing oligonucleotides targeting the human A1AT gene (hA1AT) or the mouse GAPDH gene (mGAPDH) as the target RNA.

**[Table 1]**

| | Sequence (5'-3') | SEQ ID NO: |
|---|---|---|
| hACTB Oigonucleotide | AAAGCAUGCCAUCACCUCCCCUGU | 1 |
| hACTB | ACAGGGGAGGUGAUAGCAUUGCUUU | 2 |
| hA_{1AT} Oigonucleotide | GUCCCUUCUCGUCGAUGGUCAGCA | 126 |
| mGAPDH Oigonucleotide | GAGGGCCCCCAGGCCCCUCCUGUU | 127 |

U A
3' - UUUCGU ACG UAGUGGAGGGACA - 5'
| | | | | | | | | | | | | | | | | | | | | | | |
5' - AAAGCA – UGCC AUCACCUCCCCUGU - 3'

### Examples 1 to 83 and Comparative Examples 1 to 22

The target-editing oligonucleotides of Examples 1 to 83 and Comparative Examples 1 to 22 were obtained by introducing modified oligonucleosides and phosphorus-containing linking groups as shown in the modification patterns of Table 2 to Table 5 on the basis of the base sequences of the target-editing oligonucleotides in Table 1. The target-editing oligonucleotides of Examples 1 to 83 and Comparative Examples 1 to 22 were synthesized in accordance with the phosphoramidite method (Nucleic Acids Research, 12, 4539 (1984), Nature Communications 6, Article number: 6317 (2015)), using the raw materials shown below.

"2'-O-methylnucleoside" linked by a phosphodiester bond or a phosphorothioate bond was synthesized using a phosphoramidite product described in Nucleic Acids Research 17, 3373 (1989). "DNA" linked by a phosphodiester bond or a phosphorothioate bond was synthesized using a phosphoramidite product described in Nucleic Acids Research 11, 4539 (1984). "2'-deoxy-2'-fluoronucleoside" linked by a phosphodiester bond or a phosphorothioate bond was synthesized using a phosphoramidite product described in J. Med. Chem. 36, 831 (1993). "2'-O,4'-C-methylene nucleosides" linked by a phosphodiester bond or a phosphorothioate bond was synthesized using a phosphoramidite product described in WO99/14226. "2'-O,4'-C-methylene nucleoside" linked by a phosphodiester bond or a phosphorothioate bond was synthesized using a compound described in Example 14 (5'-O-dimethoxytrityl-2'-0,4'-C-ethylene-6-N-benzoyladenosine-3'-O-(2-cyanoethyl-N,N-diisopropyl)phosphoramidite), Example 27 (5'-O-dimethoxytrityl-2'-0,4'-C-ethylene-2-N-isobutyrylguanosine-3'-O-(2-cyanoethyl-N,N-diisopropyl)phosphoramidite), Example 22 (5'-O-dimethoxytrityl-2'-O,4'-C-ethylene-4-N-benzoyl-5-methylcytidine-3'-O-(2-cyanoethyl-N,N-diisopropyl)phosphoramidite), or Example 9 (5'-O-dimethoxytrityl-2'-0,4'-C-ethylene-5-methyluridine-3'-O-(2-cyanoethyl-N,N-diisopropyl)phosphoramidite) of JP2000-297097A. "2'-deoxyinosine" linked by a phosphodiester bond or a phosphorothioate bond was synthesized using a phosphoramidite product (deoxy Inosine CED phosphoramidite (ANP-6411, manufactured by ChemGenes Corporation)).

"DNA" linked by "methylphosphonate (PM)" or "methylthiophosphonate (PSM)" was synthesized using a P-methylphosphonamidite product (deoxy Adenosine(N-bz) P-methylphosphonamidite (ANP-7551, manufactured by ChemGenes Corporation); or deoxyInosine P-methylphosphonamidite (ANP-7558, manufactured by ChemGenes Corporation)). "2'-O-methylnucleoside" linked by "methylphosphonate (PM)" or "methylthiophosphonate (PSM)" was synthesized using a phosphonamidite product (2'-O-MethylAdenosine(N-bz) P-methylphosphonamidite (ANP-8551, manufactured by ChemGenes Corporation); 2'-O-MethylCytidine(N-acetyl) P-methylphosphonamidite (ANP-8556, manufactured by ChemGenes Corporation); 2'-O-MethylGuanosine(N-ibu) P-methylphosphonamidite (ANP-8553, manufactured by ChemGenes Corporation); or 2'-O-MethylUridine P-methylphosphonamidite (ANP-8554, manufactured by ChemGenes Corporation)). "2'-deoxyadenosine" linked by "ethyl phosphate (POE)" was synthesized using a phosphoramidite product (deoxyAdenosine(N-bz) P-ethoxyphosphoramidite (ANP-6541, manufactured by ChemGenes Corporation)). "2'-O-methylnucleoside" linked by "ethyl phosphate (POE)" was synthesized using a phosphoramidite product (2'-O-MethylAdenosine(N-bz) ethylphosphoramidite (ANP-7221, manufactured by ChemGenes); 2'-O-MethylCytidine(N-bz) ethylphosphoramidite (ANP-7222, manufactured by ChemGenes); 2'-O-MethylGuanosine(N-ibu) ethylphosphoramidite (ANP-7223, manufactured by ChemGenes); or 2'-O-MethylUridine ethylphosphoramidite (ANP-7225, manufactured by ChemGenes Corporation)). "2'-deoxyadenosine" linked by "isopropyl phosphate (POIP)" was synthesized using a phosphoramidite product (deoxyAdenosine(N-bz) P-isopropoxyphosphoramidite (ANP-6901, manufactured by ChemGenes Corporation)).

"2'-deoxyadenosine" linked by "ethylphosphonate (PE)" was synthesized using the phosphonamidite product of Reference Example 6. "2'-deoxyadenosine" linked by "propylphosphonate (PP)" was synthesized using the phosphonamidite product of Reference Example 7. "2'-deoxyadenosine" linked by "isopropylphosphonate (PIP)" was synthesized using the phosphonamidite product of Reference Example 8. "2'-deoxyadenosine" linked by "butylphosphonate (PB)" was synthesized using the phosphonamidite product of Reference Example 9. "2'-deoxyadenosine" linked by "methoxypropylphosphonate (PMOP)" was synthesized using the phosphonamidite product of Reference Example 10. "2'-O-methyladenosine" linked by "ethylphosphonate (PE)" was synthesized using the phosphonamidite product of Reference Example 11. "2'-O-methyladenosine" linked by "propylphosphonate (PP)" was synthesized using the phosphonamidite product of Reference Example 12. "2'-O-methyladenosine" linked by "isopropylphosphonate (PIP)" was synthesized using the phosphonamidite product of Reference Example 13. "2'-O-methyladenosine" linked by "butylphosphonate (PB)" was synthesized using the phosphonamidite product of Reference Example 14. "2'-O-methyladenosine" linked by "methoxypropylphosphonate (PMOP)" was synthesized using the phosphonamidite product of Reference Example 15.

"2'-deoxyadenosine" linked by "propyl phosphate (POP)" was synthesized using the phosphoramidite product of Reference Example 16. "2'-deoxyadenosine" linked by "butyl phosphate (POB)" was synthesized using the phosphoramidite product of Reference Example 17. "2'-deoxyadenosine" linked by "methoxyethyl phosphate (POMOE)" was synthesized using the phosphoramidite product of Reference Example 18. "2'-deoxyadenosine" linked by "cyclopropyl phosphate (POcP)" was synthesized using the phosphoramidite product of Reference Example 19. "2'-deoxyadenosine" linked by "cyclobutyl phosphate (POcB)" was synthesized using the phosphoramidite product of Reference Example 20. "2'-O-methyladenosine" linked by "propyl phosphate (POP)" was synthesized using the phosphoramidite product of Reference Example 21. "2'-O-methyladenosine" linked by "butyl phosphate (POB)" was synthesized using the phosphoramidite product of Reference Example 22. "2'-O-methyladenosine" linked by "methoxyethyl phosphate (POMOE)" was synthesized using the phosphoramidite product of Reference Example 23. "2'-O-methyladenosine" linked by "cyclopropyl phosphate (POcP)" was synthesized using the phosphoramidite product of Reference Example 24. "2'-O-methyladenosine" linked by "cyclobutyl phosphate (POcB)" was synthesized using the phosphoramidite product of Reference Example 25.

"2'-deoxy-2'-fluoroadenosine" linked by "ethyl phosphate (POE)" was synthesized using the phosphoramidite product of Reference Example 26. "2'-deoxy-2'-fluoroguanosine" linked by "ethyl phosphate (POE)" was synthesized using the phosphoramidite product of Reference Example 27. "2'-deoxy-2'-fluorocytidine" linked by "ethyl phosphate (POE)" was synthesized using the phosphoramidite product of Reference Example 28. "2'-deoxy-2'-fluorouridine" linked by "ethyl phosphate (POE)" was synthesized using the phosphoramidite product of Reference Example 29.

"2'-deoxyinosine" linked by "ethyl phosphate (POE)" was synthesized using the phosphoramidite product of Reference Example 30. "2'-deoxy-purine riboside" or "nebularine" linked by "ethyl phosphate (POE)" was synthesized using the phosphoramidite product of Reference Example 31.

A moiety containing "N-methanesulfonylphosphoramide (PMs)" was synthesized using the method described in Nucleic Acids Research, (2021) 49, 9026-9041. A moiety containing "1,3-dimethylimidazolidin-2-ylidene phosphate (PN)" was synthesized using the method described in Front. Pharmacol. 2019; 10: 1049.

Deprotection of an acyl group in an oligonucleotide containing an alkyl phosphonate, alkyl thiophosphonate, or alkyl phosphate triester was performed using ethylenediamine (Tetrahedron Letter, 37, 8691 (1996)).

The modification patterns of the target-editing oligonucleotides of Examples 1 to 83 and Comparative Examples 1 to 22 are shown in Table 2 to Table 5.

**[Table 2]**

| | Sequece name | Sequence (5'-3') | Molecular weight | SEQ ID NO: |
|---|---|---|---|---|
| Example 1 | AD1_hACTB.39_PM01 | A(M)^A(L)^A(M)^G(L)^C(M)^A(F)^U(F)^G(M)^C(M)^c^a(PM)U(M)^C(F)^A(M)(PM)C(F)^ C(M)^U(F)^C(M)^C(F)^C(M)^C(L)^U(M)^G(L)^U(M) | 8076.05 | 15 |
| Example 2 | AD1_hACTB.39_PM02 | A(M)^A(L)^A(M)^G(L)^C(M)^A(F)^U(F)^G(M)^C(M)^c^a(PM)U(M)^C(F)^A(M)^C(F)^C(M )^U(F)^C(M)^C(F)^C(M)^C(L)^U(M)^G(L)^U(M) | 8094.22 | 16 |
| Example 3 | AD1_hACTB.39_PM03 | A(M)^A(L)^A(M)^G(L)^C(M)^A(F)^U(F)^G(M)^C(M)^c^a^U(M)^C(F)^A(M)(PM)C(F)^C(M )^U(F)^C(M)^C(F)^C(M)^C(L)^U(M)^G(L)^U(M) | 8093.92 | 17 |
| Comparative Example 1 | AD1_hACTB.39_PM04 | A(M)^A(L)^A(M)^G(L)^C(M)^A(F)^U(F)^G(M)^C(M)^c^a^U(M)(PM)C(F)^A(M)^C(F)^C(M )^U(F)^C(M)^C(F)^C(M)^C(L)^U(M)^G(L)^U(M) | 8094.18 | 18 |
| Comparative Example 2 | AD1_hACTB.39_PM05 | A(M)(PM)A(L)^A(M)^G(L)^C(M)^A(F)^U(F)^G(M)^C(M)^c^a^U(M)^C(F)^A(M)^C(F)^C(M )^U(F)^C(M)^C(F)^C(M)^C(L)^U(M)^G(L)^U(M) | 8093.48 | 19 |
| Comparative Example 3 | AD1_hACTB.39_PM06 | A(M)^A(L)^A(M)(PM)G(L)^C(M)^A(F)^U(F)^G(M)^C(M)^c^a^U(M)^C(F)^A(M)^C(F)^C(M )^U(F)^C(M)^C(F)^C(M)^C(L)^U(M)^G(L)^U(M) | 8094.09 | 20 |
| Comparative Example 4 | AD1_hACTB.39_PM07 | A(M)^A(L)^A(M)^G(L)^C(M)(PM)A(F)^U(F)^G(M)^C(M)^c^a^U(M)^C(F)^A(M)^C(F)^C(M )^U(F)^C(M)^C(F)^C(M)^C(L)^U(M)^G(L)^U(M) | 8093.48 | 21 |
| Comparative Example 5 | AD1_hACTB.39_PM08 | A(M)^A(L)^A(M)^G(L)^C(M)pA(F)^U(F)^G(M)^C(M)(PM)c^a^U(M)^C(F)^A(M)^C(F)^C(M )^U(F)^C(M)^C(F)^C(M)^C(L)^U(M)^G(L)^U(M) | 8075.52 | 22 |
| Comparative Example 6 | AD1_hACTB.39_PM09 | A(M)^A(L)^A(M)^G(L)^C(M)^A(F)^U(F)^G(M)^C(M)^c^a^U(M)^C(F)^A(M)^C(F)^C(M)(P M)U(F)^C(M)^C(F)^C(M)^C(L)^U(M)^G(L)^U(M) | 8093.11 | 23 |
| Comparative Example 7 | AD1_hACTB.39_PM10 | A(M)^A(L)^A(M)^G(L)^C(M)^A(F)^U(F)^G(M)^C(M)^c^a^U(M)^C(F)^A(M)^C(F)^C(M)^U( F)^C(M)(PM)C(F)^C(M)^C(L)^U(M)^G(L)^U(M) | 8092.93 | 24 |
| Comparative Example 8 | AD1_hACTB.39_PM11 | A(M)^A(L)^A(M)^G(L)^C(M)^A(F)^U(F)^G(M)^C(M)^c^a^U(M)^C(F)^A(M)^C(F)^C(M)^U( F)^C(M)^C(F)^C(M)(PM)C(L)^U(M)^G(L)^U(M) | 8093.23 | 25 |
| Comparative Example 9 | AD1_hACTB.39_PM12 | A(M)^A(L)^A(M)^G(L)^C(M)^A(F)^U(F)^G(M)^C(M)^c^a^U(M)^C(F)^A(M)^C(F)^C(M)^U( F)^C(M)^C(F)^C(M)^C(L)^U(M)(PM)G(L)^U(M) | 8091.75 | 28 |
| Example 4 | AD1_hACTB.39_POE01 | A(M)^A(L)^A(M)^G(L)^C(M)^A(F)^U(F)^G(M)^C(M)^c^a(POE)U(M)^C(F)^A(M)(POE)C( F)^C(M)^U(F)^C(M)^C(F)^C(M)^C(L)^U(M)^G(L)^U(M) | 8132.24 | 27 |
| Example 5 | AD1_hACTB.39_POE02 | A(M)^A(L)^A(M)^G(L)^C(M)^A(F)^U(F)^G(M)^C(M)^c^a(POE)U(M)^C(F)^A(M)^C(F)^C( M)^U(F)^C(M)^C(F)^C(M)^C(L)^U(M)^G(L)^U(M) | 8119.99 | 28 |
| Example 6 | AD1_hACTB.39_POE03 | A(M)^A(L)^A(M)^G(L)^C(M)^A(F)^U(F)^G(M)^C(M)^c^a^U(M)^C(F)^A(M)(POE)C(F)^C( M)^U(F)^C(M)^C(F)^C(M)^C(L)^U(M)^G(L)^U(M) | 8121.07 | 29 |
| Example 7 | AD1_hACTB.39_POIP02 | A(M)^A(L)^A(M)^G(L)^C(M)^A(F)^U(F)^G(M)^C(M)^c^a(POIP)U(M)^C(F)^A(M)^C(F)^C( M)^U(F)^C(M)^C(F)^C(M)^C(L)^U(M)^G(L)^U(M) | 8133.89 | 30 |
| Example 8 | AD1_hACTB.39_PSM01 | A(M)^A(L)^A(M)^G(L)^C(M)^A(F)^U(F)^G(M)^C(M)^c^a(PSM)U(M)^C(F)^A(M)(PSM)C( F)^C(M)^U(F)^C(M)^C(F)^C(M)^C(L)^U(M)^G(L)^U(M) | 8105.51 | 31 |
| Example 9 | AD1_hACTB.39_PSM02 | A(M)^A(L)^A(M)^G(L)^C(M)^A(F)^U(F)^G(M)^C(M)^c^a(PSM)U(M)^C(F)^A(M)^C(F)^C( M)^U(F)^C(M)^C(F)^C(M)^C(L)^U(M)^G(L)^U(M) | 8111.87 | 32 |
| Example 10 | AD1_hACTB.39_PSM03 | A(M)^A(L)^A(M)^G(L)^C(M)^A(F)^U(F)^G(M)^C(M)^c^a^U(M)^C(F)^A(M)(PSM)C(F)^C( M)^U(F)^C(M)^C(F)^C(M)^C(L)^U(M)^G(L)^U(M) | 8106.97 | 33 |
| Comparative Example 10 | AD1_hACTB.39_PM13 | A(M)^A(L)^A(M)^G(L)^C(M)^A(F)^U(F)^G(M)(PM)C(M)^c^a^U(M)^C(F)^A(M)^C(F)^C(M )^U(F)^C(M)^C(F)^C(M)^C(L)^U(M)^G(L)^U(M) | 8095.64 | 34 |
| Example 11 | AD1_hACTB.39_PE02 | A(M)^A(L)^A(M)^G(L)^C(M)^A(F)^U(F)^G(M)^C(M)^c^a(PE)U(M)^C(F)^A(M)^C(F)^C(M) ^U(F)^C(M)^C(F)^C(M)^C(L)^U(M)^G(L)^U(M) | 8104.98 | 35 |
| Example 12 | AD1_hACTB.39_PP02 | A(M)^A(L)^A(M)^G(L)^C(M)^A(F)^U(F)^G(M)^C(M)^c^a(PP)U(M)^C(F)^A(M)^C(F)^C(M) ^U(F)^C(M)^C(F)^C(M)^C(L)^U(M)^G(L)^U(M) | 8118.18 | 36 |
| Example 13 | AD1_hACTB.39_PIP02 | A(M)^A(L)^A(M)^G(L)^C(M)^A(F)^U(F)^G(M)^C(M)^c^a(PIP)U(M)^C(F)^A(M)^C(F)^C(M )^U(F)^C(M)^C(F)^C(M)^C(L)^U(M)^G(L)^U(M) | 8119.01 | 37 |
| Comparative Example 11 | AD1_hACTB.39_PO01 | A(M)^A(L)^A(M)^G(L)^C(M)^A(F)^U(F)^G(M)^C(M)^c^apU(M)^C(F)^A(M)pC(F)^C(M)^U( F)^C(M)^C(F)^C(M)^C(L)^U(M)^G(L)^U(M) | 8079.75 | 38 |
| Comparative Example 12 | AD1_hACTB.39_RO02 | A(M)^A(L)^A(M)^G(L)^C(M)^A(F)^U(F)^G(M)^C(M)^c^apU(M)^C(F)^A(M)^C(F)^C(M)^U( F)^C(M)^C(F)^C(M)^C(L)^U(M)^G(L)^U(M) | 8096.00 | 39 |
| Comparative Example 13 | AD1_hACTB.39_PO03 | A(M)^A(L)^A(M)^G(L)^C(M)^A(F)^U(F)^G(M)^C(M)^c^a^U(M)^C(F)^A(M)pC(F)^C(M)^U( F)^C(M)^C(F)^C(M)^C(L)^U(M)^G(L)^U(M) | 8095.82 | 40 |
| Comparative Example 14 | AD1_hACTB.39_POE14 | A(M)^A(L)^A(M)^G(L)^C(M)^A(F)(POE)U(F)^G(M)^C(M)^c^a^U(M)^C(F)^A(M)^C(F)^C( M)^U(F)^C(M)^C(F)^C(M)^C(L)^U(M)^G(L)^U(M) | 8121 | 41 |
| Comparative Example 15 | AD1_hACTB.39_POE15 | A(M)^A(L)^A(M)^G(L)^C(M)^A(F)^U(F)(POE)G(M)^C(M)^c^a^U(M)^C(F)^A(M)^C(F)^C( M)^U(F)^C(M)^C(F)^C(M)^C(L)^U(M)^G(L)^U(M) | 8122 | 42 |
| Comparative Example 16 | AD1_hACTB.39_POE16 | A(M)^A(L)^A(M)^G(L)^C(M)^A(F)^U(F)^G(M)^C(M)^c^a^U(M)^C(F)(POE)A(M)^C(F)^C( M)^U(F)^C(M)^C(F)^C(M)^C(L)^U(M)^G(L)^U(M) | 8122 | 43 |
| Comparative Example 17 | AD1_hACTB.39_POE17 | A(M)^A(L)^A(M)^G(L)^C(M)^A(F)^U(F)^G(M)^C(M)^c^a^U(M)^C(F)^A(M)^C(F)(POE)C( M)^U(F)^C(M)^C(F)^C(M)^C(L)^U(M)^G(L)^U(M) | 8122 | 44 |

**[Table 3]**

| | Sequece name | Sequence (5'-3') | Molecular weight | SEQ ID NO: |
|---|---|---|---|---|
| Comparative Example 18 | AD1_hACTB.39_POE18 | A(M)^A(L)^A(M)^G(L)^C(M)^A(F)^U(F)^G(M)^C(M)^c^a^U(M)^C(F)^A(M)^C(F)^C(M)^U( F)(POE)C(M)^C(F)^C(M)^C(L)^U(M)^G(L)^U(M) | 8122 | 45 |
| Comparative Example 19 | AD1_hACTB.39_POE19 | A(M)^A(L)^A(M)^G(L)^C(M)^A(F)^U(F)^G(M)^C(M)^c^a^U(M)^C(F)^A(M)^C(F)^C(M)^U( F)^C(M)^C(F)(POE)C(M)^C(L)^U(M)^G(L)^U(M) | 8122 | 46 |
| Example 14 | AD1_hACTB.86_PM01 | A(M)^A(L)^A(M)^G(L)^C(M)^A(F)^U(F)^G(M)^C(M)^c^a(PM)U(M)^C(F)^A(M)(PM)C(F)^ C(M)^U(F)^C(M)^C(F)^C(M)^C(M)^U(M)^G(L)^U(M) | 8063.42 | 47 |
| Example 15 | AD1_hACTB.87_PM01 | A(M)^A(L)^A(M)^G(M)^C(M)^A(F)^U(F)^G(M)^C(M)^c^a(PM)U(M)^C(F)^A(M)(PM)C(F)^ C(M)^U(F)^C(M)^C(F)^C(M)^C(L)^U(M)^G(L)^U(M) | 8072.28 | 48 |
| Example 16 | AD1_hACTB.88_PM01 | A(M)^A(L)^A(M)^G(M)^C(M)^A(F)^U(F)^G(M)^C(M)^c^a(PM)U(M)^C(F)^A(M)(PM)C(F)^ C(M)^U(F)^C(M)^C(F)^C(M)^C(M)^U(M)^G(L)^U(M) | 8065.57 | 49 |
| Example 17 | AD1_hACTB.89_PM01 | A(L)^A(M)^A(M)^G(M)^C(M)^A(F)^U(F)^G(M)^C(M)^c^a(PM)U(M)^C(F)^A(M)(PM)C(F)^ C(M)^U(F)^C(M)^C(F)^C(M)^C(M)^U(M)^G(L)^U(M) | 8066.30 | 50 |
| Example 18 | AD1_hACTB.90_PM01 | A(M)^A(M)^A(M)^G(M)^C(M)^A(F)^U(F)^G(M)^C(M)pc^a(PM)U(M)^C(F)^A(M)(PM)C(F )^C(M)^U(F)^C(M)^C(F)^C(M)^C(M)^U(M)^G(M)^U(M) | 8067.22 | 51 |
| Example 19 | AD1_hACTB.39_POE20 | A(M)^A(L)^A(M)^G(L)^C(M)(POE)A(F)^U(F)^G(M)^C(M)^c^a(POE)U(M)^C(F)^A(M)(PO E)C(F)^C(M)^U(F)^C(M)^C(F)^C(M)^C(L)^U(M)^G(L)^U(M) | 8147 | 52 |
| Example 20 | AD1_hACTB.39_POE21 | A(M)^A(L)^A(M)^G(L)^C(M)^A(F)^U(F)^G(M)^C(M)^c^a(POE)U(M)^C(F)^A(M)(POE)C( F)^C(M)^U(F)^C(M)(POE)C(F)^C(M)^C(L)^U(M)^G(L)^U(M) | 8146 | 53 |
| Example 21 | AD1_hACTB.39_POE22 | A(M)^A(L)^A(M)^G(L)^C(M)^A(F)^U(F)^G(M)^C(M)^c^a(POE)U(M)^C(F)^A(M)(POE)C( F)^C(M)^U(F)^C(M)^C(F)^C(M)(POE)C(L)^U(M)^G(L)^U(M) | 8146 | 54 |
| Example 22 | AD1_hACTB.39_POE23 | A(M)^A(L)^A(M)^G(L)^C(M)^A(F)^U(F)^G(M)^C(M)^c^a(POE)U(M)pC(F)^A(M)(POE)C( F)^C(M)^U(F)^C(M)^C(F)^C(M)^C(L)^U(M)^G(L)^U(M) | 8118 | 55 |
| Example 23 | AD1_hACTB.39_POE24 | A(M)^A(L)^A(M)^G(L)^C(M)^A(F)^U(F)pG(M)^C(M)^c^a(POE)U(M)pC(F)^A(M)(POE)C( F)^C(M)^U(F)^C(M)^C(F)^C(M)^C(L)^U(M)^G(L)^U(M) | 8102 | 56 |
| Example 24 | AD1_hACTB.39_POE25 | A(M)^A(L)pA(M)^G(L)pC(M)^A(F)^U(F)^G(M)^C(M)^c^A(M)(POE)U(M)pC(F)^A(M)(PO E)C(F)^C(M)^U(F)^C(M)^C(F)^C(M)^C(L)pU(M)^G(L)pU(M) | 8054 | 57 |
| Example 25 | AD1_hACTB.39_POE26 | A(M)^A(L)^A(M)pG(L)^C(M)pA(F)^U(F)^G(M)^C(M)^c^A(M)(POE)U(M)pC(F)^A(M)(PO E)C(F)^C(M)^U(F)^C(M)^C(F)^C(M)pC(L)^U(M)pG(L)^U(M) | 8054 | 58 |
| Example 28 | AD1_hACTB.39_POE27 | A(M)^A(L)^A(M)^G(L)^C(M)^A(F)^U(F)^G(M)^C(M)^c^a(POE)U(M)pC(F)pA(M)(POE)C( F)pC(M)^U(F)^C(M)^C(F)^C(M)^C(L)^U(M)^G(L)^U(M) | 8086 | 59 |
| Example 27 | AD1_hACTB.39_POE28 | A(M)^A(L)^A(M)^G(L)^C(M)^A(F)^U(F)pG(M)pC(M)pcpa(POE)U(M)pC(F)pA(M)(POE) C(F)pC(M)^U(F)^C(M)^C(F)^C(M)^C(L)^U(M)^G(L)^U(M) | 8022 | 60 |
| Example 28 | AD1_hACTB.39_POE29 | A(M)^A(L)^A(M)^G(L)^C(M)^A(F)^U(F)pG(M)pC(M)pcpa(POE)U(M)pC(F)pA(M)(POE) C(F)pC(M)pU(F)pC(M)pC(F)pC(M)^C(L)^U(M)^G(L)^U(M) | 7958 | 61 |
| Comparative Example 18 | AD1_hACTB.39_POE30 | A(M)pA(L)pA(M)pG(L)pC(M)pA(F)pU(F)pG(M)pC(M)pcpa(POE)U(M)pC(F)pA(M)(PO E)C(F)pC(M)pU(F)pC(M)pC(F)pC(M)pC(L)pU(M)pG(L)pU(M) | 7798 | 62 |
| Example 29 | AD1_hACTB.39_PE03 | A(M)^A(L)^A(M)^G(L)^C(M)^A(F)^U(F)^G(M)^C(M)^c^a^U(M)^C(F)^A(M)(PE)C(F)^C(M) ^U(F)^C(M)^C(F)^C(M)^C(L)^U(M)^G(L)^U(M) | 8107 | 63 |
| Example 30 | AD1_hACTB.39_PP03 | A(M)^A(L)^A(M)^G(L)^C(M)^A(F)^U(F)^G(M)^C(M)^c^a^U(M)^C(F)^A(M)(PP)C(F)^C(M) ^U(F)^C(M)^C(F)^C(M)^C(L)^U(M)^G(L)^U(M) | 8122 | 64 |
| Example 31 | AD1_hACTB.39_PIP03 | A(M)^A(L)^A(M)^G(L)^C(M)^A(F)^U(F)^G(M)^C(M)^c^a^U(M)^C(F)^A(M)(PIP)C(F)^C(M )^U(F)^C(M)^C(F)^C(M)^C(L)^U(M)^G(L)^U(M) | 8120 | 65 |
| Example 32 | AD1_hACTB.39_PB03 | A(M)^A(L)^A(M)^G(L)^C(M)^A(F)^U(F)^G(M)^C(M)^c^a^U(M)^C(F)^A(M)(PB)C(F)^C(M) ^U(F)^C(M)^C(F)^C(M)^C(L)^U(M)^G(L)^U(M) | 8135 | 66 |
| Example 33 | AD1_hACTB.39_PMOP03 | A(M)^A(L)^A(M)^G(L)^C(M)^A(F)^U(F)^G(M)^C(M)^c^a^U(M)^C(F)^A(M)(PMOP)C(F)^ C(M)^U(F)^C(M)^C(F)^C(M)^C(L)^U(M)^G(L)^U(M) | 8150 | 67 |
| Example 34 | AD1_hACTB.39_PB02 | A(M)^A(L)^A(M)^G(L)^C(M)^A(F)^U(F)^G(M)^C(M)^c^a(PB)U(M)^C(F)^A(M)^C(F)^C(M) ^U(F)^C(M)^C(F)^C(M)^C(L)^U(M)^G(L)^U(M) | 8134 | 68 |
| Example 35 | AD1_hACTB.39_PMOP02 | A(M)^A(L)^A(M)^G(L)^C(M)^A(F)^U(F)^G(M)^C(M)^c^a(PMOP)U(M)^C(F)^A(M)^C(F)^ C(M)^U(F)^C(M)^C(F)^C(M)^C(L)^U(M)^G(L)^U(M) | 8151 | 69 |
| Example 36 | AD1_hACTB.39_POE32 | A(M)^A(L)^A(M)^G(L)^C(M)^A(F)^U(F)^G(M)^C(M)pc^a(POE)U(M)^C(F)^A(M)(POE)C( F)^C(M)^U(F)^C(M)^C(F)^C(M)^C(L)^U(M)^G(L)^U(M) | 8118 | 70 |
| Example 37 | AD1_hACTB.39_POE33 | A(M)^A(L)^A(M)^G(L)^C(M)^A(F)^U(F)^G(M)pC(M)^c^a(POE)U(M)^C(F)^A(M)(POE)C( F)^C(M)^U(F)^C(M)^C(F)^C(M)^C(L)^U(M)^G(L)^U(M) | 8119 | 71 |
| Example 38 | AD1_hACTB.39_POE34 | A(M)^A(L)^A(M)^G(L)pC(M)^A(F)^U(F)^G(M)^C(M)^c^a(POE)U(M)pC(F)^A(M)(POE)C( F)^C(M)^U(F)^C(M)^C(F)^C(M)^C(L)pU(M)^G(L)^U(M) | 8086 | 72 |
| Example 39 | AD1_hACTB.39_POE35 | A(M)^A(L)^A(M)^G(L)pC(M)^A(F)^U(F)^G(M)^C(M)^c^a(POE)U(M)pC(F)^A(M)(POE)C( F)^C(M)pU(F)^C(M)^C(F)^C(M)^C(L)pU(M)^G(L)^U(M) | 8070 | 73 |
| Example 40 | AD1_hACTB.39_POE36 | A(M)^A(L)^A(M)^G(L)pC(M)^A(F)^U(F)^G(M)^C(M)^c^a(POE)U(M)pC(F)^A(M)(POE)C( F)^C(M)^U(F)^C(M)pC(F)^C(M)^C(L)pU(M)^G(L)^U(M) | 8070 | 74 |

**[Table 4]**

| | Sequece name | Sequence (5'-3') | Molecular weight | SEQ ID NO: |
|---|---|---|---|---|
| Example 41 | AD1_hACTB.39_POE37 | A(M)^A(L)^A(M)^G(L)pC(M)^A(F)^U(F)^G(M)^C(M)^c^a(POE)U(M)pC(F)^A(M)(POE)C( F)^C(M)^U(F)^C(M)^C(F)^C(M)pC(L)pU(M)^G(L)^U(M) | 8070 | 75 |
| Example 42 | AD1_hACTB.39_POE38 | A(M)^A(L)^A(M)^G(L)pC(M)pA(F)^U(F)^G(M)^C(M)^c^a(POE)U(M)pC(F)^A(M)(POE)C (F)^C(M)^U(F)^C(M)^C(F)^C(M)^C(L)pU(M)^G(L)^U(M) | 8070 | 76 |
| Example 43 | AD1_hACTB.39_POE39 | A(M)^A(L)^A(M)^G(L)pC(M)pA(F)^U(F)^G(M)^C(M)^c^a(POE)U(M)pC(F)^A(M)(POE)C (F)^C(M)^U(F)^C(M)^C(F)^C(M)pC(L)pU(M)^G(L)^U(M) | 8054 | 77 |
| Example 44 | AD1_hACTB.39_POE40 | A(M)^A(L)^A(M)^G(L)pC(M)^A(F)^U(F)pG(M)^C(M)^c^a(POE)U(M)pC(F)^A(M)(POE)C (F)^C(M)pU(F)^C(M)^C(F)^C(M)^C(L)pU(M)^G(L)^U(M) | 8054 | 78 |
| Example 45 | AD1_hACTB.39_POE41 | A(M)^A(L)^A(M)^G(L)pC(M)^A(F)^U(F)pG(M)^C(M)^c^a(POE)U(M)pC(F)^A(M)(POE)C (F)^C(M)^U(F)^C(M)pC(F)^C(M)^C(L)pU(M)^G(L)^U(M) | 8054 | 79 |
| Example 46 | AD1_hACTB.39_POE42 | A(M)^A(L)^A(M)^G(L)pC(M)^A(F)^U(F)pG(M)^C(M)^c^a(POE)U(M)pC(F)^A(M)(POE)C (F)^C(M)^U(F)^C(M)^C(F)^C(M)pC(L)pU(M)^G(L)^U(M) | 8054 | 80 |
| Example 47 | AD1_hACTB.39_POE43 | A(M)^A(L)^A(M)^G(L)pC(M)pA(F)^U(F)pG(M)^C(M)^c^a(POE)U(M)pC(F)^A(M)(POE)C (F)^C(M)^U(F)^C(M)^C(F)^C(M)^C(L)pU(M)^G(L)^U(M) | 8054 | 81 |
| Example 48 | AD1_hACTB.39_POE44 | A(M)^A(L)^A(M)^G(L)pC(M)pA(F)^U(F)pG(M)^C(M)^c^a(POE)U(M)pC(F)^A(M)(POE)C (F)^C(M)^U(F)^C(M)^C(F)^C(M)pC(L)pU(M)^G(L)^U(M) | 8038 | 82 |
| Example 49 | AD1_hACTB.39_POP03 | A(M)^A(L)^A(M)^G(L)^C(M)^A(F)^U(F)^G(M)^C(M)^c^a^U(M)^C(F)^A(M)(POP)C(F)^C( M)^U(F)^C(M)^C(F)^C(M)^C(L)^U(M)^G(L)^U(M) | 8136.50 | 83 |
| Example 50 | AD1_hACTB.39_POB03 | A(M)^A(L)^A(M)^G(L)^C(M)^A(F)^U(F)^G(M)^C(M)^c^a^U(M)^C(F)^A(M)(POB)C(F)^C( M)^U(F)^C(M)^C(F)^C(M)^C(L)^U(M)^G(L)^U(M) | 8152 | 84 |
| Example 51 | AD1_hAC1B.39_POMOE03 | A(M)^A(L)^A(M)^G(L)^C(M)^A(F)^U(F)^G(M)^C(M)^c^a^U(M)^C(F)^A(M)(POMOE)C(F) ^C(M)^U(F)^C(M)^C(F)^C(M)^C(L)^U(M)^G(L)^U(M) | 8154 | 85 |
| Example 52 | AD1_hAC1B.39_POcP03 | A(M)^A(L)^A(M)^G(L)^C(M)^A(F)^U(F)^G(M)^C(M)^c^a^U(M)^C(F)^A(M)(POcP)C(F)^C (M)^U(F)^C(M)^C(F)^C(M)^C(L)^U(M)^G(L)^U(M) | 8137 | 86 |
| Example 53 | AD1_hAC1B.39_POcB03 | A(M)^A(L)^A(M)^G(L)^C(M)^A(F)^U(F)^G(M)^C(M)^c^a^U(M)^C(F)^A(M)(POcB)C(F)^C (M)^U(F)^C(M)^C(F)^C(M)^C(L)^U(M)^G(L)^U(M) | 8149 | 87 |
| Example 54 | AD1_hAC1B.39_POP02 | A(M)^A(L)^A(M)^G(L)^C(M)^A(F)^U(F)^G(M)^C(M)^c^a(POP)U(M)^C(F)^A(M)^C(F)^C( M)^U(F)^C(M)^C(F)^C(M)^C(L)^U(M)^G(L)^U(M) | 8139 | 88 |
| Example 55 | AD1_hAC1B.39_POB02 | A(M)^A(L)^A(M)^G(L)^C(M)^A(F)^U(F)^G(M)^C(M)^c^a(POB)U(M)^C(F)^A(M)^C(F)^C( M)^U(F)^C(M)^C(F)^C(M)^C(L)^U(M)^G(L)^U(M) | 8150 | 89 |
| Example 56 | AD1_hAC1B.39_POMOE02 | A(M)^A(L)^A(M)^G(L)^C(M)^A(F)^U(F)^G(M)^C(M)^c^a(POMOE)U(M)^C(F)^A(M)^C(F) ^C(M)^U(F)^C(M)^C(F)^C(M)^C(L)^U(M)^G(L)^U(M) | 8152.60 | 90 |
| Example 57 | AD1_hACTB.39_POcP02 | A(M)^A(L)^A(M)^G(L)^C(M)^A(F)^U(F)^G(M)^C(M)^c^a(POcP)U(M)^C(F)^A(M)^C(F)^C (M)^U(F)^C(M)^C(F)^C(M)^C(L)^U(M)^G(L)^U(M) | 8136 | 91 |
| Example 58 | AD1_hAC1B.39_POcB02 | A(M)^A(L)^A(M)^G(L)^C(M)^A(F)^U(F)^G(M)^C(M)^c^a(POcB)U(M)^C(F)^A(M)^C(F)^C (M)^U(F)^C(M)^C(F)^C(M)^C(L)^U(M)^G(L)^U(M) | 8148 | 92 |
| Example 59 | AD1_hAC1B39e_PMs04 | A(M)^A(E)^A(M)^G(E)^C(M)^A(F)^U(F)^G(M)^C(M)^c^a(PMs)U(M)^C(F)^A(M)(PMs)C( F)^C(M)^U(F)^C(M)^C(F)^C(M)^C(E)^U(M)^G(E)^U(M) | 8289.60 | 93 |
| Example 60 | AD1_hAC1B39e_PMs01 | A(M)^A(E)^A(M)^G(E)^C(M)^A(F)^U(F)^G(M)^C(M)^c^a(PMs)U(M)^C(F)^A(M)^C(F)^C( M)^U(F)^C(M)^C(F)^C(M)^C(E)^U(M)^G(E)^U(M) | 8228.55 | 94 |
| Example 61 | AD1_hACTB39e_PMs02 | A(M)^A(E)^A(M)^G(E)^C(M)^A(F)^U(F)^G(M)^C(M)^c^a^U(M)^C(F)^A(M)(PMs)C(F)^C( M)^U(F)^C(M)^C(F)^C(M)^C(E)^U(M)^G(E)^U(M) | 8228.55 | 95 |
| Comparative Example 21 | AD1_hACTB39e_PMs03 | A(M)^A(E)^A(M)^G(E)^C(M)^A(F)^U(F)^G(M)^C(M)^c^a^U(M)(PMs)C(F)^A(M)^C(F)^C( M)^U(F)^C(M)^C(F)^C(M)^C(E)^U(M)^G(E)^U(M) | 8228.55 | 96 |
| Example 62 | AD1_hAC1B.39e_PN01 | A(M)^A(E)^A(M)^G(E)^C(M)^A(F)^U(F)^G(M)^C(M)^c^a(PN)U(M)^C(F)^A(M)(PN)C(F)^ C(M)^U(F)^C(M)^C(F)^C(M)^C(E)^U(M)^G(E)^U(M) | 8325.68 | 97 |
| Example 63 | AD1_hACTB.86_PM01 | A(M)^A(L)^A(M)^G(L)^C(M)^A(F)^U(F)^G(M)^C(M)^c^a(PM)U(M)^C(F)^A(M)(PM)C(F)^ C(M)^U(F)^C(M)^C(F)^C(M)^C(M)^U(M)^G(L)^U(M) | 8063.42 | 98 |
| Example 64 | AD1_hACTB.87_PM01 | A(M)^A(L)^A(M)^G(M)^C(M)^A(F)^U(F)^G(M)^C(M)^c^a(PM)U(M)^C(F)^A(M)(PM)C(F)^ C(M)^U(F)^C(M)^C(F)^C(M)^C(L)^U(M)^G(L)^U(M) | 8072.28 | 29 |
| Example 65 | AD1_hACTB.88_PM01 | A(M)^A(L)^A(M)^G(M)^C(M)^A(F)^U(F)^G(M)^C(M)^c^a(PM)U(M)^C(F)^A(M)(PM)C(F)^ C(M)^U(F)^C(M)^C(F)^C(M)^C(M)^U(M)^G(L)^U(M) | 8065.57 | 100 |
| Example 66 | AD1_hAC1B89_PM01 | A(L)^A(M)^A(M)^G(M)^C(M)^A(F)^U(F)^G(M)^C(M)^c^a(PM)U(M)^C(F)^A(M)(PM)C(F)^ C(M)^U(F)^C(M)^C(F)^C(M)^C(M)^U(M)^G(L)^U(M) | 8066.30 | 101 |
| Example 67 | AD1_A1AT.39_PM01 | G(M)^T(L)^C(M)^C(L)^C(M)^U(F)^U(F)^C(M)^U(M)^c^i(PM)U(M)^C(F)^G(M)(PM)A(F)^U (M)^G(F)^G(M)^U(F)^C(M)^A(L)^G(M)^C(L)^A(M) | 8137.37 | 102 |
| Example 68 | AD1_A1AT.39_PM02 | G(M)^T(L)^C(M)^C(L)^C(M)^U(F)^U(F)^C(M)^U(M)^c^i(PM)U(M)^C(F)^G(M)^A(F)^U(M)^ G(F)^G(M)^U(F)^C(M)^A(L)^G(M)^C(L)^A(M) | 8153.46 | 103 |
| Example 69 | AD1_A1AT.39_PM03 | G(M)^T(L)^C(M)^C(L)^C(M)^U(F)^U(F)^C(M)^U(M)^c^i^U(M)^C(F)^G(M)(PM)A(F)^U(M)^ G(F)^G(M)^U(F)^C(M)^A(L)^G(M)^C(L)^A(M) | 8154.56 | 104 |

**[Table 5]**

| | Sequece name | Sequence (5'-3') | Molecular weight | SEQ ID NO: |
|---|---|---|---|---|
| Example 70 | AD1g_03_mGAPDH_A1222_3 9_PM02 | G(M)^A(L)^G(M)^G(L)^G(M)^C(F)^C(F)^C(M)^C(M)^c^a(PM)G(M)^G(F)^C(M)^C(F)^C( M)^C(F)^U(M)^C(F)^C(M)^T(L)^G(M)^T(L)^U(M) | 8172.26 | 105 |
| Example 71 | AD1g_03_mGAPDH_A1222_3 9_POE01 | G(M)^A(L)^G(M)^G(L)^G(M)^C(F)^C(F)^C(M)^C(M)^c^a(POE)G(M)^G(F)^C(M)(POE)C( F)^C(M)^C(F)^U(M)^C(F)^C(M)^T(L)^G(M)^T(L)^U(M) | 8211 | 106 |
| Example 72 | AD1g_03_mGAPDH_A1222_3 9_POE02 | G(M)^A(L)^G(M)^G(L)^G(M)^C(F)^C(F)^C(M)^C(M)^c^a(POE)G(M)^G(F)^C(M)^C(F)^C( M)^C(F)^U(M)^C(F)^C(M)^T(L)^G(M)^T(L)^U(M) | 8199 | 107 |
| Example 73 | AD1_hACTB.86_POE45 | A(M)^A(L)^A(M)^G(L)^C(M)^A(F)^U(F)^G(M)^C(M)^c^a(POE)U(M)^C(F)^A(M)(POE)C( F)^C(M)^U(F)^C(M)^C(F)^C(M)^C(M)^U(M)^G(L)^U(M) | 8120.9 | 108 |
| Example 74 | AD1_hAC1B.86_POE46 | A(M)^A(L)^A(M)^G(L)^C(M)^A(F)^U(F)^G(M)^C(M)^c^a(POE)U(M)pC(F)^A(M)(POE)C( F)^C(M)^U(F)^C(M)^C(F)^C(M)^C(M)^U(M)^G(L)^U(M) | 8105 | 109 |
| Example 75 | AD1_hAC1B.86_POE47 | A(M)^A(L)^A(M)^G(L)^C(M)^A(F)^U(F)^G(M)^C(M)^c^a(POE)U(M)pC(F)^A(M)(POE)C( F)^C(M)pU(F)^C(M)^C(F)^C(M)^C(M)^U(M)^G(L)^U(M) | 8088.9 | 110 |
| Example 76 | AD1_hACTB.86_POE48 | A(M)^A(L)^A(M)^G(L)^C(M)^A(F)^U(F)^G(M)^C(M)^c^a(POE)U(M)pC(F)^A(M)(POE)C( F)^C(M)^U(F)^C(M)pC(F)^C(M)^C(M)^U(M)^G(L)^U(M) | 8089 | 111 |
| Example 77 | AD1_hACTB.86_POE49 | A(M)^A(L)^A(M)^G(L)^C(M)^A(F)^U(F)^G(M)^C(M)^c^a(POE)U(M)pC(F)^A(M)(POE)C( F)^C(M)^U(F)^C(M)^C(F)^C(M)pC(M)^U(M)^G(L)^U(M) | 8090 | 112 |
| Example 78 | AD1_hACTB.86_POE50 | A(M)^A(L)^A(M)^G(L)^C(M)^A(F)^U(F)^G(M)^C(M)^c^a(POE)U(M)pC(F)^A(M)(POE)C( F)^C(M)pU(F)^C(M)pC(F)^C(M)^C(M)^U(M)^G(L)^U(M) | 8073 | 113 |
| Example 79 | AD1_hACTB.86_POE51 | A(M)^A(L)^A(M)^G(L)^C(M)^A(F)^U(F)^G(M)^C(M)^c^a(POE)U(M)pC(F)^A(M)(POE)C( F)^C(M)pU(F)^C(M)^C(F)^C(M)pC(M)^U(M)^G(L)^U(M) | 8073 | 114 |
| Example 80 | AD1_hACTB.86_POE52 | A(M)^A(L)^A(M)^G(L)^C(M)^A(F)^U(F)^G(M)^C(M)^c^a(POE)U(M)pC(F)^A(M)(POE)C( F)^C(M)^U(F)^C(M)pC(F)^C(M)pC(M)^U(M)^G(L)^U(M) | 8075 | 115 |
| Example 81 | AD1_hAC1B.86_POE53 | A(M)^A(L)^A(M)^G(L)^C(M)^A(F)^U(F)^G(M)^C(M)^c^a(POE)U(M)pC(F)^A(M)(POE)C( F)^C(M)pU(F)^C(M)pC(F)^C(M)pC(M)^U(M)^G(L)^U(M) | 8058 | 116 |
| Example 82 | AD1_hACTB.86_POMOE46 | A(M)^A(L)^A(M)^G(L)^C(M)^A(F)^U(F)^G(M)^C(M)^c^a(POMOE)U(M)pC(F)^A(M)(PO E)C(F)^C(M)^U(F)^C(M)^C(F)^C(M)^C(M)^U(M)^G(L)^U(M) | 8137 | 117 |
| Example 83 | AD1_hACTB.86p_POE46 | A(M)^A(L)^A(M)^G(L)^C(M)^A(F)^U(F)^G(M)^C(M)^c^(nebularine)(POE)U(M)pC(F)^A( M)(POE)C(F)^C(M)^U(F)^C(M)^C(F)^C(M)^C(M)^U(M)^G(L)^U(M) | 8092 | 118 |
| Comparative Example 22 | AD1_hACTB.39_PM08a | A(M)^A(L)^A(M)^G(L)^C(M)^A(F)^U(F)^G(M)^C(M)(PM)c^a^U(M)^C(F)^A(M)^C(F)^C(M )^U(F)^C(M)^C(F)^C(M)^C(L)^U(M)^G(L)^U(M) | 8093 | 119 |

"Molecular weight" in the Tables indicates a measured value by negative ion ESI mass spectrometry. In "Sequence" in the Tables, uppercase letters indicate RNA, lowercase letters indicate DNA, N(M) indicates 2'-O-methylation of D-ribofuranose, N(F) indicates 2'-deoxy-2'-fluorination of D-ribofuranose, N(L) indicates 2'-O,4'-C-methylenation of D-ribofuranose, and N(E) indicates 2'-O,4'-C-ethylenation of D-ribofuranose. Here, N indicates a nucleobase. As a bond between nucleoside units, "p" indicates a phosphoric residue (-P(=O)(OH)-), and "^" indicates a thiophosphoric residue (-P(=S)(OH)-). (PM) indicates a methylphosphonate residue, and (PSM) indicates a methylthiophosphonate residue. (PE) indicates an ethylphosphonate residue, (PP) indicates a propylphosphonate residue, (PIP) indicates an isopropylphosphonate residue, (PB) indicates a butylphosphonate residue, and (PMOP) indicates a methoxypropylphosphonate residue. (POE) indicates an ethyl phosphate residue, (POP) indicates a propyl phosphate residue, (POIP) indicates an isopropyl phosphate residue, (POB) indicates a butyl phosphate residue, (POMOE) indicates a methoxyethyl phosphate residue, (POcP) indicates a cyclopropyl phosphate residue, and (POcB) indicates a cyclobutyl phosphate residue. (PMs) indicates an N-mesylphosphoramide residue, and (PN) indicates a 1,3-dimethylimidazolidin-2-ylidene phosphate ester residue. Specific structures are shown below. Note that the 5' end and 3' end of the oligonucleotide are hydroxyl groups in which a hydrogen atom is bound to the oxygen atom in the figure.

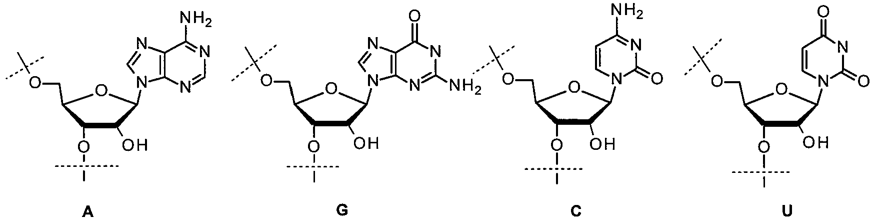

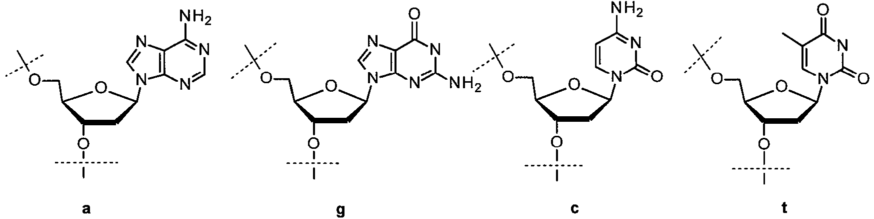

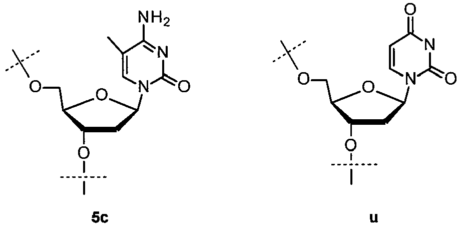

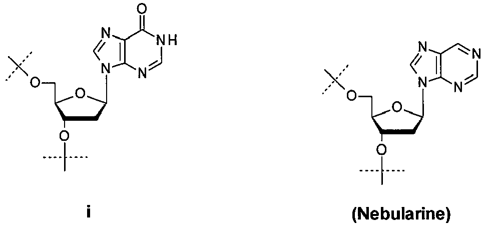

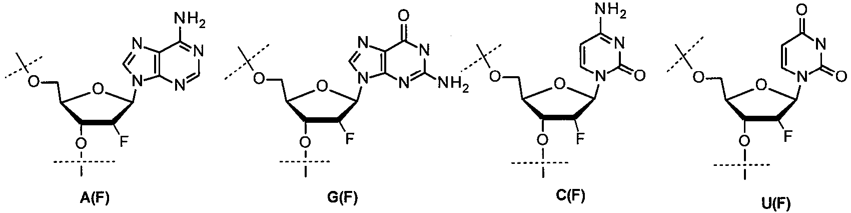

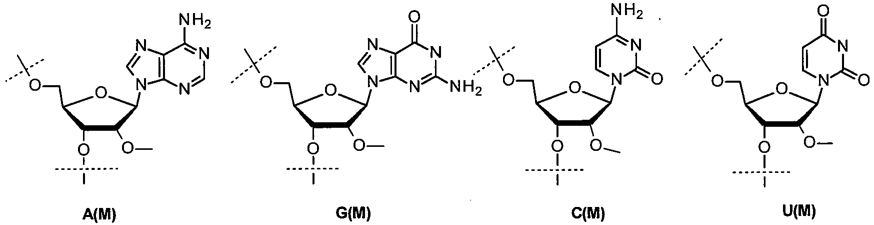

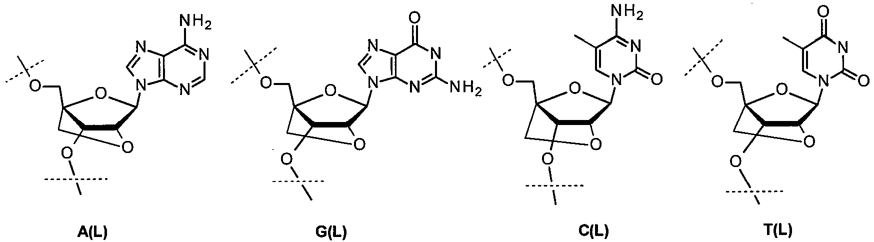

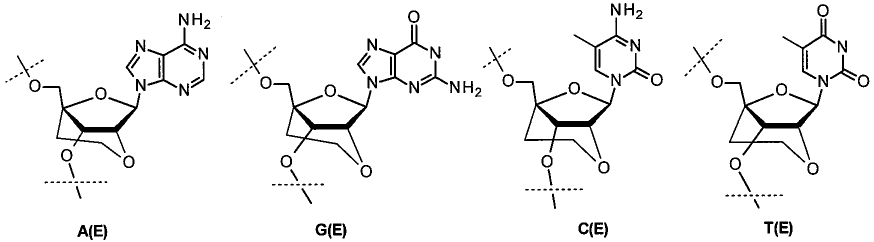

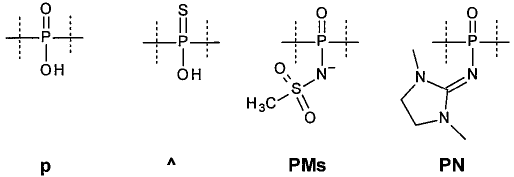

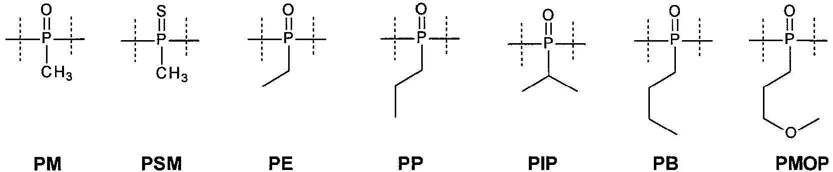

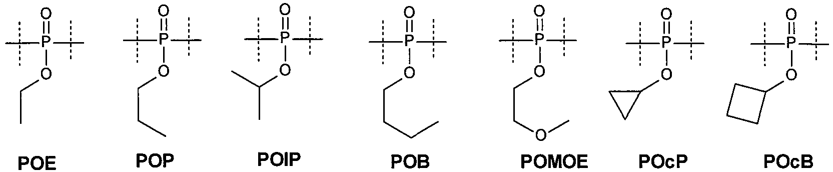

The compounds of Reference Examples 1 to 5 and Reference Example 32 were synthesized in the same manner as the compounds of Examples 1 to 83. The compounds of Reference Examples 1 to 5 and Reference Example 32 are described in Table 6.

**[Table 6]**

| | Sequece name | Sequence (5'-3') | Molecular weight | SEQ ID NO: |
|---|---|---|---|---|
| Reference Example 1 | AD1_hACTB.39 | A(M)^A(L)^A(M)^G(L)^C(M)^A(F)^U(F)^G(M)^C(M)^c^a^U(M)^C(F)^A(M)^C(F)^C(M)^U( F)^C(M)^C(F)^C(M)^C(L)^U(M)^G(L)^U(M) | 8111 | 120 |
| Reference Example 2 | AD1_hACTB.39e | A(M)^A(E)^A(M)^G(E)^C(M)^A(F)^U(F)^G(M)^C(M)^c^a^U(M)^C(F)^A(M)^C(F)^C(M)^U( F)^C(M)^C(F)^C(M)^C(E)^U(M)^G(E)^U(M) | 8167.52 | 121 |
| Reference Example 3 | AD1g_03_mGAPDH_A1222_3 9 | G(M)^A(L)^G(M)^G(L)^G(M)^C(F)^C(F)^C(M)^C(M)^c^a^G(M)^G(F)^C(M)^C(F)^C(M)^C( F)^U(M)^C(F)^C(M)^T(L)^G(M)^T(L)^U(M) | 8186.59 | 122 |
| Reference Example 4 | AD1_hACTB.39_POE31 | A(M)pA(L)pA(M)pG(L)pC(M)pA(F)pU(F)pG(M)pC(M)pcpapU(M)pC(F)pA(M)pC(F)pC( M)pU(F)pC(M)pC(F)pC(M)pC(L)pU(M)pG(L)pU(M) | 7743 | 123 |
| Reference Example 5 | AD1g _03_mGAPDH_A1222_3 9e | G(M)^A(E)^G(M)^G(E)^G(M)^C(F)^C(F)^C(M)^C(M)^c^a^G(M)^G(F)^C(M)^C(F)^C(M)^C (F)^U(M)^C(F)^C(M)^T(E)^G(M)^T(E)^U(M) | 8244.51 | 124 |
| Reference Example 32 | AD1_hACTB.86 | A(M)^A(L)^A(M)^G(L)^C(M)^A(F)^U(F)^G(M)^C(M)^c^a^U(M)^C(F)^A(M)^C(F)^C(M)^U( F)^C(M)^C(F)^C(M)^C(M)^U(M)^G(L)^U(M) | 8098 | 125 |

The descriptions of "Molecular Weight" and "Sequence" in Table 6 are the same as in Tables 2 to 5.

### Test Example 1

Evaluation of editing-inducing capability of target-editing oligonucleotide for RNA having hACTB gene sequence, in cell-free system

### (A) Synthesis of ACTB_RNA

Using pUC19_ACTB (a plasmid having the β-actin gene obtained in Test Example 3(A) described in the specification of WO2020/246560) as a template, template DNA was prepared by PCR (denaturation, 98°C, 10 seconds; annealing, 55°C, 15 seconds; elongation, 68°C, 20 seconds) with the T7_pUC19_FW primer (SEQ ID NO: 3), the M13_RV primer (SEQ ID NO: 4), and PrimeStar GXL DNA Polymerase (TaKaRa Bio Inc.), and purified using phenol/chloroform extraction and ethanol precipitation. RNA was synthesized by performing in vitro transcription (37°C, 3 hours) using AmpliScribe T7 Kit (Epicentre Biotechnologies Corp.). Then, DNase (the final concentration, 2 U) was added. The resultant mixture was treated at 37°C for 30 minutes. RNA was purified using phenol/chloroform extraction and ethanol precipitation. The resultant RNA was excised using 8 M Urea PAGE (8%), extracted by crushing and soaking, and purified using a 0.22 µm filter (Millipore) and gel filtration (BIO RAD).

### (B) Annealing reaction

ACTB_RNA (the final concentration: 0.3 µM) and a target-editing oligonucleotide (hereinafter also referred to as gRNA) (the final concentration, 0.9 µM) were heated in an annealing buffer (10 mM Tri-HCl (pH 7.6), 150 mM NaCl) at 80°C for 3 minutes, and then cooled to 25°C over a period of 15 minutes to yield an RNA complex.

### (C) In vitro editing reaction

Assuming that ACTB_RNA completely formed a complex with the target-editing oligonucleotide by the annealing reaction, the following complex concentration was calculated. The resultant RNA complex (the final concentration, 0.3 µM) was diluted with dH₂O to 50 nM. After the dilution, recombinant hADAR1p110 (synthesized and purified using a yeast expression system; see Macbeth, MR. and Bass, BL. Methods Enzymol. 424, 319-331 (2007), and Fukuda, M. et al. Sci. Rep. srep41478 (2017)) was added to the RNA complex (the final concentration, 5 nM) to a final concentration of 250 nM, and incubated at 37°C for 30 minutes.

### (D) Editing analysis

ACTB_RNA after the editing reaction was purified using phenol/chloroform extraction and ethanol precipitation. The resultant precipitate of each RNA complex was dissolved in 5 µL of TE Buffer. Reverse transcription reaction was performed using Primescript Reverse Transcriptase II (TaKaRa Bio Inc.) and M13_RV primer (SEQ ID NO: 4) (65°C, 5 minutes, quenching, 42°C, 15 minutes, 70°C, 15 minutes). Using the resultant cDNA as a template, cDNA was amplified by PCR (denaturation, 98°C, 10 seconds; annealing, 55°C, 15 seconds; elongation, 68°C, 20 seconds) using T7_pUC19_FW primer (SEQ ID NO: 3), M13_RV primer (SEQ ID NO: 4), and PrimeStar GXL DNA Polymerase (TaKaRa Bio Inc.). Amplification of cDNA was confirmed by DNA-500 kit (Shimadzu Corporation) and MultiNA (Shimadzu Corporation). Sequencing reaction (denaturation, 96°C, 10 seconds; annealing, 50°C, 5 seconds; elongation, 60°C, 30 seconds) was performed using T7proGGG primer (SEQ ID NO: 5) and SupreDye v3.1 Cycle Sequencing Kit (Edge BioSystems), and purification was performed using ethanol precipitation. The resultant precipitate was dissolved in 15 µL of Hi-Di formamide (Thermo Fisher Scientific Inc.), and base sequence analysis was performed by Applied Biosystem 3500 Genetic Analyzer (Thermo Fisher Scientific Inc.). The peak heights of A and G at the target editing site in the sequencing chromatogram obtained by the analysis were analyzed, and the editing rate (%) was calculated from the peak height rate (G/(G+A)).

**[Table 7]**

| Primer | Sequence | SEQ ID NO: |
|---|---|---|
| T7_pUC19_FW | CTAATACGACTCACTATAGGGCCTCTTCGCTATTACGCCAG | 3 |
| M13_RV | GGAAACAGCTATGACCATGATTAC | 4 |
| T7proGGG | CTAATACGACTCACTATAGGG | 5 |

### (E) Results of editing analysis

The results of the editing rate by ADAR1p110 are shown in Fig. 1A. Regarding the RNA editing efficiency by ADAR1p110, Example compound 2 (AD1_hACTB.39_PM02) in which the bond at the internucleoside +1 site was a methylphosphonate bond and Example compound 3 (AD1_hACTB.39_PM03) in which the bond at the internucleoside +4 site was a methylphosphonate bond exhibited a high editing rate, compared to Reference Example compound 1 (AD1_hACTB.39) in which all the bonds at the internucleosides in the target-editing oligonucleotide (gRNA) were phosphorothioate bonds. Furthermore, Example compound 1 (AD1_hACTB.39_PM01) in which the bonds at the internucleoside +1 and +4 sites were methylphosphonate bonds in the gRNA exhibited an even higher editing rate. On the other hand, Comparative Example compounds 1 to 3 (AD1_hACTB.39_PM04 to 06) in which the bond at the internucleoside +2, -9, or -7 site was a methylphosphonate bond exhibited a slight increase or decrease in the editing rate.

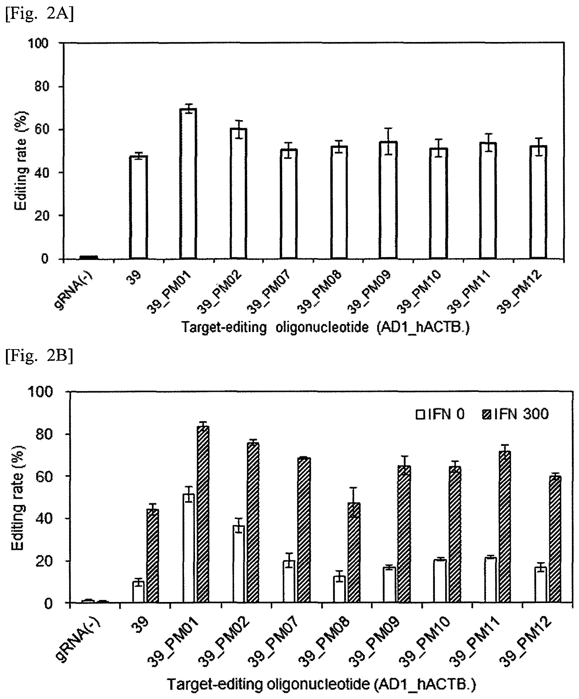

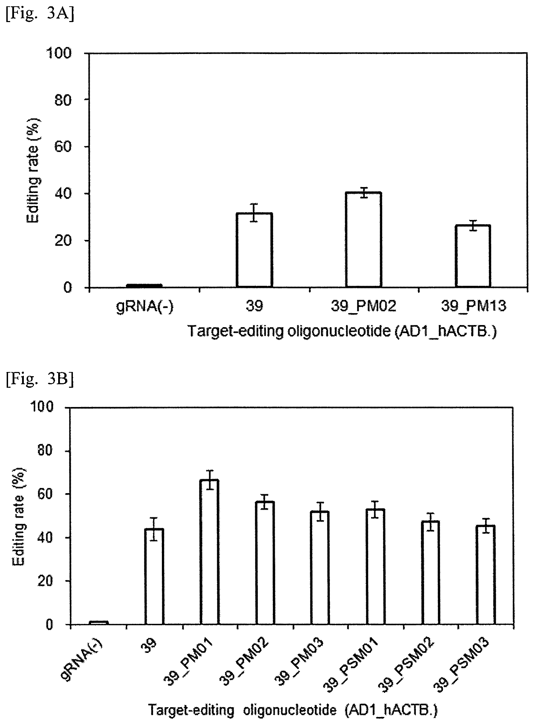

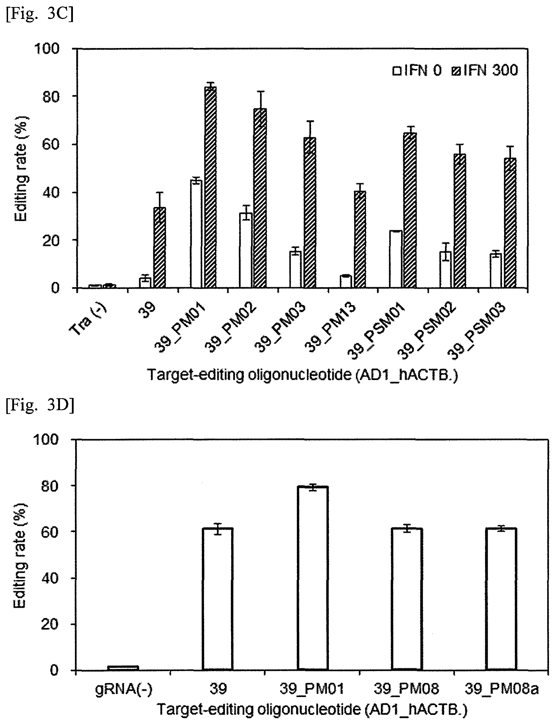

As shown in Fig. 2A, in Comparative Example compounds 4 to 9 (AD1_hACTB.39_PM07 to 12) in which the bond at the internucleoside -5, -1, +6, +8, +10, or +12 site was a methylphosphonate bond, the increase in the editing rate was slight. Additionally, as shown in Fig. 3A, Comparative Example compound 10 (AD1_hACTB.39_PM13) in which the bond at the internucleoside -2 site was a methylphosphonate bond exhibited a decrease in the editing rate, compared to Reference Example compound 1 (AD1_hACTB.39). These results have revealed that a high editing rate is exhibited when a methylphosphonate bond is used in the gRNA, particularly at at least one site selected from the group consisting of the internucleoside +1 and +4 sites. Additionally, as shown in Fig. 3D, Comparative Example compound 22 (AD1_hACTB.39_PM08a) in which the bond at the internucleoside -1 site was a methylphosphonate bond exhibited no increase in the editing rate.

The results of the editing rate by ADAR1p110 are shown in Fig. 3B. Regarding the RNA editing efficiency by ADAR1p110, Example compound 9 (AD1_hACTB.39_PSM02) in which the bond at the internucleoside +1 site was a methylthiophosphonate bond and Example compound 10 (AD1_hACTB.39_PSM03) in which the bond at the internucleoside +4 site was a methylthiophosphonate bond exhibited a high editing rate, compared to Reference Example compound 1 (AD1_hACTB.39) in which all the bonds at the internucleosides in gRNA were phosphorothioate bonds. Furthermore, Example compound 8 (AD1_hACTB.39_PSM01) in which the bonds at the internucleoside +1 and +4 sites were methylthiophosphonate bonds in the gRNA exhibited an even higher editing rate. These results have revealed that a high editing rate is exhibited when a methylthiophosphonate bond is used in the gRNA, particularly at the internucleoside +1 and/or +4 site(s).

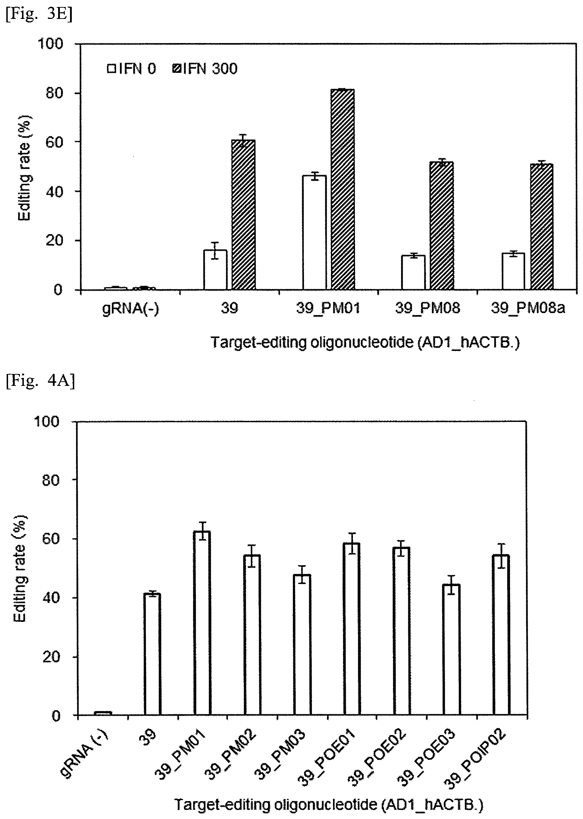

The results of the editing rate by ADAR1p110 are shown in Fig. 4A. Regarding the RNA editing efficiency by ADAR1p110, Example compound 5 (AD1_hACTB.39_POE02) in which the bond at the internucleoside +1 site was an ethyl phosphate bond, Example compound 7 (AD1_hACTB.39_POIP02) in which the bond at the internucleoside +1 site was an isopropyl phosphate bond, and Example compound 6 (AD1_hACTB.39_POE03) in which the bond at the internucleoside +4 site was an ethyl phosphate bond exhibited a high editing rate, compared to Reference Example compound 1 (AD1_hACTB.39) in which all bonds between nucleosides in the gRNA were phosphorothioate bonds. Furthermore, Example compound 4 (AD1_hACTB.39_POE01) in which the bonds at the internucleoside +1 and +4 sites in the gRNA were ethyl phosphate bonds exhibited an even higher editing rate. These results have revealed that a high editing rate is exhibited when an ethyl phosphate bond is used in the gRNA at the internucleoside +1 and/or +4 site(s), or particularly when an isopropyl phosphate bond is used at the internucleoside +1 site.

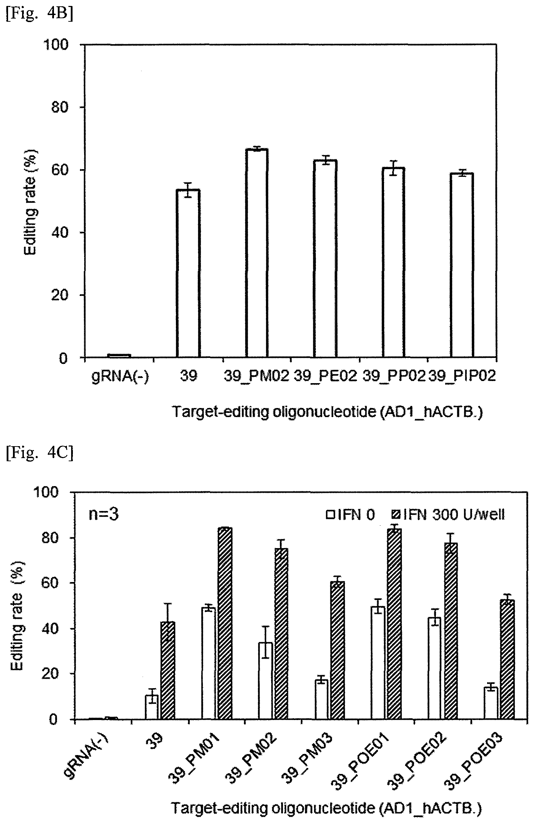

The results of the editing rate by ADAR1p110 are shown in Fig. 4B. Regarding the RNA editing efficiency by ADAR1p110, Example compound 11 (AD1_hACTB.39_PE02) in which the bond at the internucleoside +1 site was an ethylphosphonate bond, Example compound 12 (AD1_hACTB.39_PP02) in which the bond was a propylphosphonate bond, and Example compound 13 (AD1_hACTB.39_PIP02) in which the bond was an isopropylphosphonate bond exhibited a high editing rate, compared to Reference Example compound 1 (AD1_hACTB.39) in which all the bonds at the internucleosides in the gRNA were phosphorothioate bonds. These results have revealed that a high editing rate is exhibited when an ethylphosphonate bond, a propylphosphonate bond, or an isopropylphosphonate bond is used in the gRNA, particularly at the internucleoside +1 site.

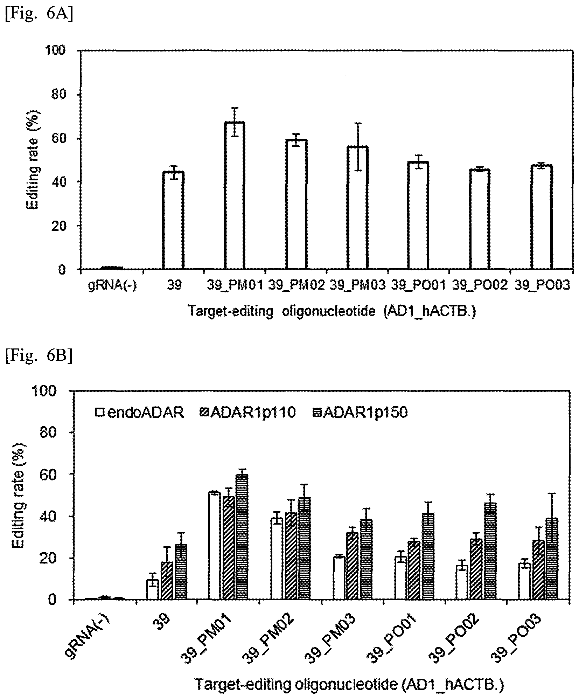

The results of the editing rate by ADAR1p110 are shown in Fig. 6A. Regarding the RNA editing efficiency by ADAR1p110, Comparative Example compound 12 (AD1_hACTB.39_PO02) in which the bond at the internucleoside +1 site was a phosphodiester bond and Comparative Example compound 13 (AD1_hACTB.39_PO03) in which the bond at the internucleoside +4 site was a phosphodiester bond exhibited a high editing rate, compared to Reference Example compound 1 (AD1_hACTB.39) in which all the bonds at the internucleosides in the gRNA were phosphorothioate bonds. Furthermore, Comparative Example compound 11 (AD1_hACTB.39_PO01) in which the bonds at the internucleoside +1 and +4 sites in the gRNA were phosphodiester bonds exhibited an even higher editing rate. These results have revealed that a high editing rate is exhibited when a phosphodiester bond is used in the gRNA, particularly at the internucleoside +1 and/or +4 site(s).

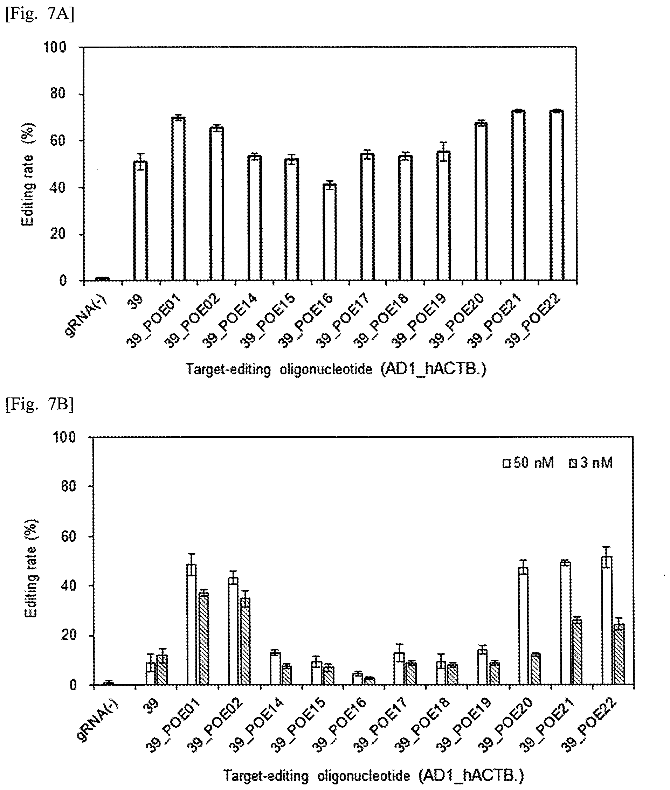

The results of the editing rate by ADAR1p110 are shown in Fig. 7A. Regarding the RNA editing efficiency by ADAR1p110, Comparative Example compound 14 (AD1_hACTB.39_POE14) in which the bond at the internucleoside -4 site was an ethyl phosphate bond, Comparative Example compound 15 (AD1_hACTB.39_POE15) in which the bond at the internucleoside -3 site was an ethyl phosphate bond, Comparative Example compound 17 (AD1_hACTB.39_POE17) in which the bond at the internucleoside +5 site was an ethyl phosphate bond, Comparative Example compound 18 (AD1_hACTB.39_POE18) in which the bond at the internucleoside +7 site was an ethyl phosphate bond, and Comparative Example compound 19 (AD1_hACTB.39_POE19) in which the bond at the internucleoside +9 site was an ethyl phosphate bond showed comparable editing rates, compared to Reference Example compound 1 (AD1_hACTB.39) in which all the bonds at the internucleosides in the gRNA were phosphorothioate bonds. On the other hand, Comparative Example compound 16 (AD1_hACTB.39_POE16) in which the bond at the internucleoside +3 site in the gRNA was an ethyl phosphate bond exhibited a slight decrease in the editing rate. Additionally, Example compound 19 (AD1_hACTB.39_POE20) in which the bonds at the three sites, internucleosides -5, +1, and +4, in the gRNA were ethyl phosphate bonds, Example compound 20 (AD1_hACTB.39_POE21) in which the bonds at the three sites, internucleosides +1, +4, and +8, were ethyl phosphate bonds, and Example compound 21 (AD1_hACTB.39_POE22) in which the bonds at the three sites, internucleosides +1, +4, and +10, were ethyl phosphate bonds exhibited a high editing rate. These results have revealed that a high editing rate is exhibited when an ethyl phosphate bond is used in the gRNA, at the internucleoside +1 and/or +4 site(s). The results have also revealed that, in particular, the compounds having an ethyl phosphate bond at any of the internucleoside -5, +8, or +10 sites in addition to the internucleoside +1 and +4 sites also exhibit a high editing rate.

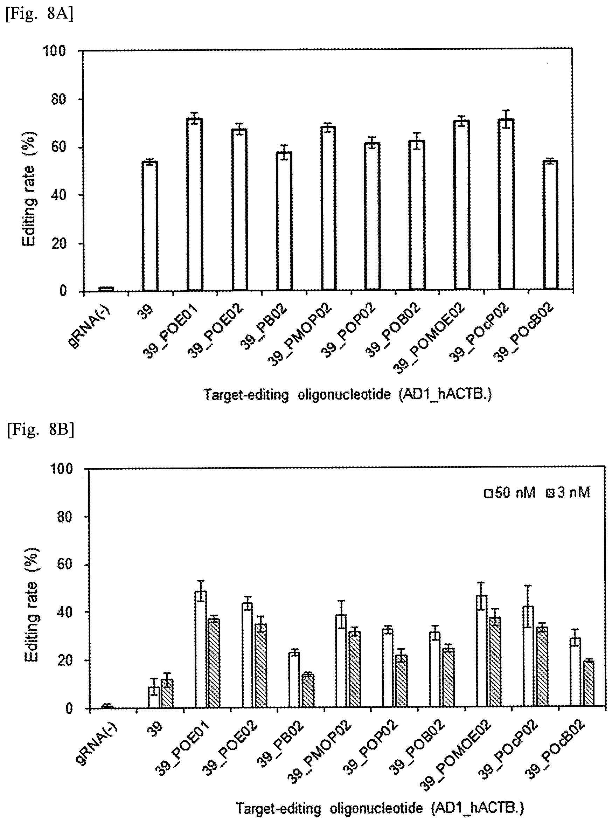

The results of the editing rate by ADAR1p110 are shown in Fig. 8A. Regarding the RNA editing efficiency by ADAR1p110, Example compound 34 (AD1_hACTB.39_PB02) in which the bond at the internucleoside +1 site was a butylphosphonate bond, Example compound 35 (AD1_hACTB.39_PMOP02) in which the bond at the internucleoside +1 site was a methoxypropylphosphonate bond, Example compound 54 (AD1_hACTB.39_POP02) in which the bond at the internucleoside +1 site was a propyl phosphate bond, Example compound 55 (AD1_hACTB.39_POB02) in which the bond at the internucleoside +1 site was a butyl phosphate bond, Example compound 56 (AD1_hACTB.39_POMOE02) in which the bond at the internucleoside +1 site was a methoxyethyl phosphate bond, Example compound 57 (AD1_hACTB.39_POcP02) in which the bond at the internucleoside +1 site was a cyclopropyl phosphate bond, and Example compound 58 (AD1_hACTB.39_POcB02) in which the bond at the internucleoside +1 site was a cyclobutyl phosphate bond exhibited a high editing rate, compared to Reference Example compound 1 (AD1_hACTB.39) in which all the bonds at the internucleosides in the gRNA were phosphorothioate bonds. These results have revealed that a high editing rate is exhibited when a butylphosphonate bond, a methoxypropylphosphonate bond, a propyl phosphate bond, a butyl phosphate bond, a methoxyethyl phosphate bond, a cyclopropyl phosphate bond, or a cyclobutyl phosphate bond is used in the gRNA, particularly at the internucleoside +1 site.

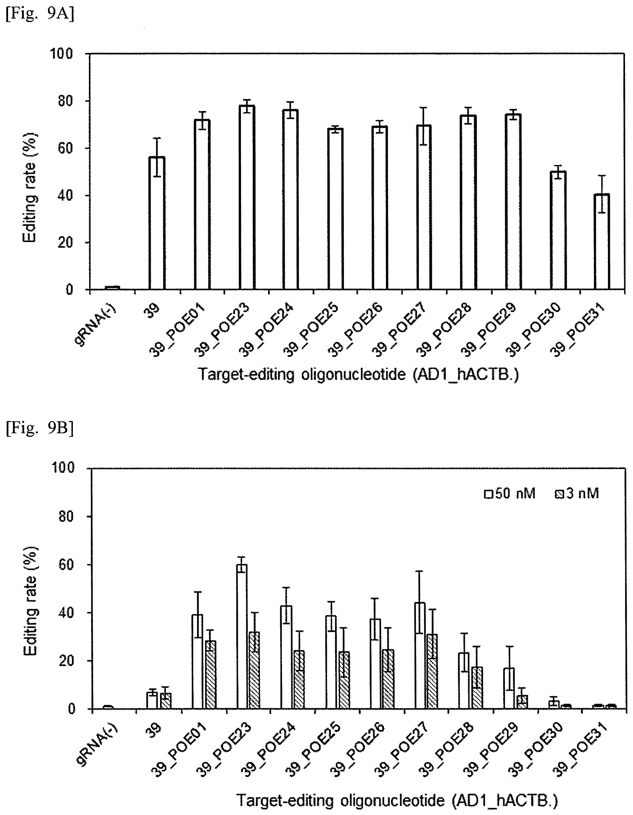

The results of the editing rate by ADAR1p110 are shown in Fig. 9A. Regarding the RNA editing efficiency by ADAR1p110, Example compound 22 (AD1_hACTB.39_POE23) having ethyl phosphate bonds at the internucleoside +1 and +4 sites and further having a phosphodiester bond at the internucleoside +2 site, Example compound 23 (AD1_hACTB.39_POE24) having ethyl phosphate bonds at the internucleoside +1 and +4 sites and further having phosphodiester bonds at the internucleoside -3 and +2 sites, Example compound 24 (AD1_hACTB.39_POE25) having ethyl phosphate bonds at the internucleoside +1 and +4 sites and further having phosphodiester bonds at the internucleoside -8, -6, +2, +11, and +13 sites, Example compound 25 (AD1_hACTB.39_POE26) having ethyl phosphate bonds at the internucleoside +1 and +4 sites and further having phosphodiester bonds at the internucleoside -7, -5, +2, +10, and +12 sites, Example compound 26 (AD1_hACTB.39_POE27) having ethyl phosphate bonds at the internucleoside +1 and +4 sites and further having phosphodiester bonds at the internucleoside +2, +3, and +5 sites, Example compound 27 (AD1_hACTB.39_POE28) having ethyl phosphate bonds at the internucleoside +1 and +4 sites and further having phosphodiester bonds at the internucleoside -3, -2, -1, 0, +2, +3, and +5 sites, and Example compound 28 (AD1_hACTB.39_POE29) having ethyl phosphate bonds at the internucleoside +1 and +4 sites and further having phosphodiester bonds at the internucleoside -3 to -1, 0, +2, +3, and +5 to +9 sites exhibited a high editing rate, compared to Reference Example compound 1 (AD1_hACTB.39) in which all the bonds at the internucleosides in the gRNA were phosphorothioate bonds.

Additionally, regarding the RNA editing efficiency by ADAR1p110, Comparative Example compound 20 (AD1_hACTB.39_POE30) having ethyl phosphate bonds at the internucleoside +1 and +4 sites and having phosphodiester bonds at the other sites exhibited a slightly higher editing rate, compared to Reference Example compound 4 (AD1_hACTB.39_POE31) in which all the bonds at the internucleosides in the gRNA were phosphodiester bonds. These results have revealed that a high editing rate is exhibited particularly when an ethyl phosphate bond is used in the gRNA at the internucleoside +1 and +4 sites.

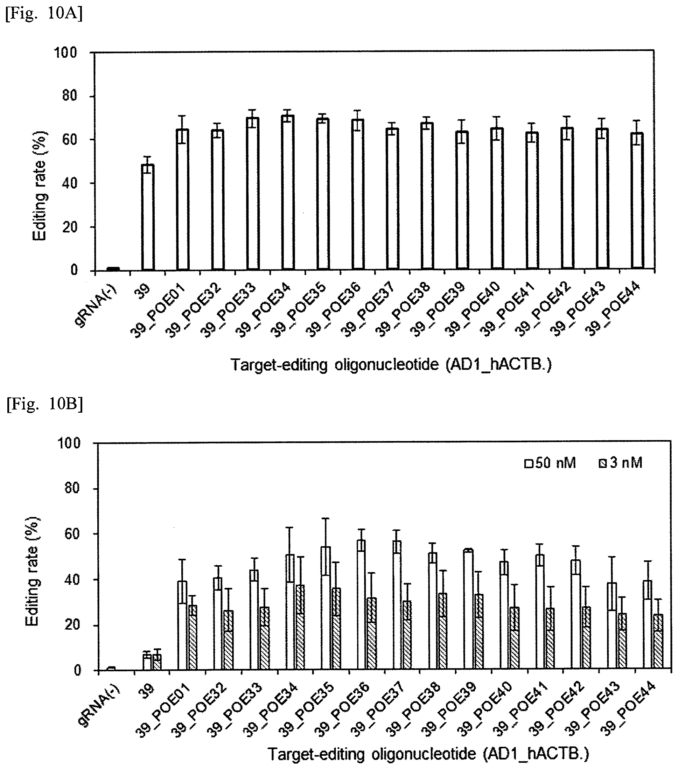

The results of the editing rate by ADAR1p110 are shown in Fig. 10A. Regarding the RNA editing efficiency by ADAR1p110, Example compound 36 (AD1_hACTB.39_POE32) having ethyl phosphate bonds at the internucleoside +1 and +4 sites and further having a phosphodiester bond at the internucleoside -1 site, Example compound 37 (AD1_hACTB.39_POE33) having ethyl phosphate bonds at the internucleoside +1 and +4 sites and further having a phosphodiester bond at the internucleoside -2 site, Example compound 38 (AD1_hACTB.39_POE34) having ethyl phosphate bonds at the internucleoside +1 and +4 sites and further having phosphodiester bonds at the internucleoside -6, +2, and +11 sites, Example compound 39 (AD1_hACTB.39_POE35) having ethyl phosphate bonds at the internucleoside +1 and +4 sites and further having phosphodiester bonds at the internucleoside -6, +2, +6, and +11 sites, Example compound 40 (AD1_hACTB.39_POE36) having ethyl phosphate bonds at the internucleoside +1 and +4 sites and further having phosphodiester bonds at the internucleoside -6, +2, +8, and +11 sites, Example compound 41 (AD1_hACTB.39_POE37) having ethyl phosphate bonds at the internucleoside +1 and +4 sites and further having phosphodiester bonds at the internucleoside -6, +2, +10, and +11 sites, Example compound 42 (AD1_hACTB.39_POE38) having ethyl phosphate bonds at the internucleoside +1 and +4 sites and further having phosphodiester bonds at the internucleoside -6, -5, +2, and +11 sites, Example compound 43 (AD1_hACTB.39_POE39) having ethyl phosphate bonds at the internucleoside +1 and +4 sites and further having phosphodiester bonds at the internucleoside -6, -5, +2, +10, and +11 sites, Example compound 44 (AD1_hACTB.39_POE40) having ethyl phosphate bonds at the internucleoside +1 and +4 sites and further having phosphodiester bonds at the internucleoside -6, -3, +2, +6, and +11 sites, Example compound 45 (AD1_hACTB.39_POE41) having ethyl phosphate bonds at the internucleoside +1 and +4 sites and further having phosphodiester bonds at the internucleoside -6, -3, +2, +8, and +11 sites, Example compound 46 (AD1_hACTB.39_POE42) having ethyl phosphate bonds at the internucleoside +1 and +4 sites and further having phosphodiester bonds at the internucleoside -6, -3, +2, +10, and +11 sites, Example compound 47 (AD1_hACTB.39_POE43) having ethyl phosphate bonds at the internucleoside +1 and +4 sites and further having phosphodiester bonds at the internucleoside -6, -5, -3, +2, and +11 sites, and Example compound 48 (AD1_hACTB.39_POE44) having ethyl phosphate bonds at the internucleoside +1 and +4 sites and further having phosphodiester bonds at the internucleoside -6, -5, -3, +2, +10, and +11 sites exhibited a high editing rate, compared to Reference Example compound 1 (AD1_hACTB.39) in which all the bonds at the internucleosides in the gRNA were phosphorothioate bonds. These results have revealed that a high editing rate is exhibited particularly when an ethyl phosphate bond is used in the gRNA at the internucleoside +1 and +4 sites.

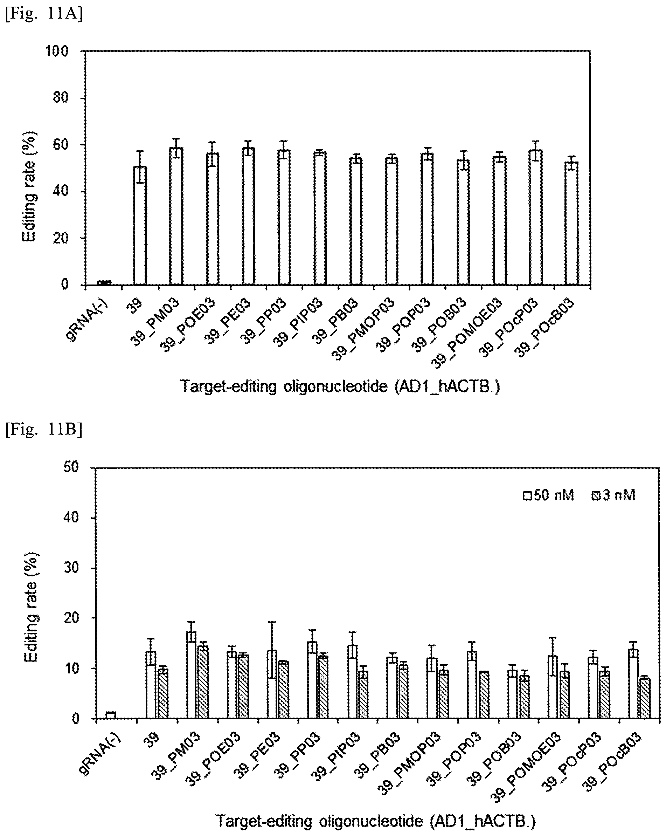

The results of the editing rate by ADAR1p110 are shown in Fig. 11A. Regarding the RNA editing efficiency by ADAR1p110, Example compound 29 (AD1_hACTB.39_PE03) in which the bond at the internucleoside +4 site was an ethylphosphonate bond, Example compound 30 (AD1_hACTB.39_PP03) in which the bond at the internucleoside +4 site was a propylphosphonate bond, Example compound 31 (AD1_hACTB.39_PIP03) in which the bond at the internucleoside +4 site was an isopropylphosphonate bond, Example compound 32 (AD1_hACTB.39_PB03) in which the bond at the internucleoside +4 site was a butylphosphonate bond, Example compound 33 (AD1_hACTB.39_PMOP03) in which the bond at the internucleoside +4 site was a methoxypropylphosphonate bond, Example compound 49 (AD1_hACTB.39_POP03) in which the bond at the internucleoside +4 site was a propyl phosphate bond, Example compound 50 (AD1_hACTB.39_POB03) in which the bond at the internucleoside +4 site was a butyl phosphate bond, Example compound 51 (AD1_hACTB.39_POMOE03) in which the bond at the internucleoside +4 site was a methoxyethyl phosphate bond, Example compound 52 (AD1_hACTB.39_POcP03) in which the bond at the internucleoside +4 site was a cyclopropyl phosphate bond, and Example compound 53 (AD1_hACTB.39_POcB03) in which the bond at the internucleoside +4 site was a cyclobutyl phosphate bond exhibited a high editing rate, compared to Reference Example compound 1 (AD1_hACTB.39) in which all the bonds at the internucleosides in the gRNA were phosphorothioate bonds. These results have revealed that a high editing rate is exhibited when an ethylphosphonate bond, a propylphosphonate bond, an isopropylphosphonate bond, a butylphosphonate bond, a methoxypropylphosphonate bond, a propyl phosphate bond, a butyl phosphate bond, a methoxyethyl phosphate bond, a cyclopropyl phosphate bond, or a cyclobutyl phosphate bond is used in the gRNA, particularly at the internucleoside +4 site.

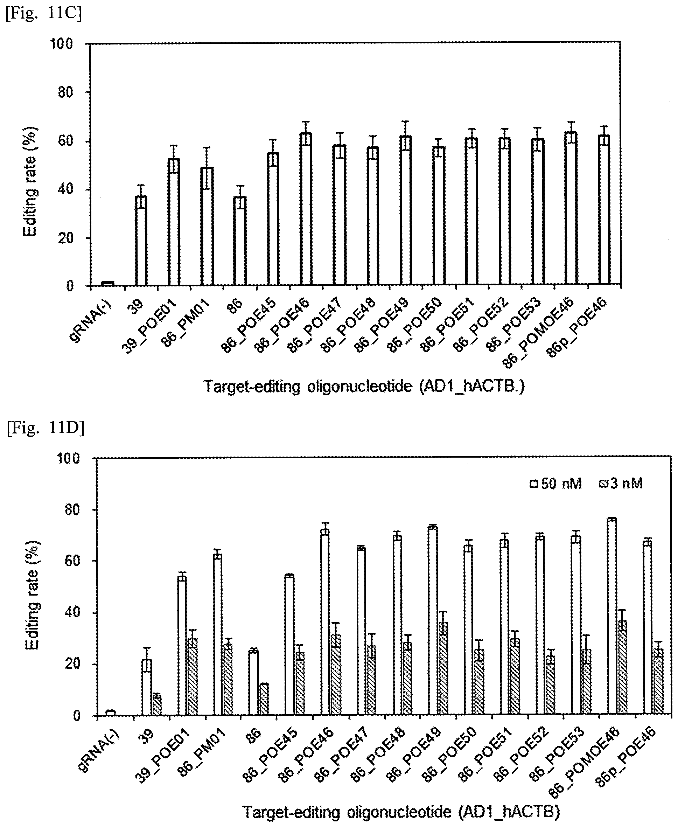

The results of the editing rate by ADAR1p110 are shown in Fig. 11C. Regarding the RNA editing efficiency by ADAR1p110, Example compound 73 (AD1_hACTB.86_POE45) in which the internucleoside +1 and +4 sites were ethyl phosphate bonds, Example compound 74 (AD1_hACTB.86_POE46) having ethyl phosphate bonds at the internucleoside +1 and +4 sites and further having a phosphodiester bond at the internucleoside +2 site, Example compound 75 (AD1_hACTB.86_POE47) having ethyl phosphate bonds at the internucleoside +1 and +4 sites and further having phosphodiester bonds at the internucleoside +2 and +6 sites, Example compound 76 (AD1_hACTB.86_POE48) having ethyl phosphate bonds at the internucleoside +1 and +4 sites and further having phosphodiester bonds at the internucleoside +2 and +8 sites, Example compound 77 (AD1_hACTB.86_POE49) having ethyl phosphate bonds at the internucleoside +1 and +4 sites and further having phosphodiester bonds at the internucleoside +2 and +10 sites, Example compound 78 (AD1_hACTB.86_POE50) having ethyl phosphate bonds at the internucleoside +1 and +4 sites and further having phosphodiester bonds at the internucleoside +2, +6, and +8 sites, Example compound 79 (AD1_hACTB.86_POE51) having ethyl phosphate bonds at the internucleoside +1 and +4 sites and further having phosphodiester bonds at the internucleoside +2, +6, and +10 sites, Example compound 80 (AD1_hACTB.86_POE52) having ethyl phosphate bonds at the internucleoside +1 and +4 sites and further having phosphodiester bonds at the internucleoside +2, +8, and +10 sites, Example compound 81 (AD1_hACTB.86_POE53) having ethyl phosphate bonds at the internucleoside +1 and +4 sites and further having phosphodiester bonds at the internucleoside +2, +6, +8, and +10 sites, Example compound 82 (AD1_hACTB.86_POMOE46) having a methoxyethyl phosphate bond at the internucleoside +1 site, an ethyl phosphate bond at the internucleoside +4 site, and further having a phosphodiester bond at the internucleoside +2 site, and Example compound 83 (AD1_hACTB.86p_POE46) having nebularine as the nucleoside at +1, having ethyl phosphate bonds at the internucleoside +1 and +4 sites, and further having a phosphodiester bond at the internucleoside +2 site exhibited a high editing rate, compared to Reference Example compound 32 (AD1_hACTB.86) in which all the bonds at the internucleosides in the gRNA were phosphorothioate bonds. These results have revealed that a high editing rate is exhibited particularly when ethyl phosphate bonds are used in the gRNA at the internucleoside +1 and +4 sites, or when a methoxyethyl phosphate bond is used at the internucleoside +1 site, and when an ethyl phosphate bond is used at the internucleoside +4 site. The results have further revealed, in addition to the above, that a high editing rate is exhibited when a phosphodiester bond is used at at least one site selected from the group consisting of the internucleoside +2, +6, +8, and +10 sites.

### Test Example 2

### Evaluation of editing-inducing capability of target-editing oligonucleotide for RNA having hACTB gene sequence, in cultured cell

### (A) Cell culture

In a case where interferon α was added, HEK293 cells (JCRB Cell Bank) were seeded in a 24-well Plate at 5.0×10⁴ cells/well. In the case of evaluation under conditions where ADAR1p150 was overexpressed, human interferon α (PBL Assay Science Inc.) was added at 300 U/well. After 48 hours, the medium was exchanged, and the target-editing oligonucleotide was transfected to a final concentration of 50 nM, using Lipofectamine^{™} RNAiMAX Transfection Reagent (Thermo Fisher Scientific Inc.). After 48 hours, the cells were collected.

In a case where interferon α was not added, HEK293 cells (JCRB Cell Bank) were seeded in a 24-well Plate at 5.0×10⁴ cells/well. After 48 hours, the target-editing oligonucleotide was transfected (the final concentration, 3 nM, 10 nM, 50 nM, or the like), using Lipofectamine^{™} RNAiMAX Transfection Reagent (Thermo Fisher Scientific Inc.). After 48 hours, the cells were collected.

### (B) Editing analysis

Total RNA was extracted from the cells cultured in the 24-well Plate, using RNeasy Plus Mini Kit (QIAGEN). Reverse transcription reaction was performed using ReverTra Ace^{(R)} qPCR RT Master Mix with gDNA Remover (TOYOBO) and 0.5 µg of Total RNA to amplify cDNA. The ACTB fragment was amplified by PCR (denaturation, 98°C, 10 seconds; annealing, 55°C, 15 seconds; elongation, 68°C, 30 seconds), using PrimeStar GXL DNA polymerase (TaKaRa), the endoACTB_F02 primer (SEQ ID NO: 6), and the endoACTB_R02 primer (SEQ ID NO: 7). The amplification was confirmed by a DNA-1000 kit (Shimadzu Corporation) and MultiNA (Shimadzu Corporation). Sequencing reaction (denaturation, 96°C, 10 seconds; annealing, 50°C, 5 seconds; elongation, 60°C, 30 seconds) was performed using endoACTB_seqF04 primer (SEQ ID NO: 8) and SupreDye v3.1 Cycle Sequencing Kit (Edge BioSystems), and purification was performed using ethanol precipitation.

**[Table 8]**

| Primer | Sequence | SEQ ID NO: |
|---|---|---|
| endo_ACTB_F02 | CAGCAGATGTGGATCAGCAAGCAGGAG | 6 |
| endo_ACTB_R02 | GGAAGGGGGGGCACGAAGGCTCATC | 7 |
| endo_ACTB_seq04 | GGTGACAGCAGTCGGTTGGAGCGAGC | 8 |

### (C) Results of editing analysis

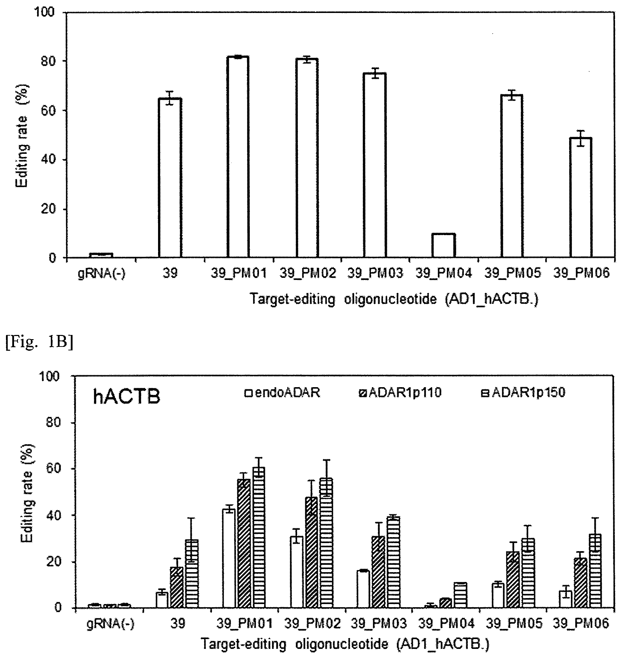

The results of the RNA editing rate that were obtained using the target-editing oligonucleotide (gRNA) are shown in Fig. 1B. The intracellular editing induction of the gRNA that was an Example compound or a Comparative Example compound was evaluated, in which the gRNA was transfected to a concentration of 50 nM. As a result, gRNA exhibited a higher editing rate, compared to gRNA(-). Additionally, Example compound 2 (AD1_hACTB.39_PM02) in which the bond at the internucleoside +1 site was a methylphosphonate bond and Example compound 3 (AD1_hACTB.39_PM03) in which the bond at the internucleoside +4 site was a methylphosphonate bond exhibited an increased editing rate, compared to Reference Example compound 1 (AD1_hACTB.39) in which all the bonds at the internucleosides in RNA were phosphorothioate bonds. Furthermore, Example compound 1 (AD1_hACTB.39_PM01) in which the bonds at the internucleoside +1 and +4 sites were methylphosphonate bonds in the gRNA exhibited a further increased editing rate. On the other hand, Comparative Example compounds 1 to 3 (AD1_hACTB.39_PM04 to 06) in which the internucleoside +2, -9, and -7 sites were methylphosphonate bonds exhibited a slight increase or decrease in the editing rate, compared to Reference Example compound 1 (AD1_hACTB.39). As shown in Fig. 2B, Comparative Example compounds 4 to 9 (AD1_hACTB.39_PM07 to 12) in which any of the internucleoside -5, -1, +6, +8, +10, or +12 sites was a methylphosphonate bond exhibited a slight increase in the editing rate. As shown in Fig. 3C, Comparative Example compound 10 (AD1_hACTB.39_PM13) in which the internucleoside -2 site was a methylphosphonate bond exhibited a comparable editing rate, compared to Reference Example compound 1 (AD1_hACTB.39). Additionally, as shown in Fig. 3E, Comparative Example compound 21 (AD1_hACTB.39_PM08a) in which the internucleosides -1 site was a methylphosphonate bond did not exhibit an increase in the editing rate. These results have revealed that, in particular, using a methylphosphonate bond(s) at the internucleoside(s) +1 and/or +4 site(s) in the gRNA results in exhibiting a high editing rate.

The results of the RNA editing rate that were obtained using the Example compounds are shown in Fig. 3C. Example compound 9 (AD1_hACTB.39_PSM02) in which the bond at the internucleoside +1 site was a methylthiophosphonate bond and Example compound 10 (AD1_hACTB.39_PSM03) in which the bond at the internucleoside +4 site was a methylthiophosphonate bond exhibited an increased editing rate, compared to Reference Example compound 1 (AD1_hACTB.39) in which all the bonds at the internucleosides in gRNA were phosphorothioate bonds. Furthermore, Example compound 8 (AD1_hACTB.39_PSM01) in which the bonds at the internucleoside +1 and +4 sites were methylthiophosphonate bonds in the gRNA exhibited a further increased editing rate. These results have revealed that, in particular, using a methylthiophosphonate bond(s) at the internucleoside(s) +1 and/or +4 site(s) in the gRNA results in exhibiting a high editing rate.

The results of the RNA editing rate that were obtained using the Example compounds are shown in Fig. 4C. Example compound 5 (AD1_hACTB.39_POE02) in which the bond at the internucleoside +1 site was an ethyl phosphate bond and Example compound 6 (AD1_hACTB.39_POE03) in which the bond at the internucleoside +4 site was an ethyl phosphate bond exhibited a high editing rate, compared to Reference Example compound 1 (AD1_hACTB.39) in which all the bonds at the internucleosides in the gRNA were phosphorothioate bonds. Furthermore, Example compound 4 (AD1_hACTB.39_POE01) in which the bonds at the inter nucleoside +1 and +4 sites in the gRNA were ethyl phosphate bonds exhibited an even higher editing rate. These results have revealed that, in particular, using an ethyl phosphate bond at the bond(s) at the internucleoside +1 and/or +4 site(s) in the gRNA results in exhibiting a high editing rate.

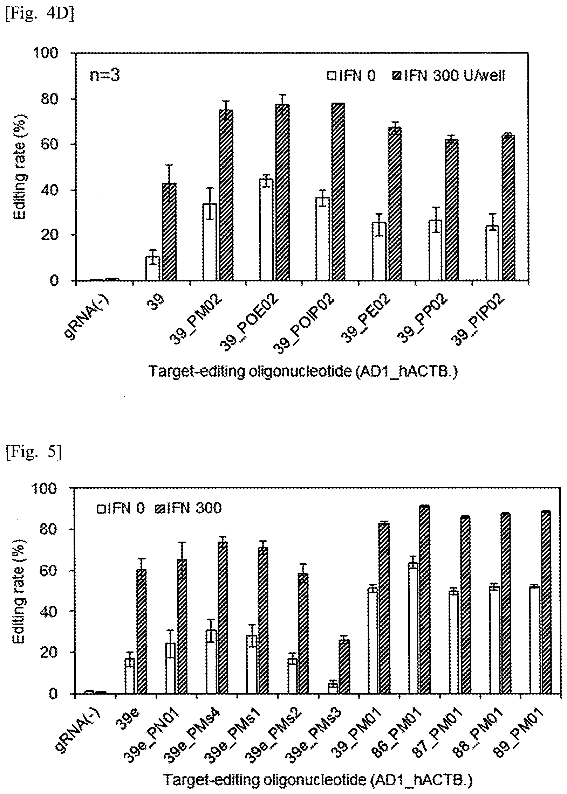

The results of the RNA editing rate that were obtained using the Example compounds are shown in Fig. 4D. Example compound 7 (AD1_hACTB.39_POIP02) in which the bond at the internucleoside +1 site was an isopropyl phosphate bond, Example compound 11 (AD1_hACTB.39_PE02) in which the bond at the internucleoside +1 site was an ethylphosphonate bond, Example compound 12 (AD1_hACTB.39_PP02) in which the bond was a propylphosphonate bond, and Example compound 13 (AD1_hACTB.39_PIP02) in which the bond was an isopropylphosphonate bond exhibited a high editing rate, compared to Reference Example compound 1 (AD1_hACTB.39) in which all the bonds at the internucleosides in the gRNA were phosphorothioate bonds. These results have revealed that, in particular, using an isopropyl phosphate bond, an ethylphosphonate bond, a propylphosphonate bond, or an isopropylphosphonate bond at the bond at the internucleoside +1 site in the gRNA results in exhibiting a high editing rate.

The results of the RNA editing rate that were obtained using the Example compounds and Comparative Example compounds are shown in Fig. 5. Example compound 60 (AD1_hACTB.39e_PMs01) in which the bond at the internucleoside +1 site was a mesylphosphoramidate bond and Example compound 61 (AD1_hACTB.39_PMs02) in which the bond at the internucleoside +4 site was a mesylphosphoramidate bond exhibited a high editing rate, compared to Reference Example compound 2 (AD1_hACTB.39e) in which all the bonds at the nucleosides in the gRNA were phosphorothioate bonds. Furthermore, Example compound 62 (AD1_hACTB.39e_PN01) in which the bonds at the internucleoside +1 and +4 sites in the gRNA were 1,3-dimethylimidazolidin-2-ylidene phosphate bonds, and Example compound 59 (AD1_hACTB.39e_PMs04) in which the bonds at the internucleoside +1 and +4 sites were mesylphosphoramidate bonds exhibited an even higher editing rate. On the other hand, Comparative Example compound 21 (AD1_hACTB.39e_PMs3) in which the bond at the internucleosides +2 site was a mesylphosphoramidate bond exhibited a decrease in the editing rate. These results have revealed that, in particular, using a mesylphosphoramidate bond or a 1,3-dimethylimidazolidin-2-ylidene phosphate bond at the bond(s) at the internucleoside +1 and/or +4 site(s) in the gRNA results in exhibiting a high editing rate.

The results of the RNA editing rate that were obtained using Example compounds in which the number and positions of LNAs in the gRNA were changed are shown in Fig. 5. Example compound 1 (AD1_hACTB.39_PM01) in which the bonds at the internucleoside +1 and +4 sites in the gRNA were methylphosphonate bonds has four LNAs, but Example compound 63 (AD1_hACTB.86_PM01), Example compound 64 (AD1_hACTB.87_PM01), Example compound 65 (AD1_hACTB.88_PM01), and Example compound 66 (AD1_hACTB.89_PM01), in which compounds the number of LNAs was reduced and/or the position(s) thereof was/were changed, exhibited a comparable or higher editing rate. These results have revealed that, in particular, a gRNA having a methylphosphonate bond at the bond(s) at the internucleoside +1 and/or +4 site(s) in the gRNA exhibits a high editing rate.

The results of the RNA editing rate that were obtained using the Comparative Example compounds are shown in Fig. 6B. Comparative Example compound 12 (AD1_hACTB.39_PO02) in which the bond at the internucleoside +1 site was a phosphodiester bond and Comparative Example compound 13 (AD1_hACTB.39_PO03) in which the bond at the internucleoside +4 site was a phosphodiester bond exhibited a high editing rate, compared to Reference Example compound 1 (AD1_hACTB.39) in which all the bonds at the internucleosides in the gRNA were phosphorothioate bonds. Furthermore, Comparative Example compound 11 (AD1_hACTB.39_PO01) in which the bonds at the internucleoside +1 and +4 sites in the gRNA were phosphodiester bonds exhibited an even higher editing rate. These results have revealed that a high editing rate is exhibited when a phosphodiester bond is used in the gRNA, particularly at the internucleoside +1 and/or +4 site(s).

The results of the RNA editing rate that were obtained using the Example compounds and Comparative Example compounds are shown in Fig. 7B. Comparative Example compound 14 (AD1_hACTB.39_POE14) in which the bond at the internucleoside -4 site was an ethyl phosphate bond, Comparative Example compound 15 (AD1_hACTB.39_POE15) in which the bond at the internucleoside -3 site was an ethyl phosphate bond, Comparative Example compound 17 (AD1_hACTB.39_POE17) in which the bond at the internucleoside +5 site was an ethyl phosphate bond, Comparative Example compound 18 (AD1_hACTB.39_POE18) in which the bond at the internucleoside +7 site was an ethyl phosphate bond, and Comparative Example compound 19 (AD1_hACTB.39_POE19) in which the bond at the internucleoside +9 site was an ethyl phosphate bond showed comparable editing rates, compared to Reference Example compound 1 (AD1_hACTB.39) in which all the bonds at the internucleosides in the gRNA were phosphorothioate bonds. On the other hand, Comparative Example compound 15 (AD1_hACTB.39_POE16) in which the bond at the internucleoside +3 site in the gRNA was an ethyl phosphate bond exhibited a slight decrease in the editing rate. Additionally, Example compound 19 (AD1_hACTB.39_POE20) in which the bonds at the three sites, internucleosides -5, +1, and +4, in the gRNA were ethyl phosphate bonds, Example compound 20 (AD1_hACTB.39_POE21) in which the bonds at the three sites, internucleosides +1, +4, and +8, were ethyl phosphate bonds, and Example compound 21 (AD1_hACTB.39_POE22) in which the bonds at the three sites, internucleosides +1, +4, and +10 were ethyl phosphate bonds exhibited a high editing rate. These results have revealed that, in particular, using an ethyl phosphate bond at the bond(s) at the internucleoside +1 and/or +4 site(s) in the gRNA results in exhibiting a high editing rate. The results have also revealed that the compounds having an ethyl phosphate bond at any of the internucleoside -5, +8, or +10 sites in addition to the internucleoside +1 and +4 sites also exhibit a high editing rate.

The results of the RNA editing rate that were obtained using the Example compounds are shown in Fig. 8B. Example compound 34 (AD1_hACTB.39_PB02) in which the bond at the internucleoside +1 site was a butylphosphonate bond, Example compound 35 (AD1_hACTB.39_PMOP02) in which the bond at the internucleoside +1 site was a methoxypropylphosphonate bond, Example compound 54 (AD1_hACTB.39_POP02) in which the bond at the internucleoside +1 site was a P-propoxy bond, Example compound 55 (AD1_hACTB.39_POB02) in which the bond at the internucleoside +1 site was a butyl phosphate bond, Example compound 56 (AD1_hACTB.39_POMOE02) in which the bond at the internucleoside +1 site was a methoxyethyl phosphate bond, Example compound 57 (AD1_hACTB.39_POcP02) in which the bond at the internucleoside +1 site was a cyclopropyl phosphate bond, and Example compound 58 (AD1_hACTB.39_POcB02) in which the bond at the internucleoside +1 site was a cyclobutyl phosphate bond exhibited a high editing rate, compared to Reference Example compound 1 (AD1_hACTB.39) in which all the bonds at the internucleosides in the gRNA were phosphorothioate bonds. These results have revealed that a high editing rate is exhibited when a butylphosphonate bond, a methoxypropylphosphonate bond, a propyl phosphate bond, a butyl phosphate bond, a methoxyethyl phosphate bond, a cyclopropyl phosphate bond, or a cyclobutyl phosphate bond is used in the gRNA, particularly at the internucleoside +1 site.

The results of the RNA editing rate that were obtained using the Example compounds are shown in Fig. 9B. Example compound 22 (AD1_hACTB.39_POE23) having ethyl phosphate bonds at the internucleoside +1 and +4 sites and further having a phosphodiester bond at the internucleoside +2 site, Example compound 23 (AD1_hACTB.39_POE24) having ethyl phosphate bonds at the internucleoside +1 and +4 sites and further having phosphodiester bonds at the internucleoside -3 and +2 sites, Example compound 24 (AD1_hACTB.39_POE25) having ethyl phosphate bonds at the internucleoside +1 and +4 sites and further having phosphodiester bonds at the internucleoside -8, -6, +2, +11 and +13 sites, Example compound 25 (AD1_hACTB.39_POE26) having ethyl phosphate bonds at the internucleoside +1 and +4 sites and further having phosphodiester bonds at the internucleoside -7, -5, +2, +10 and +12 sites, Example compound 26 (AD1_hACTB.39_POE27) having ethyl phosphate bonds at the internucleoside +1 and +4 sites and further having phosphodiester bonds at the internucleoside +2, +3 and +5 sites, Example compound 27 (AD1_hACTB.39_POE28) having ethyl phosphate bonds at the internucleoside +1 and +4 sites and further having phosphodiester bonds at the internucleoside -3, -2, -1, 0, +2, +3 and +5 sites, and Example compound 28 (AD1_hACTB.39_POE29) having ethyl phosphate bonds at the internucleoside +1 and +4 sites and further having phosphodiester bonds at the internucleoside -3 to -1, 0, +2, +3 and +5 to +9 sites exhibited a high editing rate, compared to Reference Example compound 1 (AD1_hACTB.39) in which all the bonds at the internucleosides in the gRNA were phosphorothioate bonds. Additionally, none of Reference Example compound 4 (AD1_hACTB.39_POE31) in which all the bonds at the internucleosides in the gRNA were phosphodiester bonds and Comparative Example compound 20 (AD1_hACTB.39_POE30) having ethyl phosphate bonds at the internucleoside +1 and +4 sites and having phosphodiester bonds at the other sites exhibited a high editing rate. The reason was considered to be that there were many phosphodiester bonds. These results have revealed that a high editing rate is exhibited particularly when an ethyl phosphate bond is used in the gRNA at the internucleoside +1 and +4 sites. The results have revealed, in addition to the above, that a high editing rate is exhibited when an ethyl phosphate bond is used at the +2 site.

The results of the RNA editing rate that were obtained using the Example compounds are shown in Fig. 10B. Example compound 36 (AD1_hACTB.39_POE32) having ethyl phosphate bonds at the internucleoside +1 and +4 sites and further having a phosphodiester bond at the internucleoside -1 site, Example compound 37 (AD1_hACTB.39_POE33) having ethyl phosphate bonds at the internucleoside +1 and +4 sites and further having a phosphodiester bond at the internucleoside -2 site, Example compound 38 (AD1_hACTB.39_POE34) having ethyl phosphate bonds at the internucleoside +1 and +4 sites and further having phosphodiester bonds at the internucleoside -6, +2, and +11 sites, Example compound 39 (AD1_hACTB.39_POE35) having ethyl phosphate bonds at the internucleoside +1 and +4 sites and further having phosphodiester bonds at the internucleoside -6, +2, +6, and +11 sites, Example compound 40 (AD1_hACTB.39_POE36) having ethyl phosphate bonds at the internucleoside +1 and +4 sites and further having phosphodiester bonds at the internucleoside -6, +2, +8, and +11 sites, Example compound 41 (AD1_hACTB.39_POE37) having ethyl phosphate bonds at the internucleoside +1 and +4 sites and further having phosphodiester bonds at the internucleoside -6, +2, +10, and +11 sites, Example compound 42 (AD1_hACTB.39_POE38) having ethyl phosphate bonds at the internucleoside +1 and +4 sites and further having phosphodiester bonds at the internucleoside -6, -5, +2, and +11 sites, Example compound 43 (AD1_hACTB.39_POE39) having ethyl phosphate bonds at the internucleoside +1 and +4 sites and further having phosphodiester bonds at the internucleoside -6, -5, +2, +10, and +11 sites, Example compound 44 (AD1_hACTB.39_POE40) having ethyl phosphate bonds at the internucleoside +1 and +4 sites and further having phosphodiester bonds at the internucleoside -6, -3, +2, +6, and +11 sites, Example compound 45 (AD1_hACTB.39_POE41) having ethyl phosphate bonds at the internucleoside +1 and +4 sites and further having phosphodiester bonds at the internucleoside -6, -3, +2, +8, and +11 sites, Example compound 46 (AD1_hACTB.39_POE42) having ethyl phosphate bonds at the internucleoside +1 and +4 sites and further having phosphodiester bonds at the internucleoside -6, -3, +2, +10, and +11 sites, Example compound 47 (AD1_hACTB.39_POE43) having ethyl phosphate bonds at the internucleoside +1 and +4 sites and further having phosphodiester bonds at the internucleoside -6, -5, -3, +2, and +11 sites, and Example compound 48 (AD1_hACTB.39_POE44) having ethyl phosphate bonds at the internucleoside +1 and +4 sites and further having phosphodiester bonds at the internucleoside -6, -5, -3, +2, +10, and +11 sites exhibited a high editing rate, compared to Reference Example compound 1 (AD1_hACTB.39) in which all the bonds at the internucleosides in the gRNA were phosphorothioate bonds. These results have revealed that a high editing rate is exhibited particularly when an ethyl phosphate bond is used in the gRNA at the internucleoside +1 and +4 sites. The results have revealed, in addition to the above, that a high editing rate is exhibited when an ethyl phosphate bond is used at the internucleoside +2, -6, and +11 sites.

The results of the RNA editing rate that were obtained using the Example compounds are shown in Fig. 11B. Example compound 29 (AD1_hACTB.39_PE03) in which the bond at the internucleoside +4 site was an ethylphosphonate bond, Example compound 30 (AD1_hACTB.39_PP03) in which the bond at the internucleoside +4 site was a propylphosphonate bond, Example compound 31 (AD1_hACTB.39_PIP03) in which the bond at the internucleoside +4 site was an isopropylphosphonate bond, Example compound 32 (AD1_hACTB.39_PB03) in which the bond at the internucleoside +4 site was a butylphosphonate bond, Example compound 33 (AD1_hACTB.39_PMOP03) in which the bond at the internucleoside +4 site was a methoxypropylphosphonate bond, Example compound 49 (AD1_hACTB.39_POP03) in which the bond at the internucleoside +4 site was a propyl phosphate bond, Example compound 50 (AD1_hACTB.39_POB03) in which the bond at the internucleoside +4 site was a butyl phosphate bond, Example compound 51 (AD1_hACTB.39_POMOE03) in which the bond at the internucleoside +4 site was a methoxyethyl phosphate bond, Example compound 52 (AD1_hACTB.39_POcP03) in which the bond at the internucleoside +4 site was a cyclopropyl phosphate bond, and Example compound 53 (AD1_hACTB.39_POcB03) in which the bond at the internucleoside +4 site was a cyclobutyl phosphate bond exhibited an increased editing rate, compared to Reference Example compound 1 (AD1_hACTB.39) in which all the bonds at the internucleosides in the gRNA were phosphorothioate bonds. These results have revealed that a high editing rate is exhibited when an ethylphosphonate bond, a propylphosphonate bond, an isopropylphosphonate bond, a butylphosphonate bond, a methoxypropylphosphonate bond, a propyl phosphate bond, a butyl phosphate bond, a methoxyethyl phosphate bond, a cyclopropyl phosphate bond, or a cyclobutyl phosphate bond is used in the gRNA, particularly at the internucleoside +4 site.

The results of the editing rate by ADAR1p110 are shown in Fig. 11D. Example compound 73 (AD1_hACTB.86_POE45) in which the bonds at the internucleoside +1 and +4 sites were ethyl phosphate bonds, Example compound 74 (AD1_hACTB.86_POE46) having ethyl phosphate bonds at the internucleoside +1 and +4 sites and further having a phosphodiester bond at the internucleoside +2 site, Example compound 75 (AD1_hACTB.86_POE47) having ethyl phosphate bonds at the internucleoside +1 and +4 sites and further having phosphodiester bonds at the internucleoside +2 and +6 sites, Example compound 76 (AD1_hACTB.86_POE48) having ethyl phosphate bonds at the internucleoside +1 and +4 sites and further having phosphodiester bonds at the internucleoside +2 and +8 sites, Example compound 77 (AD1_hACTB.86_POE49) having ethyl phosphate bonds at the internucleoside +1 and +4 sites and further having phosphodiester bonds at the internucleoside +2 and +10 sites, Example compound 78 (AD1_hACTB.86_POE50) having ethyl phosphate bonds at the internucleoside +1 and +4 sites and further having phosphodiester bonds at the internucleoside +2, +6 and +8 sites, Example compound 79 (AD1_hACTB.86_POE51) having ethyl phosphate bonds at the internucleoside +1 and +4 sites and further having phosphodiester bonds at the internucleoside +2, +6 and +10 sites, Example compound 80 (AD1_hACTB.86_POE52) having ethyl phosphate bonds at the internucleoside +1 and +4 sites and further having phosphodiester bonds at the internucleoside +2, +8 and +10 sites, Example compound 81 (AD1_hACTB.86_POE53) having ethyl phosphate bonds at the internucleoside +1 and +4 sites and further having phosphodiester bonds at the internucleoside +2, +6, +8 and +10 sites, Example compound 82 (AD1_hACTB.86_POMOE46) having a methoxyethyl phosphate bond at the bond at the internucleoside +1 site, an ethyl phosphate bond at the internucleoside +4 site, and further having a phosphodiester bond at the internucleoside +2 site, and Example compound 83 (AD1_hACTB.86p_POE46) having nebularine as the nucleoside at +1, having ethyl phosphate bonds at the internucleoside +1 and +4 sites, and further having a phosphodiester bond at the internucleoside +2 site exhibited a high editing rate, compared to Reference Example compound 32 (AD1_hACTB.86) in which all the bonds at the internucleosides in the gRNA were phosphorothioate bonds. These results have revealed that a high editing rate is exhibited particularly when ethyl phosphate bonds are used in the gRNA at the internucleoside +1 and +4 sites, or when a methoxyethyl phosphate bond is used at the internucleoside +1 site, and when an ethyl phosphate bond is used at the internucleoside +4 site. Furthermore, the results have revealed, in addition to the above, that using a phosphodiester bond at the internucleoside +2, +6, +8 and/or +10 site(s), in particular, using a phosphodiester bond at the +2 site, results in exhibiting a high editing rate.

### Test Example 3

### Evaluation of editing-inducing capability of target-editing oligonucleotide for RNA having A1AT gene sequence, in cell-free system

### (A) Synthesis of RNA

The operation was performed in the same manner as described in (A) Synthesis of ACTB_RNA in Test Example 1. However, the oligo DNA used was as described below. The A1AT (also referred to as SERPINA1) gene sequence used here has a base sequence from positions 1129 to 1153 of GenBank accession No. NM_000295.5, and further has a nucleoside mutation called c.1096G>A (rs28929474).

**[Table 9]**

| Primer | Sequence | SEQ ID NO: |
|---|---|---|
| A1AT_FW | AATTCGCATAAGGCTGTGCTGACCATCGACAAGAAAGGGACTGAAGCTGCTGGGGCA | 9 |
| A1AT_RV | AGCTTGCCCCAGCAGCTTCAGTCCCTTTCTTGTCGATGGTCAGCACAGCCTTATGCG | 10 |

### (B) Evaluation of editing-inducing capability

The method of evaluating the editing-inducing capability of the Example compounds was the same as in (B) to (D) in Test Example 1.

### (C) Results of editing analysis

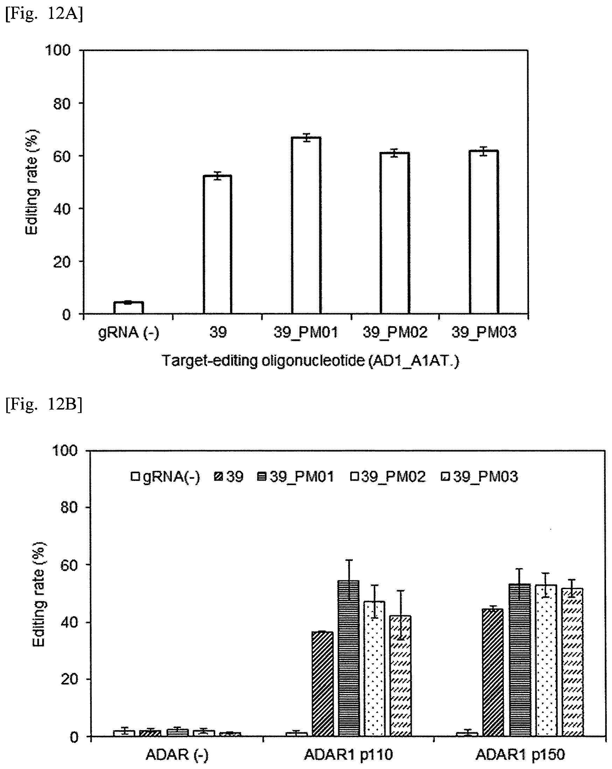

The results of the editing rate by ADAR1p110 are shown in Fig. 12A. Regarding the RNA editing efficiency by ADAR1p110, Example compound 68 (AD1_A1AT_PM02) in which the bond at the internucleoside +1 site was a methylphosphonate bond and Example compound 69 (AD1_A1AT_PM03) in which the bond at the internucleoside +4 site was a methylphosphonate bond exhibited a high editing rate, compared to a compound (AD1_A1AT.39, a compound described in Example 136 in the specification of WO2022/124345) in which all the bonds at the internucleosides in the gRNA were phosphorothioate bonds. Furthermore, Example compound 67 (AD1_A1AT_PM01) in which the bonds at the internucleoside +1 and +4 sites in the gRNA were methylphosphonate bonds exhibited an even higher editing rate. These results have revealed that using a methylphosphonate bond(s) at the internucleoside(s) +1 and/or +4 site(s) in the gRNA results in exhibiting a high editing rate.

### Test Example 4

### Evaluation of editing-inducing capability of Example compound for RNA having A1AT gene sequence in cultured cell

### (A) Method of evaluating editing-inducing capability

The editing-inducing capability of the Example compounds for the A1AT gene sequence was evaluated using the method described in Test Example 35 in the specification of WO2022/124345. The A1AT (also referred to as SERPINA1) gene sequence used here has a base sequence from positions 1129 to 1153 of GenBank accession No. NM_000295.5, and further has a nucleoside mutation called c.1096G>A (rs28929474).

### (B) Results of editing analysis

The results of the RNA editing rate that were obtained using the Example compounds are shown in Fig. 12B. Example compound 68 (AD1_A1AT_PM02) in which the bond at the internucleoside +1 site was a methylphosphonate bond and Example compound 69 (AD1_A1AT_PM03) in which the bond at the internucleoside +4 site was a methylphosphonate bond exhibited a high editing rate, compared to a compound (AD1_A1AT.39, a compound described in Example 136 in the specification of WO2022/124345) in which all the bonds at the internucleosides in the gRNA were phosphorothioate bonds. Furthermore, Example compound 67 (AD1_A1AT_PM01) in which the bonds at the internucleoside +1 and +4 sites in the gRNA were methylphosphonate bonds exhibited an even higher editing rate. These results have revealed that using a methylphosphonate bond(s) at the internucleoside(s) +1 and/or +4 site(s) in the gRNA results in exhibiting a high editing rate.

### Test Example 5

### Evaluation of editing-inducing capability of target-editing oligonucleotide for RNA having mouse GAPDH gene sequence, in cell-free system

### (A) Synthesis of RNA

The operation was performed in the same manner as described in (A) Synthesis of ACTB_RNA in Test Example 1. However, the oligo DNA used was as described below. The mouse GAPDH gene sequence used here has a base sequence from positions 1129 to 1153 of GenBank accession No. NM_000295.5.

**[Table 10]**

| Primer | Sequence | SEQ ID NO: |
|---|---|---|
| mGAPDH_A1222_T7F01 | CTAATACGACTCACTATAGGTATGACAATGAATACGGCTAC | 11 |
| mGAPDH_A1222_2ndR1 | GGTATTCAAGAGAGTAGGGAGG | 12 |

### (B) Evaluation of editing-inducing capability

The method of evaluating the editing-inducing capability of the Example compounds was the same as in (B) to (D) in Test Example 1.

### (C) Results of editing analysis

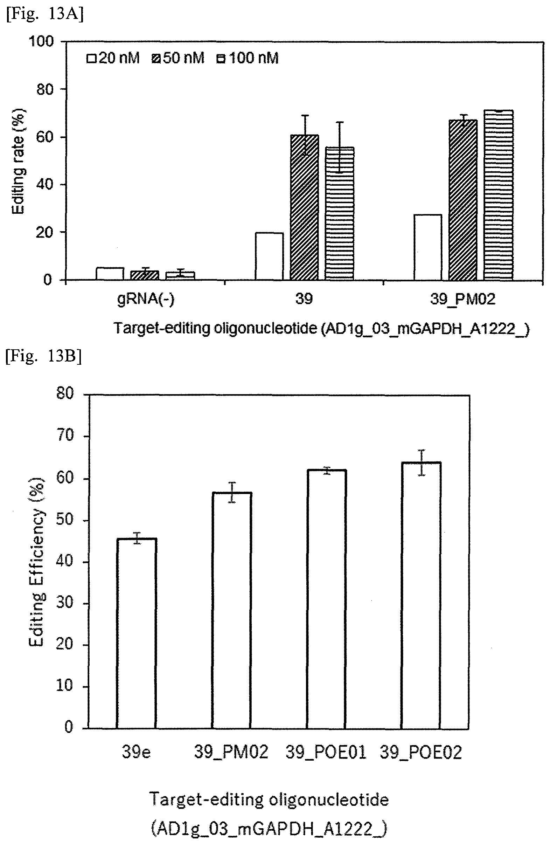

The results of the editing rate by ADAR1p110 are shown in Fig. 13A. Regarding the RNA editing efficiency by ADAR1p110, Example compound 70 (AD1g_03_mGAPDH_A1222_PM02) in which the bond at the internucleoside +1 site was a methylphosphonate bond exhibited a high editing rate, compared to Reference Example compound 3 (AD1g_03_mGAPDH_A1222_39) in which all the bonds at the internucleosides in the gRNA were phosphorothioate bonds. These results have revealed that using a methylphosphonate bond at the internucleoside +1 site in the gRNA results in exhibiting a high editing rate.

### Test Example 6

Evaluation of editing-inducing capability of target-editing oligonucleotide for RNA having mouse GAPDH gene sequence, in cultured cell

### (A) Method of evaluating editing-inducing capability

CD-1-derived mouse primary hepatocytes (Thermo Fisher Scientific Inc.) were seeded in a 96-well plate at 2.0 × 10⁴ cells/well. After 24 hours, the Example compound was added to a final concentration of 2 µM. Total RNA was extracted from the cells cultured in the 96-well Plate, using RNeasy Plus Micro Kit (QIAGEN). Reverse transcription reaction was performed using SuperScript^{™} IV VILO^{™} Master Mix with ezDNase^{™} Enzyme (Thermo Fisher Scientific Inc.) and 0.5 µg of Total RNA to amplify cDNA. The Gapdh fragment was amplified by PCR (denaturation, 98°C, 10 seconds; annealing, 55°C, 5 seconds; elongation, 72°C, 5 seconds) using PrimeSTAR^{(R)} Max DNA Polymerase (TaKaRa Bio Inc.), the endomGapdh_Fw primer (SEQ ID NO: 13), and the endomGapdh_Rv (SEQ ID NO: 14). The amplification was confirmed by E-Gel Precast Agarose Electrophoresis System (Thermo Fisher Scientific Inc.). The amplification product was purified using QIAquick PCR Purification Kit (Qiagen N.V.), and sequence analysis was performed using the endoGapdh_Fw primer (SEQ ID NO: 13).

**[Table 11]**

| Primer | Sequence | SEQ ID NO: |
|---|---|---|
| endomGapdh_Fw | TGTTCCTACCCCCAATGTGT | 13 |
| endomGapdh_Rv | GCAGCGAACTTTATTGATGG | 14 |

### (B) Results of editing analysis

The results of the RNA editing rate that were obtained using the Example compounds are shown in Fig. 13(B). Example compound 70 (AD1g_03_mGAPDH_A1222_PM02) in which the bond at the internucleoside +1 site was a methylphosphonate bond, Example compound 71 (AD1g_03_mGAPDH_A1222_POE01), and Example compound 72 (AD1g_03_mGAPDH_A1222_POE02) exhibited a high editing rate, compared to Reference Example compound 5 (AD1g_03_mGAPDH_A1222_39e) in which all the bonds at the internucleosides in the gRNA were phosphorothioate bonds. These results have revealed that a high editing rate is exhibited when a methylphosphonate bond is used at the internucleoside +1 site, ethyl phosphate bonds are used at the internucleoside +1 and +4 sites, or an ethyl phosphate bond is used at the internucleoside +1 site, in the gRNA.

### Examples 84 to 88

In the same manner as the target-editing oligonucleotides in Examples 1 to 83, the target-editing oligonucleotides in Examples 84 to 88 were obtained by introducing modified oligonucleotides and phosphorus-containing linking groups as shown in the modification patterns in Table 12 on the basis of the base sequences of the target-editing oligonucleotides shown in Table 1.

**[Table 12]**

| | Sequence Name | Sequence (5'-3) | Molecular weight | SEQ ID NO: |
|---|---|---|---|---|
| Example 84 | AD1g_03_mGAPDH_A1222_3 9e_POMOE23 | G(M)^A(E)^G(M)^G(E)^G(M)^C(F)^C(F)^C(M)^C(M)^c^a(POMOE)G(M)pG(F)^C(M)(PO E)C(F)^C(M)^C(F)^U(M)^C(F)^C(M)^T(E)^G(M)^T(E)^U(M) | 8282.0 | 128 |
| Example 85 | AD1g_03_mGAPDH_A1222_3 9e_POMOE34 | G(M)^A(E)^G(M)^G(E)pG(M)^C(F)^C(F)^C(M)^C(M)^c^a(POMOE)G(M)pG(F)^C(M)(P OE)C(F)^C(M)^C(F)^U(M)^C(F)^C(M)^T(E)pG(M)^T(E)^U(M) | 8248.8 | 129 |
| Example 86 | AD1g_03_mGAPDH_A1222_3 9e_POMOE46 | G(M)^A(E)^G(M)^G(E)^G(M)^C(F)^C(F)^C(M)^C(M)^c^a(POMOE)G(M)pG(F)^C(M)(PO E)C(F)^C(M)^C(F)^U(M)^C(F)^C(M)^U(M)^G(M)^T(E)^U(M) | 8255.3 | 130 |
| Example 87 | AD1g_03_mGAPDH_A1222_3 9e_POMOE49 | G(M)^A(E)^G(M)^G(E)^G(M)^C(F)^C(F)^C(M)^C(M)^c^a(POMOE)G(M)pG(F)^C(M)(PO E)C(F)^C(M)^C(F)^U(M)^C(F)^C(M)pU(M)^G(M)^T(E)^U(M) | 8239.7 | 131 |
| Example 88 | AD1_hAC1B39e_POE54 | A(M)^A(E)^A(M)^G(E)^C(M)^A(F)^U(F)^G(M)^C(M)^c^a(POE)U(M)pC(F)^A(M)^C(F)^C( M)^U(F)^C(M)^C(F)^C(M)^C(E)^U(M)^G(E)^U(M) | 8162.0 | 132 |

The descriptions of "Molecular Weight" and "Sequence" in Table 12 are the same as in Tables 2 to 5.

### Test Example 7

Evaluation (2) of editing-inducing capability of target-editing oligonucleotide for RNA having mouse GAPDH gene sequence, in cultured cell

### (A) Method of evaluating editing-inducing capability

The method of evaluating the editing-inducing capability of the Example compounds was the same as in (A) in Test Example 6.

### (B) Results of editing analysis

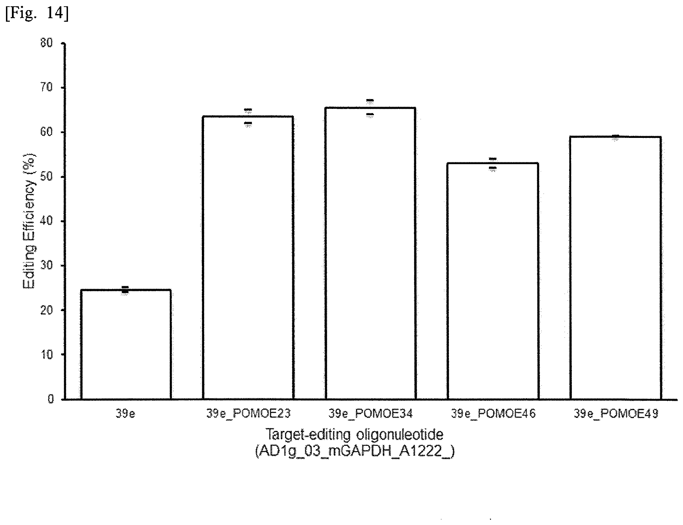

The results of the RNA editing rate that were obtained using the Example compounds are shown in Fig. 14. Fig. 14 is a bar graph obtained by conducting the test in two wells, plotting the values of the RNA editing rates of each well, and further graphing two average values. Example compound 84 (AD1g_03_mGAPDH_A1222_39e_POMOE23) in which the bond at the internucleoside +1 site was a methoxyethyl phosphate bond, the bond at the internucleoside +2 site was a phosphodiester bond, and the bond at the internucleoside +4 site was an ethyl phosphate bond, Example compound 85 (AD1g_03_mGAPDH_A1222_39e_POMOE34) in which, in addition to the modification of Example compound 84, the bonds at the internucleoside -6 and +11 sites were phosphodiester bonds, Example compound 86 (AD1g_03_mGAPDH_A1222_39e_POMOE46) in which the 11th nucleoside residue counting in the 3' direction from the target-corresponding nucleoside residue of Example compound 84 was a 2'-O-methyl nucleoside, Example compound 86 (AD1g_03_mGAPDH_A1222_39e_POMOE46) in which the bond at the internucleoside +10 site of Example compound 86 was a phosphodiester bond exhibited a high editing rate, compared to Reference Example compound 5 (AD1g_03_mGAPDH_A1222_39e) in which all the bonds at the internucleosides in the gRNA were phosphorothioate bonds. These results have revealed that a high editing rate is exhibited when a methoxyethyl phosphate bond is used at the bond at the internucleoside +1 site, a phosphodiester bond is used at the bond at the internucleoside +2 site, and an ethyl phosphate bond is used at the bond at the internucleoside +4 site, in the gRNA.

The disclosure of Japanese Patent Application No. 2023-137467 (filed on August 25, 2023) is incorporated herein by reference in its entirety. All publications, patent applications, and technical standards cited herein are incorporated by reference to the same extent as if each individual publication, patent application, or technical standard were specifically and individually indicated to be incorporated by reference.

## Claims

1. An oligonucleotide or a pharmaceutically acceptable salt thereof, the oligonucleotide comprising:
a first oligonucleotide that identifies a target RNA; and
a second oligonucleotide linked to the 5' side of the first oligonucleotide,
wherein the first oligonucleotide is composed of:
a target-corresponding nucleoside residue corresponding to an adenosine residue in the target RNA;
an oligonucleotide containing 3 to 6 residues, linked to the 5' side of the target-corresponding nucleoside residue, and having a base sequence complementary to the target RNA; and
an oligonucleotide containing 10 to 24 residues, linked to the 3' side of the target-corresponding nucleoside residue, and having a base sequence complementary to the target RNA,
wherein, in the oligonucleotide linked to the 3' side of the target-corresponding nucleoside residue, at least one phosphorus-containing linking group selected from the group consisting of a phosphorus-containing linking group linking a 1st nucleoside residue and a 2nd nucleoside residue and a phosphorus-containing linking group linking a 4th nucleoside residue and a 5th nucleoside residue, counting in the 3' direction from the target-corresponding nucleoside residue, is at least one selected from the group consisting of an alkylphosphonic residue, an alkyl phosphate residue, and a substituted phosphoramide residue,
wherein, at the 3' end of the second oligonucleotide, a nucleoside residue corresponding to a nucleoside residue of the target RNA is deleted, or, at the 3' end, the second oligonucleotide has a nucleoside residue that does not form a complementary pair with the nucleoside residue of the target RNA,
wherein the second oligonucleotide contains 2 to 10 residues, and nucleoside residues at least other than at the 3' end of the second oligonucleotide form a doublestranded structure complementary to the target RNA,
wherein at least one of the first oligonucleotide or the second oligonucleotide comprises a phosphorothioate bond, and
wherein the oligonucleotide or a pharmaceutically acceptable salt thereof induces site-specific editing in the target RNA.

2. The oligonucleotide or a pharmaceutically acceptable salt thereof according to claim 1, wherein a nucleoside residue linked to the 3' side of the target-corresponding nucleoside residue is a 2'-deoxynucleoside residue.

3. The oligonucleotide or a pharmaceutically acceptable salt thereof according to claim 1 or 2, wherein, in the oligonucleotide linked to the 3' side of the target-corresponding nucleoside residue, a 3rd nucleoside residue counting in the 3' direction from the target-corresponding nucleoside residue is a 2'-deoxy-2'-fluoronucleoside residue.

4. The oligonucleotide or a pharmaceutically acceptable salt thereof according to any one of claims 1 to 3, wherein the second oligonucleotide contains 4 to 8 residues.

5. The oligonucleotide or a pharmaceutically acceptable salt thereof according to any one of claims 1 to 4, wherein, at the 3' end of the second oligonucleotide, the nucleoside residue corresponding to the nucleoside residue of the target RNA is deleted.

6. The oligonucleotide or a pharmaceutically acceptable salt thereof according to any one of claims 1 to 5, wherein the site-specific editing is caused by an enzymatic reaction due to adenosine deaminase 1.

7. The oligonucleotide or a pharmaceutically acceptable salt thereof according to any one of claims 1 to 6, wherein the first oligonucleotide comprises a phosphorothioate bond.

8. The oligonucleotide or a pharmaceutically acceptable salt thereof according to any one of claims 1 to 7, wherein at least one phosphorus-containing linking group selected from the group consisting of: a phosphorus-containing linking group linking the 2nd nucleoside residue and the 3rd nucleoside residue, a phosphorus-containing linking group linking an 8th nucleoside residue and a 9th nucleoside residue, a phosphorus-containing linking group linking a 10th nucleoside residue and an 11th nucleoside residue, and a phosphorus-containing linking group linking the 11th nucleoside residue and a 12th nucleoside residue, counting in the 3' direction from the target-corresponding nucleoside residue; and a phosphorus-containing linking group linking a 6th nucleoside residue and the 5th nucleoside residue, counting in the 5' direction from the target-corresponding nucleoside residue, is a phosphoric residue.

9. The oligonucleotide or a pharmaceutically acceptable salt thereof according to any one of claims 1 to 8, wherein, in the first oligonucleotide, the 2nd nucleoside residue and the 3rd nucleoside residue counting in the 3' direction from the target-corresponding nucleoside residue are linked by a phosphodiester bond.

10. The oligonucleotide or a pharmaceutically acceptable salt thereof according to any one of claims 1 to 9, wherein the first oligonucleotide comprises at least one modified nucleoside residue selected from the group consisting of a 2'-O-alkylribonucleoside residue, a 2'-deoxy-2'-fluororibonucleoside residue, a bridged nucleoside residue, and a 2'-deoxyribonucleoside residue.

11. The oligonucleotide or a pharmaceutically acceptable salt thereof according to any one of claims 1 to 10, the oligonucleotide comprising a partial structure in which the target-corresponding nucleoside residue is linked to an adjacent nucleoside residue via a phosphorothioate bond.

12. The oligonucleotide or a pharmaceutically acceptable salt thereof according to any one of claims 1 to 11, wherein a nucleoside residue linked to the 5' side of the target-corresponding nucleoside residue is a 2'-O-alkylribonucleoside residue.

13. The oligonucleotide or a pharmaceutically acceptable salt thereof according to any one of claims 1 to 12, wherein the second oligonucleotide comprises at least one modified nucleoside residue selected from the group consisting of a 2'-O-alkylribonucleoside residue, a 2'-deoxy-2'-fluororibonucleoside residue, and a bridged nucleoside residue.

14. The oligonucleotide or a pharmaceutically acceptable salt thereof according to any one of claims 1 to 13, wherein the oligonucleotide linked to the 3' side of the target-corresponding nucleoside residue has a base sequence in which 2'-deoxy-2'-fluoronucleoside residues and 2'-O-alkylribonucleoside residues are alternately linked.

15. The oligonucleotide or a pharmaceutically acceptable salt thereof according to any one of claims 1 to 14, wherein the oligonucleotide linked to the 3' side of the target-corresponding nucleoside residue has a base sequence in which bridged nucleoside residues and 2'-O-alkylribonucleoside residues are alternately linked.

16. The oligonucleotide or a pharmaceutically acceptable salt thereof according to any one of claims 1 to 15, wherein, in the first oligonucleotide, the oligonucleotide linked to the 5' side of the target-corresponding nucleoside residue has a base sequence in which 2'-O-alkylribonucleoside residues are consecutive.

17. The oligonucleotide or a pharmaceutically acceptable salt thereof according to any one of claims 1 to 16, wherein the second oligonucleotide has a base sequence in which 2'-O-alkylribonucleoside residues and bridged nucleoside residues are alternately linked.

18. The oligonucleotide or a pharmaceutically acceptable salt thereof according to any one of claims 1 to 17, wherein the target-corresponding nucleoside residue is an N-alkylpyrimidine nucleoside residue or a 2'-deoxycytidine residue.

19. The oligonucleotide or a pharmaceutically acceptable salt thereof according to any one of claims 1 to 18, wherein the second oligonucleotide comprises nucleoside residues linked by a phosphorothioate bond.

20. The oligonucleotide or a pharmaceutically acceptable salt thereof according to any one of claims 1 to 19, wherein the first oligonucleotide contains a bridged nucleoside residue as the 10th or a subsequent nucleoside residue counting in the 3' direction from the target-corresponding nucleoside residue.

21. The oligonucleotide or a pharmaceutically acceptable salt thereof according to claim 20, wherein, in the first oligonucleotide, an oligonucleotide containing the 10th nucleoside residue and subsequent nucleoside residues counting in the 3' direction from the target-corresponding nucleoside residue has a base sequence in which 2'-O-alkylribonucleoside residues and bridged nucleoside residues are alternately linked.

22. The oligonucleotide or a pharmaceutically acceptable salt thereof according to any one of claims 1 to 21, wherein the second oligonucleotide contains a bridged nucleoside residue as the 2nd or a subsequent nucleoside residue counting in the 5' direction from a nucleoside residue at the 3' end of the second oligonucleotide.

23. The oligonucleotide or a pharmaceutically acceptable salt thereof according to claim 22, wherein the second oligonucleotide comprises three or more nucleoside residues, and wherein an oligonucleotide containing the 2nd nucleoside residue and subsequent nucleoside residues counting in the 5' direction from the nucleoside residue at the 3' end of the second oligonucleotide has a base sequence in which 2'-O-alkylribonucleoside residues and bridged nucleoside residues are alternately linked.

24. The oligonucleotide or a pharmaceutically acceptable salt thereof according to any one of claims 1 to 7, the oligonucleotide comprising a modified oligonucleotide having a chemical modification pattern represented by any one of the following Formulas (1) to (22): 
(1) 5'- HO-MsLsMsLsMsFsXFsMsMsdCsDqMsFsMqFsMsFsMsFsMsLsMsLsM-OH -
3'
 
(2) 5’- HO-MsLsMsLsMsFsXFsMsMsdCsDqMsFsMqFsMsFsMsFsMsMsMsLsM-OH -
3’
 
(3) 5’- HO-MsLsMsMsMsFsXFsMsMsdCsDqMsFsMqFsMsFsMsFsMsLsMsLsM-OH -
3’
 
(4) 5’- HO-MsLsMsMsMsFsXFsMsMsdCsDqMsFsMqFsMsFsMsFsMsMsMsLsM-OH
-3’
 
(5) 5'- HO-LsMsMsMsMsFsXFsMsMsdCsDqMsFsMqFsMsFsMsFsMsMsMsLsM-OH
-3'
 
(6) 5'- HO-MsLsMsLsMsFsXFsMsMsdCsDqMpFsMqFsMsFsMsFsMsLsMsLsM-OH -
3'
 
(7) 5’- HO-MsLsMsLsMsFsXFpMsMsdCsDqMpFsMqFsMsFsMsFsMsLsMsLsM-OH -
3’
 
(8) 5’- HO-MsLsMsLsMsFsXFsMpMsdCsDqMsFsMqFsMsFsMsFsMsLsMsLsM-OH -
3’
 
(9) 5’- HO-MsLsMsLpMsFsXFsMsMsdCsDqMpFsMqFsMsFsMsFsMsLpMsLsM-OH -
3’
 
(10) 5'- HO-MsLsMsLpMsFsXFsMsMsdCsDqMpFsMqFsMpFsMsFsMsLpMsLsM-
OH -3'
 
(11) 5’- HO-MsLsMsLpMsFsXFsMsMsdCsDqMpFsMqFsMsFsMpFsMsLpMsLsM-OH
-3’
 
(12) 5'- HO-MsLsMsLpMsFsXFsMsMsdCsDqMpFsMqFsMsFsMsFsMpLpMsLsM-
OH -3'
 
(13) 5’- HO-MsLsMsLsMsFsXFsMsMsdCsDqMpFsMqFsMsFsMsFsMsMsMsLsM-OH
-3’
 
(14) 5'- HO-MsLsMsLsMsFsXFsMsMsdCsDqMpFsMqFsMpFsMsFsMsMsMsLsM-
OH -3'
 
(15) 5'- HO-MsLsMsLsMsFsXFsMsMsdCsDqMpFsMqFsMsFsMpFsMsMsMsLsM-
OH -3'
 
(16) 5'- HO-MsLsMsLsMsFsXFsMsMsdCsDqMpFsMqFsMsFsMsFsMpMsMsLsM-
OH -3'
 
(17) 5'- HO-MsLsMsLsMsFsXFsMsMsdCsDqMpFsMqFsMpFsMpFsMsMsMsLsM-
OH -3'
 
(18) 5'- HO-MsLsMsLsMsFsXFsMsMsdCsDqMpFsMqFsMpFsMsFsMpMsMsLsM-
OH -3'
 
(19)  5'-  HO-MsLsMsLsMsFsXFsMsMsdCsDqMpFsMqFsMsFsMpFsMpMsMsLsM-
OH -3'
 
(20) 5'- HO-MsLsMsLsMsFsXFsMsMsdCsDqMpFsMqFsMpFsMpFsMpMsMsLsM-
OH -3'
 
(21) 5'- HO-MsLsMsLsMsFsXFsMsMsdCsDqMpFsMsFsMsFsMsFsMsLsMsLsM-OH
-3'
 
(22) 5'- HO-MsLsMsLsMsFsXFsMsMsdCsDqMpFsMsFsMsFsMsFsMsMsMsLsM-OH
-3',
 wherein D represents a 2'-deoxyribonucleoside residue, F represents a 2'-fluoro-2'-deoxyribonucleoside residue, L represents a bridged nucleoside residue, M represents a 2'-O-methyl- ribonucleoside residue, dC represents a target nucleoside residue, a 2'-deoxycytidine residue, X indicates that one nucleoside residue is missing from the target RNA, p represents a phosphate residue, s represents a thiophosphate residue, q represents an alkylphosphonate residue, an alkyl phosphate residue, or a substituted phosphoramide residue, HO- represents a hydroxyl group at the 5' end, and -OH represents a hydroxyl group at the 3' end.

25. The oligonucleotide or a pharmaceutically acceptable salt thereof according to any one of claims 1 to 7, the oligonucleotide comprising a modified oligonucleotide having a chemical modification pattern represented by any one of the following Formulas (1a) to (22a): 
(1a) 5'- HO-MsLsMsLsMsFsXFsMsMsdCsDpmMsFsMpmFsMsFsMsFsMsLsMsLsM-
OH -3'
 
(2a) 5’- HO-
MsLsMsLsMsFsXFsMsMsdCsDpmMsFsMpmFsMsFsMsFsMsMsMsLsM-OH -3’
 
(3a) 5'- HO-
MsLsMsMsMsFsXFsMsMsdCsDpmMsFsMpmFsMsFsMsFsMsLsMsLsM-OH -3'
 
(4a) 5’- HO-
MsLsMsMsMsFsXFsMsMsdCsDpmMsFsMpmFsMsFsMsFsMsMsMsLsM-OH -3’
 
(5a) 5'- HO-
LsMsMsMsMsFsXFsMsMsdCsDpmMsFsMpmFsMsFsMsFsMsMsMsLsM-OH -3'
 
(6a) 5'- HO-MsLsMsLsMsFsXFsMsMsdCsDrMpFsMrFsMsFsMsFsMsLsMsLsM-OH -
3'
 
(7a) 5’- HO-MsLsMsLsMsFsXFpMsMsdCsDrMpFsMrFsMsFsMsFsMsLsMsLsM-OH
-3’
 
(8a) 5’- HO-MsLsMsLsMsFsXFsMpMsdCsDrMsFsMrFsMsFsMsFsMsLsMsLsM-OH -
3’
 
(9a) 5’- HO-MsLsMsLpMsFsXFsMsMsdCsDrMpFsMrFsMsFsMsFsMsLpMsLsM-OH
-3’
 
(10a) 5'- HO-MsLsMsLpMsFsXFsMsMsdCsDrMpFsMrFsMpFsMsFsMsLpMsLsM-
OH -3'
 
(11a) 5’- HO-MsLsMsLpMsFsXFsMsMsdCsDrMpFsMrFsMsFsMpFsMsLpMsLsM-
OH -3’
 
(12a) 5'- HO-MsLsMsLpMsFsXFsMsMsdCsDrMpFsMrFsMsFsMsFsMpLpMsLsM-
OH -3'
 
(13a) 5'- HO-MsLsMsLsMsFsXFsMsMsdCsDrMpFsMrFsMsFsMsFsMsMsMsLsM-
OH -3'
 (14a) 5’- HO-MsLsMsLsMsFsXFsMsMsdCsDrMpFsMrFsMpFsMsFsMsMsMsLsM- OH -3' 
(15a) 5'- HO-MsLsMsLsMsFsXFsMsMsdCsDrMpFsMrFsMsFsMpFsMsMsMsLsM-
OH -3'
 
(16a) 5’- HO-MsLsMsLsMsFsXFsMsMsdCsDrMpFsMrFsMsFsMsFsMpMsMsLsM-
OH -3’
 
(17a) 5'- HO-MsLsMsLsMsFsXFsMsMsdCsDrMpFsMrFsMpFsMpFsMsMsMsLsM-
OH -3'
 
(18a) 5’- HO-MsLsMsLsMsFsXFsMsMsdCsDrMpFsMrFsMpFsMsFsMpMsMsLsM-
OH -3’
 
(19a) 5'- HO-MsLsMsLsMsFsXFsMsMsdCsDrMpFsMrFsMsFsMpFsMpMsMsLsM-
OH -3'
 
(20a) 5’- HO-MsLsMsLsMsFsXFsMsMsdCsDrMpFsMrFsMpFsMpFsMpMsMsLsM-
OH -3’
 
(21a) 5’- HO-MsLsMsLsMsFsXFsMsMsdCsDrMpFsMsFsMsFsMsFsMsLsMsLsM-OH
-3’
 
(22a) 5’- HO-MsLsMsLsMsFsXFsMsMsdCsDrMpFsMsFsMsFsMsFsMsMsMsLsM-
OH -3’,
 wherein D represents a 2'-deoxyribonucleoside residue, F represents a 2'-fluoro-2'-deoxyribonucleoside residue, L represents a bridged nucleoside residue, M represents a 2'-O-methyl- ribonucleoside residue, dC represents a target nucleoside residue, a 2'-deoxycytidine residue, X indicates that one nucleoside residue is missing from the target RNA, p represents a phosphate residue, s represents a thiophosphate residue, q represents an alkylphosphonate residue, an alkyl phosphate residue, or a substituted phosphoramide residue, HO- represents a hydroxyl group at the 5' end, and -OH represents a hydroxyl group at the 3' end.
